# EUROPEAN PATENT APPLICATION

(11) **EP 4 043 437 A1**
(43) Date of publication of application: **17.08.2022**
(21) Application number: 20863934.4
(22) Date of filing: 11.09.2020
(51) Int. Cl.: C07D 213/89, A61K 31/165

(54) **PYRIDINE OXYNITRIDE, PREPARATION METHOD THEREFOR AND USE THEREOF**

(30) Priority: 12.09.2019 CN 201910863718; 11.11.2019 CN 201911094782; 11.06.2020 CN 202010531381; 04.09.2020 CN 202010923311
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: ZHANG, Qiong, Shanghai 201203 (CN); WANG, Zhongli, Shanghai 201203 (CN); DAI, Ming, Shanghai 201203 (CN); CHENG, Fengkai, Shanghai 201203 (CN); LUO, Jiu, Shanghai 201203 (CN); YE, Yan, Shanghai 201203 (CN); PENG, Jianbiao, Shanghai 201203 (CN); GUO, Haibing, Shanghai 201203 (CN)
(74) Representative: J A Kemp LLP
(86) International application number: PCT/CN2020/114700
(87) International publication number: WO 2021/047622

(57) **Abstract**

The present invention belongs to the field of medicinal chemistry. Disclosed are a pyridine oxynitride, a preparation method therefor and the use thereof. Specifically, the present invention relates to a series of sodium ion channel blockers with a new structure, a preparation method therefor and the use thereof. The structure thereof is as shwon in general formula (I) below. The compounds or a stereoisomer, a racemate, a geometric isomer, a tautomer, a prodrug, a hydrate, a solvate, or a pharmaceutically acceptable salt thereof and a pharmaceutical composition can be used for treating or/and preventing related diseases mediated by a sodium ion channel (NaV).

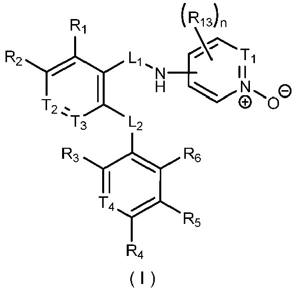

## Description

The present application claims the following priorities:
CN201910863718.6, filed on September 12, 2019;
CN201911094782.9, filed on November 11, 2019;
CN202010531381.1, filed on June 11, 2020;
CN202010923311.0, filed on September 4, 2020.

### Technical field

The present disclosure belongs to the field of medicinal chemistry. Specifically, the present disclosure relates to a new compound or a stereoisomer, a racemate, a geometric isomer, a tautomer, a prodrug, a hydrate, a solvate or a pharmaceutically acceptable salt thereof, and a pharmaceutical composition containing them, which are voltage-gated sodium channels (NaV) blockers with new structures.

### Background

Pain is one of the most common clinical symptoms, and is the fifth vital sign after respiration, pulse, blood pressure and body temperature, which seriously affects the life quality of patients. According to statistics, the global analgesic market was about US $36 billion in 2018 and is expected to reach US $56 billion in 2023. Wherein, the acute moderate to severe cases mainly rely on opioids, which account for about two-thirds of the analgesic market share, and will grow steadily at a compound annual growth rate of 2.5% in the future. The number of chronic pain patients, mainly neuropathic pain and arthritis pain, is increasing year by year, the market is expected to show a compound annual growth rate of about 18%, which will be the main driving force for the continued growth of the global pain market in the next decade.

Neuropathic pain is a chronic pain caused by damage or disease of the peripheral somatosensory nervous system, its symptoms include spontaneous pain and hypersensitivity to normal harmless stimuli. Common causes of neuropathic pain include: diabetes, herpes zoster, spinal cord injury, stroke, multiple sclerosis, cancer, HIV infection, lumbar or cervical radiculoneuropathy, trauma or postoperative nerve damage, etc. Osteoarthritis, also known as degenerative arthritis, is the degradation of bone and articular cartilage caused by a variety of factors, which can cause unevenness on the surface of the joint bone, and may form bone spurs, the clinical manifestations are mainly joint pain and joint stiffness. Long-term pain not only affects patients' ability to sleep, work and live, but also increases the incidence of emotional disorders such as depression or anxiety, thus bringing heavy economic burden to patients' families and society.

According to data released by the International congress on Neuropathic Pain (NeuPSIG), the prevalence of neuropathic pain is approximately 3.3%-8.2%. According to this calculation, there are at least 50 million patients in China alone. In 2017, there were 30.5 million patients with neuropathic pain in the United States, Japan, and the five major markets in Europe (France, Germany, Italy, Spain, and the United Kingdom), and the number is increasing year by year. Neuropathic pain is one of the most difficult diseases to treat, and most of the current treatment options still cannot achieve satisfactory results. It has been reported that only 14.9% of outpatients can alleviate pain in time through drug treatment, that is, about 85% of pain patients do not receive timely and effective drug treatment, so some patients have to seek surgical interventions. At present, the first-line drugs used clinically for the treatment of neuropathic pain are mainly calcium ion channel modulators (such as pregabalin and gabapentin), tricyclic antidepressants, 5-hydroxytryptamine and norepinephrine reuptake inhibitors (such as duloxetine, venlafaxine and other anticonvulsant and antidepressant drugs). These drugs have limited efficacy and are accompanied by various adverse reactions. Duloxetine is one of the first-line drugs used in the treatment of neuropathic pain, the main side effects include gastrointestinal reactions, nausea, drowsiness, dry mouth, hyperhidrosis and dizziness, etc, the resulting withdrawal rate reaches 15%-20%. The antiepileptic drugs gabapentin and pregabalin are the main drugs for the treatment of neuropathic pain, which can cause dizziness, drowsiness, peripheral edema, weight gain, weakness, headache and dry mouth and many other adverse reactions. In recent years, it has also been found that pregabalin can cause suicidal ideas and self-harm behaviors related to drug use in a very small number of patients.

The number of osteoarthritis patients is huge, it is estimated that there are more than 400 million osteoarthritis patients worldwide, and the number of patients in China has exceeded 100 million. There is currently no effective treatment for osteoarthritis pain. Clinically, there are physiotherapy, drug therapy and surgical treatment. Physiotherapy includes hyperthermia, hydrotherapy, ultrasound, massage, etc, in addition, assistive appliances can reduce joint pressure and relieve pain, but the effects are limited, and most of them still need to rely on drugs for treatment. These drugs all have varying degrees of side effects. Non-steroidal anti-inflammatory drugs are only suitable for mild to moderate pain, and have gastrointestinal side effects and cardiovascular and cerebrovascular risks. Opioid analgesics are used for severe pain, but have obvious side effects such as nausea and vomiting, constipation and drug dependence, and are not suitable for long-term use. Therefore, the development of new mechanisms targeting new targets and safe and effective analgesics to meet unmet clinical needs has important economic and social significance.

Research results in recent years have gradually revealed that sodium ion channel subtype 1.8 (NaV1.8) plays an important role in the occurrence and transmission of pain. NaV1.8 is a voltage-gated sodium ion channel mainly expressed on afferent neurons including sensory neurons, and plays an important role in maintaining the excitability of nociceptive neurons, the issuance and persistence of action potentials, and the regulation of nociceptive sensitivity by controlling the entry and exit of sodium ions into and out of cells. Patients with NaV1.8 activated mutations present with paroxysmal pain caused by small fiber neuropathy (damage to Aδ fibers and unmyelinated C-type fibers, which are primarily responsible for nociceptive transmission). Diseases such as chronic inflammation and diabetes can increase the expression of NaV1.8 or change its properties to sensitize nociceptive neurons and cause a variety of pain. While NaV1.8 knockout mice were insensitive to pain.

With the determination of the position of Nav1.8 in chronic pain, drug research based on this target has become increasingly hot, at present, there is one small molecule blocker in clinical phase 2 internationally, and many other small-molecule blockers and antibodies are in pre-clinical development, while there is no other new drug research and development for this target in China. Vertex's small molecule NaV1.8 blocker VX-150 is at the forefront of development, which has been tested in phase 2 clinical trials in patients with osteoarthritis, acute pain, and pain due to small fiber neuropathy, all three studies have received positive results, showing that inhibition of NaV1.8 activity can relieve a wide range of pain, including neuropathic pain. At present, VX-150 has been approved by the US FDA as a breakthrough therapy for the treatment of moderate to severe pain, which once again proves that NaV1.8 is a promising target for analgesia. In addition, the mechanism of action of NaV1.8 blocker and Phase II clinical trials show that it has a wide range of adaptations, including neuropathic pain, osteoarthritis pain and acute injury pain, etc.; and it has relatively high safety, no addiction, no gastrointestinal side effects of non-steroidal anti-inflammatory drugs and no cardiovascular side effects; it can be used in combination with other analgesics to enhance the efficacy and reduce side effects.

In recent years, studies have shown that sodium ion channel subtype 1.8 (NaV1.8) has a certain regulatory effect on cough, and NaV1.8 blockers may be used as potential drugs for the treatment of cough.

### Content of the present invention

Through repeated experimental studies, the inventors of the present disclosure have rationally designed and synthesized a series of small molecule compounds with new structures as shown in the following general formula (I) having high sodium channel 1.8 (NaV1.8) blocking activity. The compounds or a stereoisomer, a racemate, a geometric isomer, a tautomer, a prodrug, a hydrate, a solvate, or a pharmaceutically acceptable salt thereof and a pharmaceutical composition can be used for treating or/and preventing related diseases mediated by NaV1.8.

The compound of the present disclosure has high NaV1.8 blocking activity and provides a new treatment option for the treatment of pain and other diseases.

The present disclosure provides a compound as shown in formula (I), an optical isomer thereof or a pharmaceutically acceptable salt thereof, 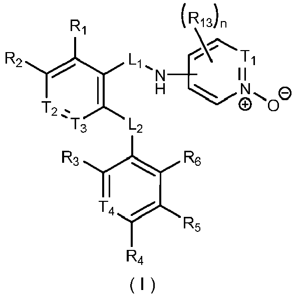 wherein,
T₁ is selected from N or C(R₇);
T₂ is selected from N or C(R₈);
T₃ is selected from N or C(R₉);
T₄ is selected from N or C(R₁₀);
R₁, R₂, R₈, R₉ are each independently selected from H, halogen, OH, NH₂, CN, SF₅, C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-O-, phenyl-C₁₋₃ alkyl-, C₃₋₆ cycloalkyl-C₁₋₃ alkyl-O-, 3-6 membered heterocycloalkyl-C₁₋₃ alkyl-O-, phenyl-C₁₋₃ alkyl-O-, phenyl-C₁₋₃ alkyl-NH-, 5-6 membered heteroaryl-C₁₋₃ alkyl-, 5-6 membered heteroaryl-C₁₋₃ alkyl-O- and 5-6 membered heteroaryl-C₁₋₃ alkyl-NH-, the C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-O-, phenyl-C₁₋₃ alkyl-, C₃₋₆ cycloalkyl-C₁₋₃ alkyl-O-, 3-6 membered heterocycloalkyl-C₁₋₃ alkyl-O-, phenyl-C₁₋₃ alkyl-O-, phenyl-C₁₋₃ alkyl-NH-, 5-6 membered heteroaryl-C₁₋₃ alkyl-, 5-6 membered heteroaryl-C₁₋₃ alkyl-O- or 5-6 membered heteroaryl-C₁₋₃ alkyl-NH- is optionally substituted by 1, 2 or 3 R;
and, when T₃ is N, R₁ is not H;
R₃, R₄, R₅, R₆, R₁₀ are each independently selected from H, halogen, OH, NH₂, SF₅, CN, C₁₋₆ alkyl, C₁₋₆ alkylamino, C₁₋₆ alkoxy, C₃₋₆ cycloalkayl, -O-C₃₋₆ cycloalkyl, 3-6 membered heterocycloalkyl, C₃₋₆ cycloalkyl-C₁₋₆ alkyl- and 3-6 membered heterocycloalkyl-C₁₋₆ alkyl-, the C₁₋₆ alkyl, C₁₋₆ alkylamino, C₁₋₆ alkoxy, C₃₋₆ cycloalkayl, -O-C₃₋₆ cycloalkyl, 3-6 membered heterocycloalkyl, C₃₋₆ cycloalkyl-C₁₋₆ alkyl- or 3-6 membered heterocycloalkyl-C₁₋₆ alkyl- is optionally substituted by 1, 2 or 3 R;
R₇ is selected from H, F, Cl, Br, I, C₁₋₆ alkyl, C₁₋₆ alkoxy and C₁₋₆ alkylamino, the C₁₋₆ alkyl, C₁₋₆ alkoxy or C₁₋₆ alkylamino is optionally substituted by 1, 2 or 3 R;
L₁ is selected from C(=O), NH and 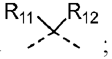
L₂ is selected from O, S, NH and CH₂, the CH is optionally substituted by 1 or 2 R, and the NH is optionally substituted by R;
R₁₁, R₁₂ are each independently selected from H, halogen, OH, NH₂ and C₁₋₆ alkyl, the C₁₋₆ alkyl is optionally substituted by 1, 2 or 3 R; or, R₁₁ and R₁₂ are connected together to form a 3-6 membered ring;
each of R₁₃ is independently selected from H, halogen and C₁₋₆ alkyl, the C₁₋₆ alkyl is optionally substituted by 1, 2 or 3 R;
n is selected from 1, 2 or 3;
each of R is independently selected from H, D, halogen, OH, NH₂, CN, 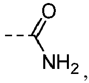 C₁₋₆ alkyl, C₁₋₆ alkoxy, C₁₋₆ alkylthio and C₁₋₆ alkylamino, the C₁₋₆ alkyl, C₁₋₆ alkoxy, C₁₋₆ alkylthio or C₁₋₆ alkylamino is optionally substituted by 1, 2 or 3 R';
R' is selected from F, Cl, Br, I, OH, NH₂ and CH₃;
the 3-6 membered heterocycloalkyl or 5-6 membered heteroaryl contains 1, 2 or 3 heteroatoms or heteroatom groups independently selected from -O-, -NH-, -S-, -C(=O)-, -C(=O)O-, -S(=O)-, -S(=O)₂- and N.

In some embodiments of the present disclosure, R is selected from H, D, F, Cl, Br, I, OH, NH₂, 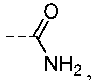 Me, CF₃, CHF₂, CH₂F, 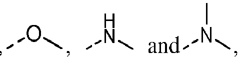 and the other variables are as defined in the present disclosure.

In some embodiments of the present disclosure, Ri, R₂, R₈, R₉ are each independently selected from H, halogen, OH, NH₂, CN, SF₅, C₁₋₃ alkyl, C₁₋₃ alkoxy, C₁₋₃ alkylamino, vinyl-C₁₋₃ alkyl-, C₃₋₆ cycloalkyl, 3-6 membered heterocycloalkyl, C₃₋₆ cycloalkyl-C₁₋₃ alkyl-, 3-6 membered heterocycloalkyl- C₁₋₃ alkyl-, 3-6 membered heterocycloalkyl-C₁₋₃ alkyl-O-, phenyl-C₁₋₃ alkyl-, phenyl-C₁₋₃ alkyl-O-, phenyl-C₁₋₃ alkyl-NH-, pyridyl-C₁₋₃ alkyl-, pyrimidinyl-C₁₋₃ alkyl-, thiophenyl-C₁₋₃ alkyl-, thiazolyl-C₁₋₃ alkyl-, pyrazolyl-C₁₋₃ alkyl-, imidazolyl-C₁₋₃ alkyl-, pyridyl-C₁₋₃ alkyl-O-, pyrimidinyl-C₁₋₃ alkyl-O-, thiophenyl-C₁₋₃ alkyl-O-, thiazolyl-C₁₋₃ alkyl-O-, pyrazolyl-C₁₋₃ alkyl-O-, imidazolyl-C₁₋₃ alkyl-O-, pyridyl-C₁₋₃ alkyl - NH-, pyrimidinyl-C₁₋₃ alkyl-NH-, thiophenyl-C₁₋₃ alkyl-NH-, thiazolyl-C₁₋₃ alkyl-NH-, pyrazolyl-C₁₋₃ alkyl -NH- and imidazolyl-C₁₋₃ alkyl-NH-, the C₁₋₃ alkyl, C₁₋₃ alkoxy, C₁₋₃ alkylamino, vinyl-C₁₋₃ alkyl-, C₃₋₆ cycloalkyl, 3-6 membered heterocycloalkyl, C₃₋₆ cycloalkyl-C₁₋₃ alkyl-, 3-6 membered heterocycloalkyl- C₁₋₃ alkyl-, 3-6 membered heterocycloalkyl-C₁₋₃ alkyl-O-, phenyl-C₁₋₃ alkyl-, phenyl-C₁₋₃ alkyl-O-, phenyl-C₁₋₃ alkyl-NH-, pyridyl-C₁₋₃ alkyl-, pyrimidinyl-C₁₋₃ alkyl-, thiophenyl-C₁₋₃ alkyl-, thiazolyl-C₁₋₃ alkyl-, pyrazolyl-C₁₋₃ alkyl-, imidazolyl-C₁₋₃ alkyl-, pyridyl-C₁₋₃ alkyl-O-, pyrimidinyl-C₁₋₃ alkyl-O-, thiophenyl-C₁₋₃ alkyl-O-, thiazolyl-C₁₋₃ alkyl-O-, pyrazolyl-C₁₋₃ alkyl-O-, imidazolyl-C₁₋₃ alkyl-O-, pyridyl-C₁₋₃ alkyl-NH-, pyrimidinyl-C₁₋₃ alkyl-NH-, thiophenyl-C₁₋₃ alkyl-NH-, thiazolyl-C₁₋₃ alkyl-NH-, pyrazolyl-C₁₋₃ alkyl-NH- or imidazolyl-C₁₋₃ alkyl-NH- is optionally substituted by 1, 2 or 3 R, and the other variables are as defined in the present disclosure.

In some embodiment of the present disclosure, R₁, R₂, R₈, R₉ are each independently selected from H, F, Cl, Br, I, OH, NH₂, CN, SF₅, Me, CF₃, CHF₂, CH₂F, CF₃CF₂, OCF₃, HOCH₂CH₂O, CH₃NHCH₂CH₂O, (CH₃)₂NCH₂CH₂O, 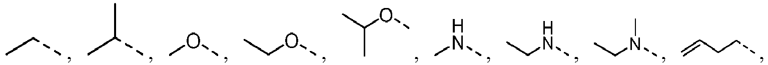 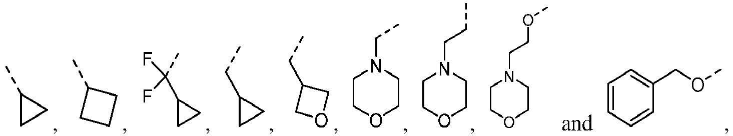 and the other variables are as defined in the present disclosure.

In some embodiments of the present disclosure, the structural moiety 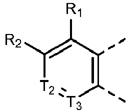 is selected from 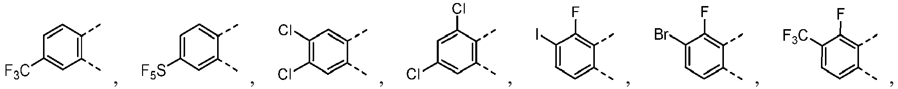 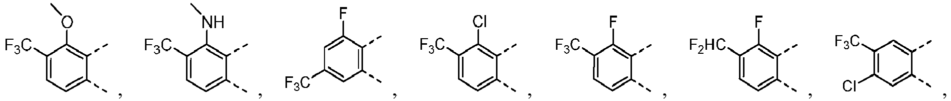 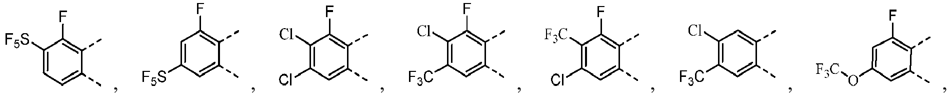 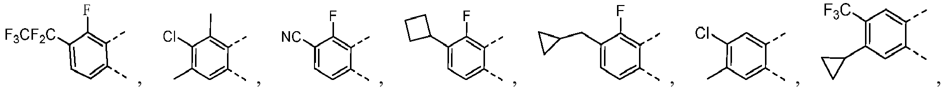 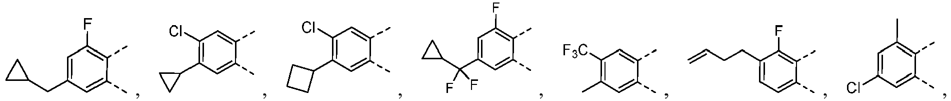 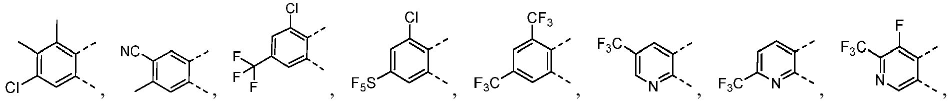 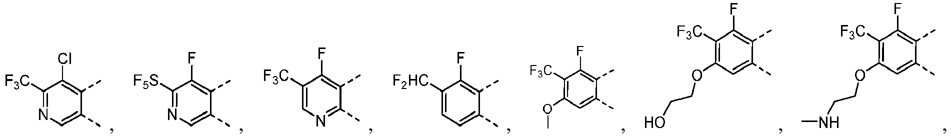 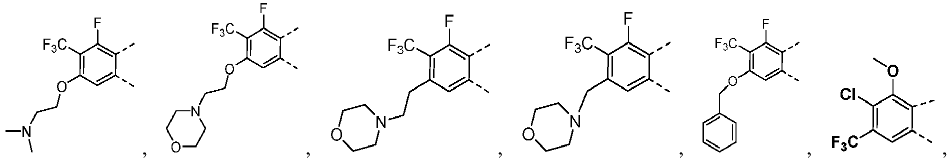 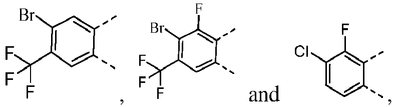 the other variables are as defined in the present disclosure.

In some embodiments of the present disclosure, R₃, R₄, R₅, R₆, R₁₀ are each independently selected from H, halogen, OH, NH₂, SF₅, CN, C₁₋₃ alkyl, C₁₋₃ alkylamino, C₁₋₃ alkoxy, C₃₋₆ cycloalkayl, -O-C₃₋₆ cycloalkyl, 3-6 membered heterocycloalkyl, C₃₋₆ cycloalkyl-C₁₋₃ alkyl- and 3-6 membered heterocycloalkyl-C₁₋₃ alkyl-, the C₁₋₃ alkyl, C₁₋₃ alkylamino, C₁₋₃ alkoxy, C₃₋₆ cycloalkayl, -O-C₃₋₆ cycloalkyl, 3-6 membered heterocycloalkyl, C₃₋₆ cycloalkyl-C₁₋₃ alkyl- or 3-6 membered heterocycloalkyl-C₁₋₃ alkyl- is optionally substituted by 1, 2 or 3 R, the other variables are as defined in the present disclosure.

In some embodiment of the present disclosure, R₃, R₄, R₅, R₆, R₁₀ are each independently selected from H, F, Cl, Br, I, OH, NH₂, SF₅, Me, CF₃, CHF₂, CH₂F, CN, CH(F₂)CH₃, CD₃, OCD₃, 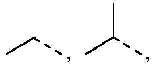 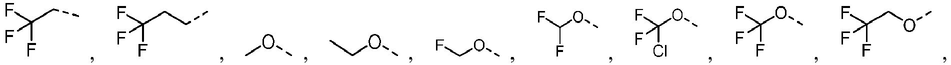 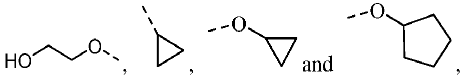 and the other variables are as defined in the present disclosure.

In some embodiments of the present disclosure, the structural moiety 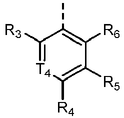 is selected from 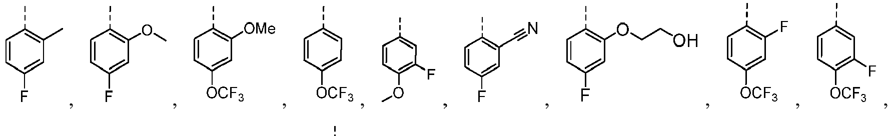 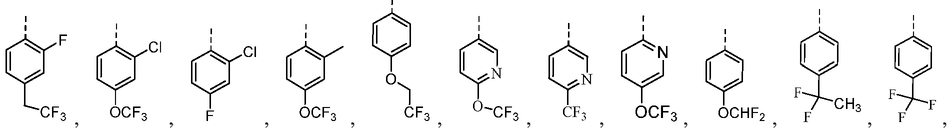 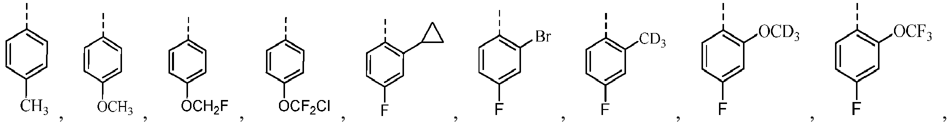 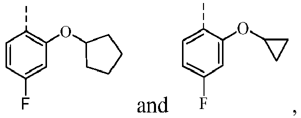 the other variables are as defined in the present disclosure.

In some embodiments of the present disclosure, the structural moiety 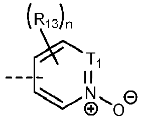 is selected from 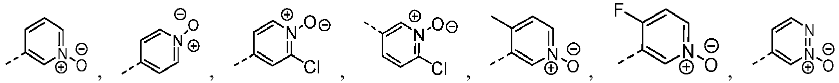 and 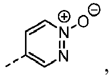 and the other variables are as defined in the present disclosure.

In some embodiments of the present disclosure, R₁₁, R₁₂ are each independently selected from H, F, Cl, Br, I, OH, NH₂, Me, CHF₂, CF₃, 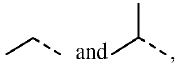 and the other variables are as defined in the present disclosure.

In some embodiments of the present disclosure, R₁₁ and R₁₂ are connected together to form a cyclopropyl, oxetanyl, azetidinyl and cyclopentanonyl, and the other variables are as defined in the present disclosure.

In some embodiments of the present disclosure, L₁ is selected from C(=O), CH₂, NH, CH(CH₃), CHF, CF₂, CHCHF₂, CHCF₃, 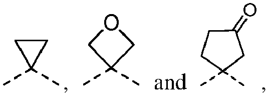 and the other variables are as defined in the present disclosure.

In some embodiments of the present disclosure, the compound , the optical isomer thereof or the pharmaceutically acceptable salt thereof is selected from 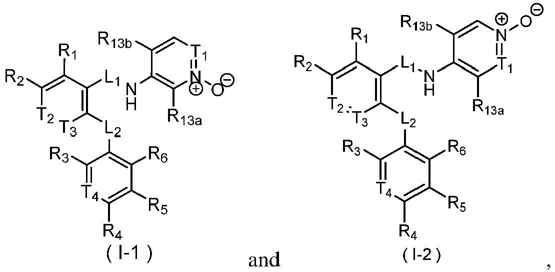 wherein,
T₁, R₁, R₂, T₂, T₃, R₃, R₄, R₈, R₆, T₄, L₁, L₂, R are as defined above;
R₁₃ₐ, R_{13b} are each independently selected from H, halogen and C₁₋₆ alkyl, the C₁₋₆ alkyl is optionally substituted by 1, 2 or 3 R.

In some embodiments of the present disclosure, R₁₃ₐ, R_{13b} are each independently selected from H, F, Cl, Br, I and Me, and the other variables are as defined in the present disclosure.

The present disclosure further provides a compound as shown in the formula below, a optical isomer thereof or a pharmaceutically acceptable salt thereof selected from

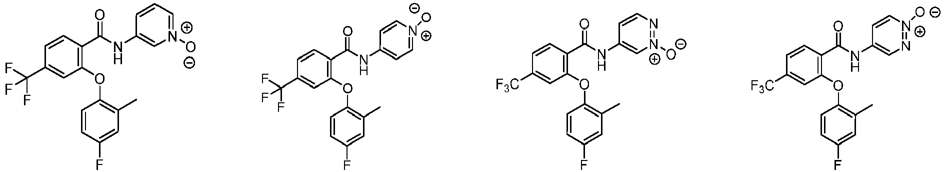

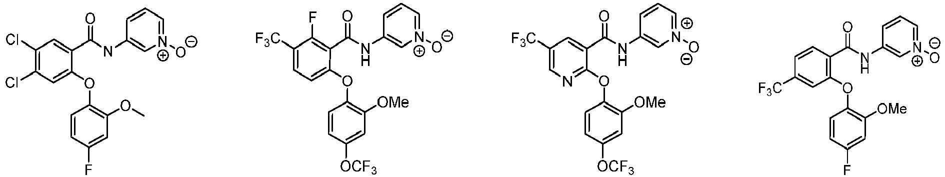

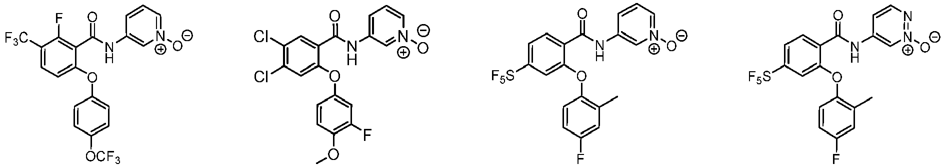

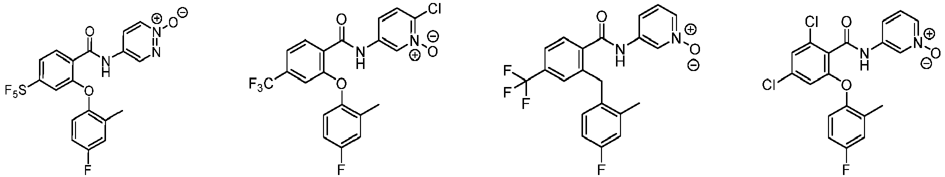

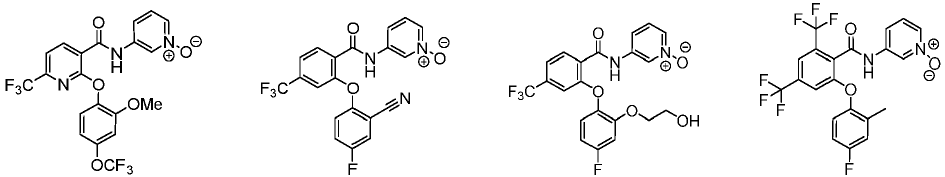

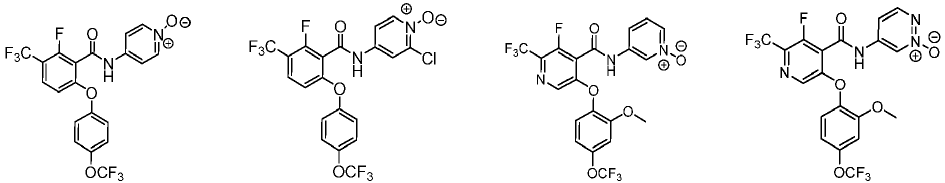

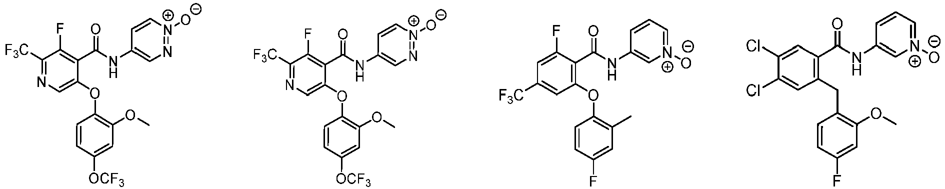

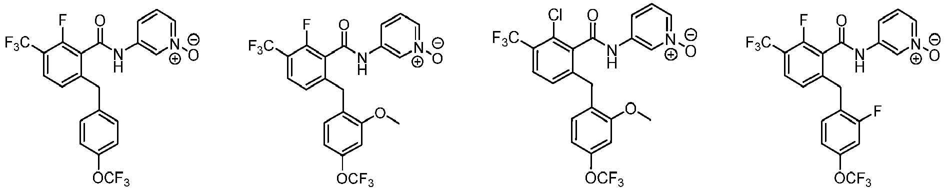

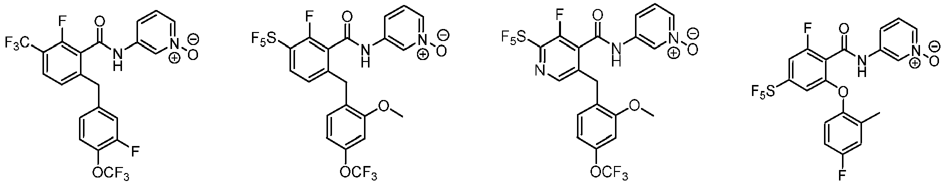

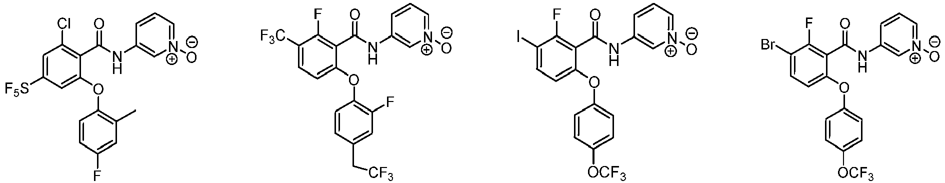

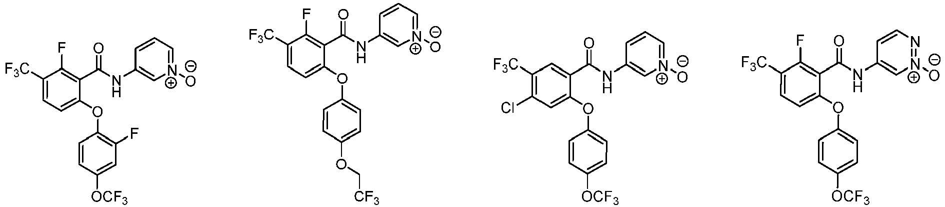

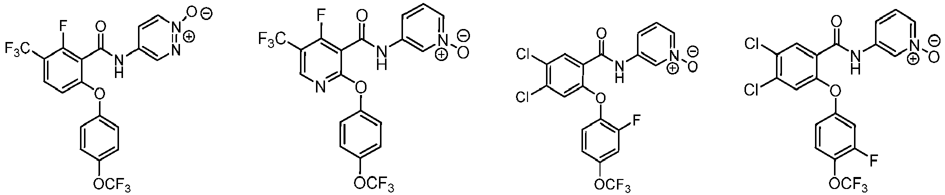

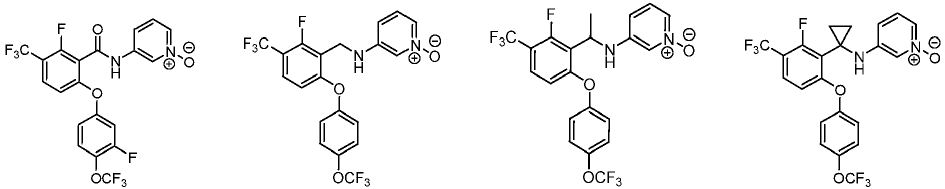

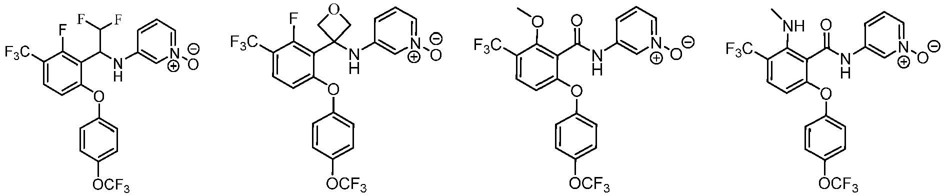

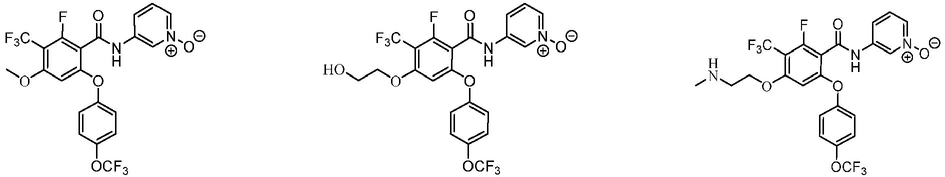

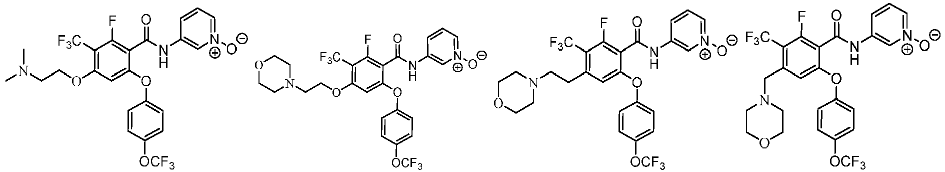

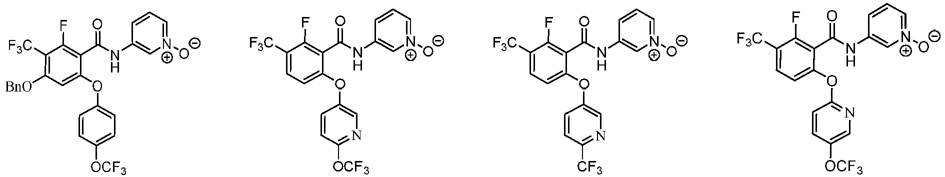

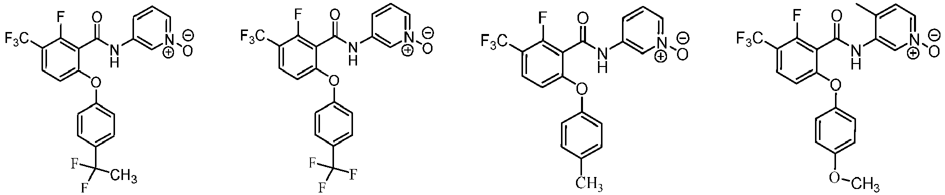

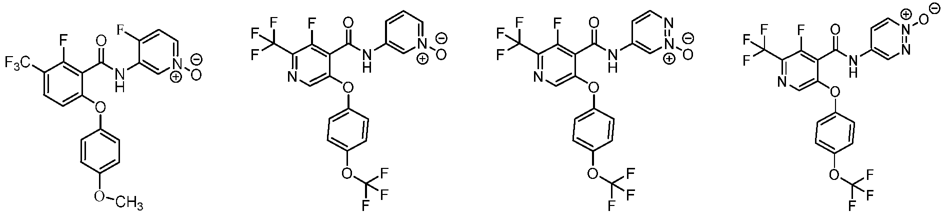

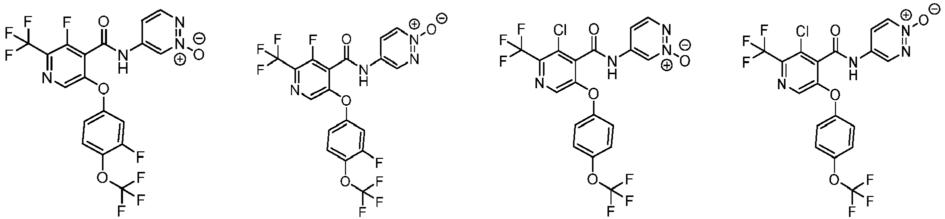

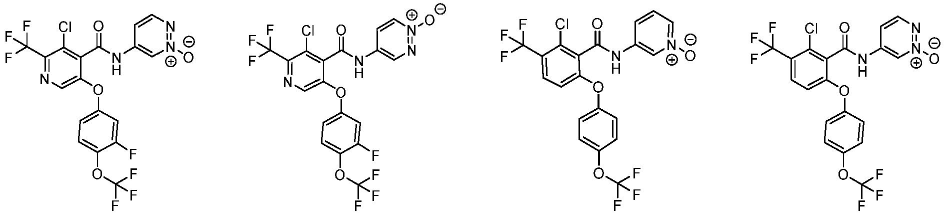

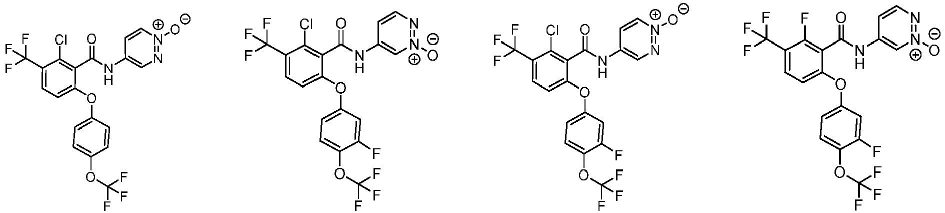

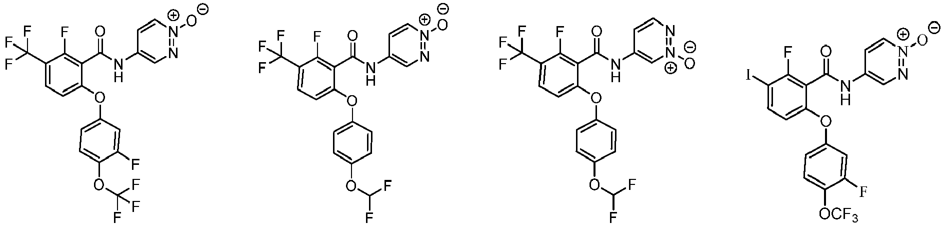

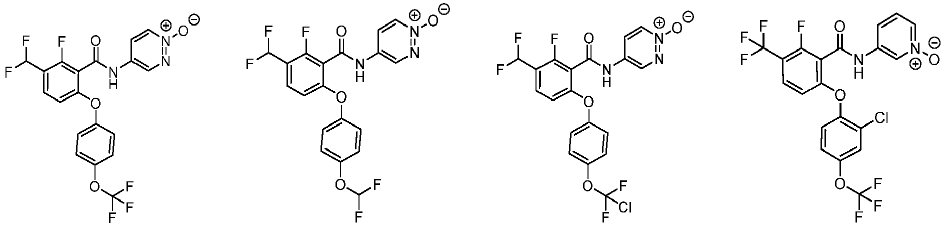

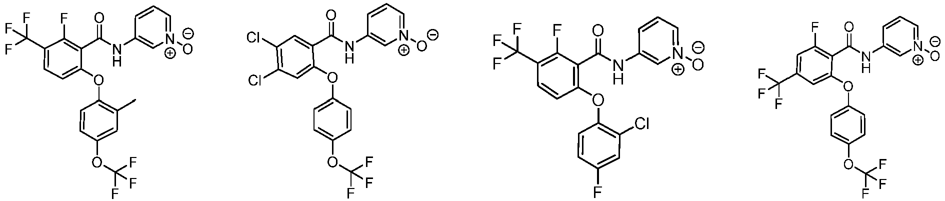

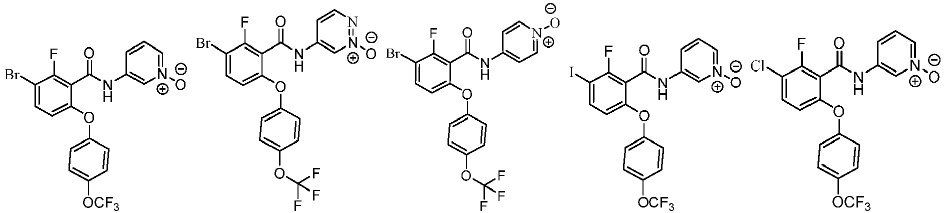

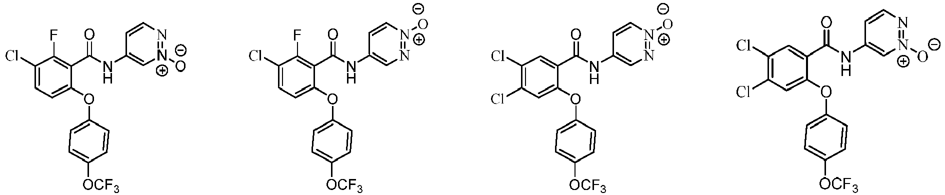

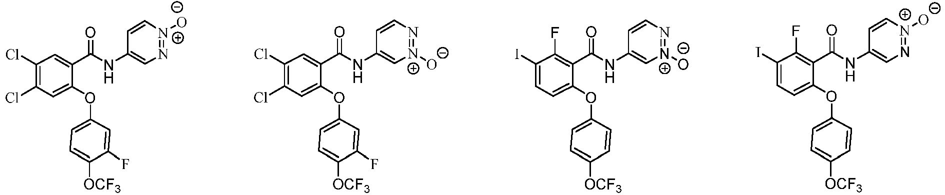

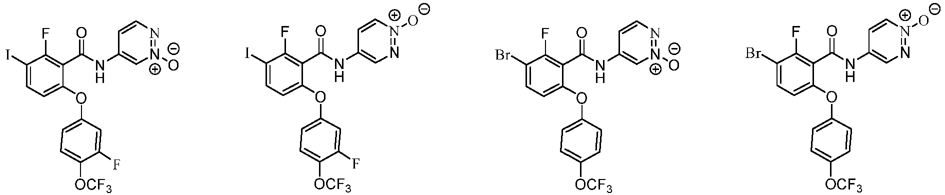

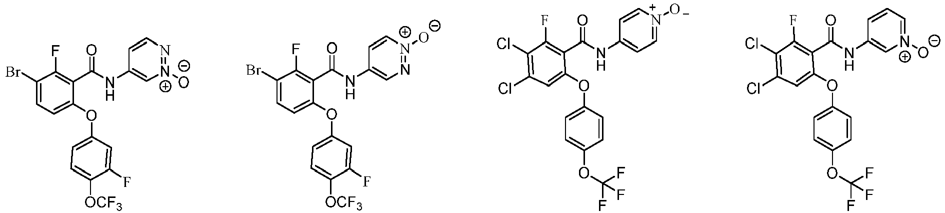

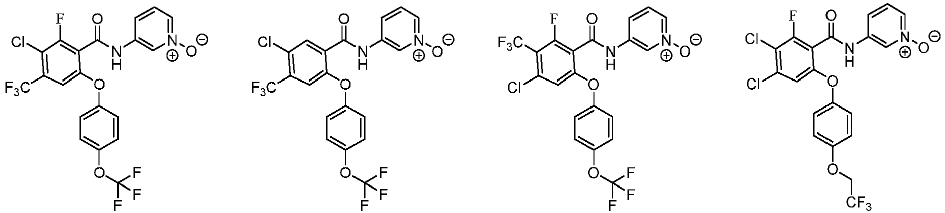

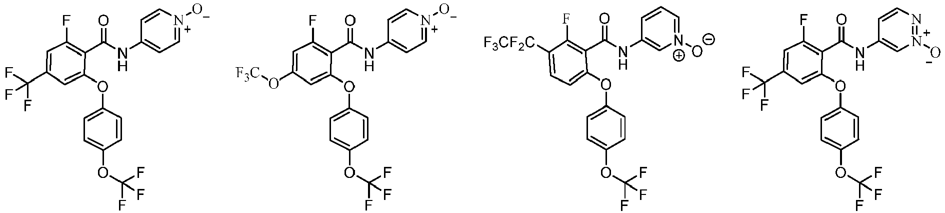

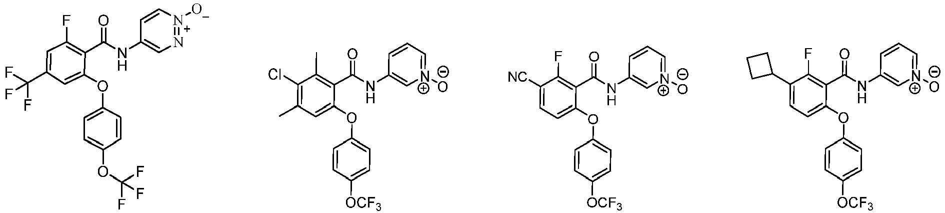

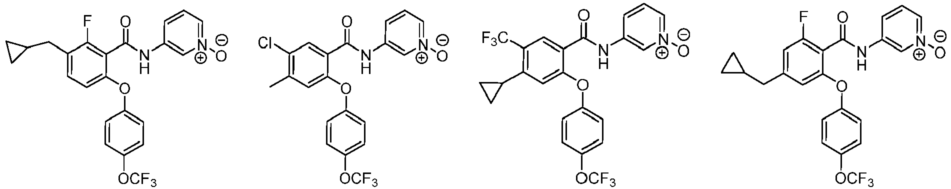

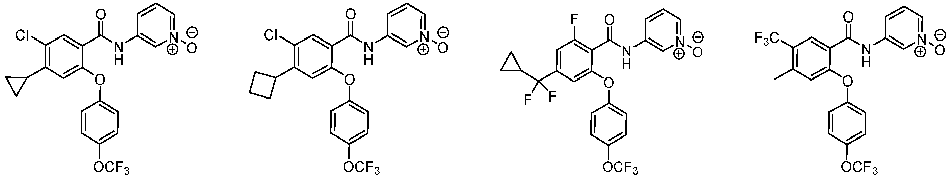

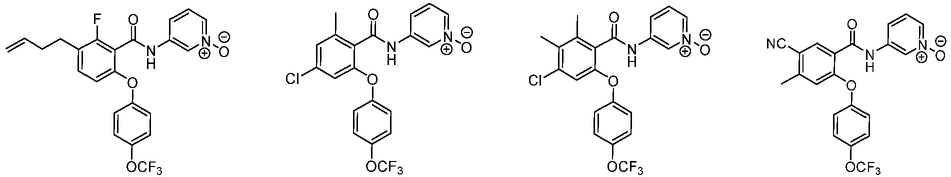

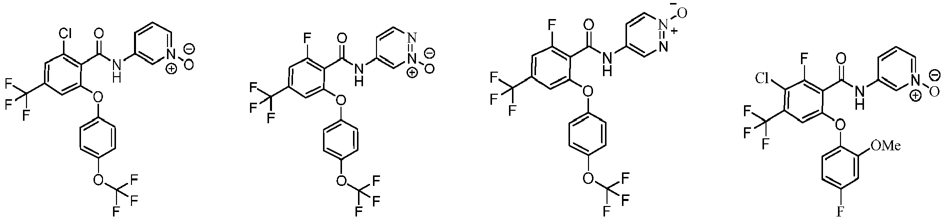

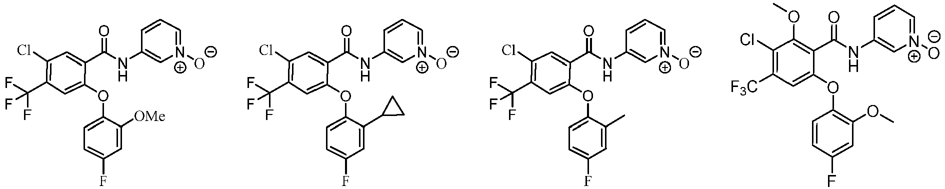

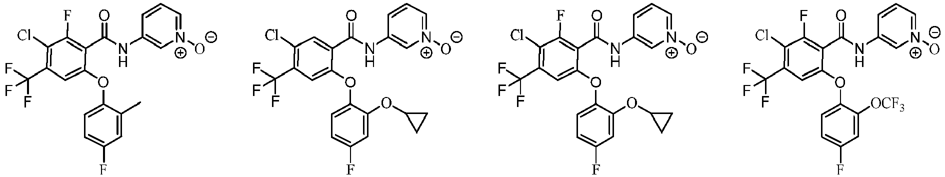

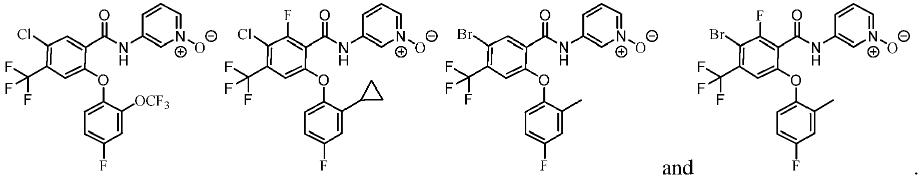

In another aspect of the present disclosure, the present disclosure also discloses a pharmaceutical composition. In some embodiments of the present disclosure, the pharmaceutical composition comprises the compound, the optical isomer thereof, and the pharmaceutically acceptable salt thereof.

In some embodiments of the present disclosure, the pharmaceutical composition further comprises a pharmaceutically acceptable carrier or excipient.

In another aspect of the present disclosure, the present disclosure also discloses a use of the compound, the optical isomer thereof and the pharmaceutically acceptable salt thereof, or the pharmaceutical composition in the manufacture of a medicament for inhibiting the voltage-gated sodium ion channel in an individual.

In some embodiments of the present disclosure, the voltage-gated sodium channel is Nav1.8.

In a further aspect of the present disclosure, the present disclosure also discloses a use of the compound, the optical isomer thereof and the pharmaceutically acceptable salt thereof, or the pharmaceutical composition in the manufacture of a medicament for treating and/or preventing pain or cough, or relieving the severity of pain or cough in an individual.

In some embodiments of the present disclosure, the pain is selected from chronic pain, intestinal pain, neuropathic pain, musculoskeletal pain, acute pain, inflammatory pain, cancer pain, primary pain, postoperative pain, visceral pain, multiple sclerosis, Charcot, Marfan and Down syndrome, incontinence and arrhythmia.

In some aspects of the present disclosure, the intestinal pain is selected from the inflammatory bowel disease pain, Crohn's disease pain and interstitial cystitis pain.

In some embodiments of the present disclosure, the neuropathic pain is selected from post-herpetic neuralgia, diabetic neuralgia, pain HIV-related sensory neuropathy, trigeminal neuralgia, mouth burn syndrome, post-amputation pain, phantom pain, painful neuroma, traumatic neuroma, Morto neuroma, nerve crush injury, spinal canal stenosis, carpal tunnel syndrome, radicular pain, sciatica, nerve avulsion, brachial plexus avulsion, complex regional pain syndrome, neuralgia caused by drug therapy, neuralgia caused by cancer chemotherapy, neuralgia caused by antiretroviral therapy, pain after spinal cord injury, primary small fiber neuropathy, primary sensory neuropathy and trigeminal autonomic headache.

In some embodiments of the present disclosure, the musculoskeletal pain is selected from osteoarthritis pain, back pain, cold pain, burn pain, and dental pain.

In some embodiments of the present disclosure, the inflammatory pain is selected from rheumatoid arthritis pain and vulvodynia.

In some embodiments of the present disclosure, the primary pain is selected from fibromyalgia.

In some embodiments of the present disclosure, one or more other therapeutic agents are administered simultaneously, before or after the administration of the compound, the optical isomer thereof and the pharmaceutically acceptable salt thereof, or the pharmaceutical composition.

In a further aspect of the present disclosure, the disclosure also provides a method for treating or alleviating pain in a subject. In some embodiments of the present disclosure, the method comprises administering a therapeutically effective amount of the compound, the optical isomer thereof, or the pharmaceutically acceptable salt thereof, or the pharmaceutical composition to the subject. In some embodiments of the present application, the pain of the subject is as defined in the present disclosure.

In a further aspect of the present disclosure, the disclosure also provides a method for inhibiting voltage-gated sodium channels in a subject. In some embodiments of the present disclosure, the method comprises administering a therapeutically effective amount of the compound, the optical isomer thereof, or the pharmaceutically acceptable salt thereof, or the pharmaceutical composition to the subject. In some embodiments of the present disclosure, the voltage-gated sodium channel is Nav1.8.

### Definition

The following terms and symbols used in this application have the following meanings, unless otherwise specified in the context.

A dash ("-") that is not between two letters or symbols indicates the connection site of the substituent. For example, C₁₋₆ alkylcarbonyl- refers to a C₁₋₆ alkyl that is attached to the rest of the molecule through a carbonyl. However, when the connection site of the substituent is obvious to those skilled in the art, for example, a halogen substituent, the "-" may be omitted.

When the valence bond of a group is marked with a dashed line " 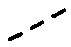 ", for example, in 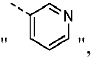 the wavy line indicates the connection site between the group and the other part of the molecule.

The term "hydrogen" as used herein refers to the group -H.

The term "deuterium" as used herein refers to the group -D.

The term "hydroxyl" as used herein refers to the group -OH.

The term "halogenated" or "halogen" as used herein refers to fluorine (F), chlorine (Cl), bromine (Br) and iodine (I).

The term "cyano" as used herein refers to the group -CN.

Unless otherwise specified, Cₙ₋ₙ₊ₘ or Cₙ₋Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered means that the number of atoms on the ring is from n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, and any range from n to n+m is also included, for example, 3-12 membered ring includes 3-6 membered ring, 3-9 membered ring, 5-6 membered ring, 5-7 membered ring, 6-7 membered ring, 6-8 membered ring, and 6-10 membered ring, etc.

Unless otherwise specified, the number of atoms in a ring is generally defined as the number of ring members. For example, "3-6 membered ring" refers to a "ring" in which 3 to 6 atoms are arranged around.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆ and C₅ alkyl groups, etc.; it can be monovalent (such as CH₃), divalent (such as -CH₂-) or multivalent (such as 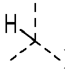 ). Examples of C₁₋₆ alkyl include, but are not limited to, CH₃, 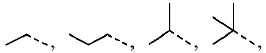 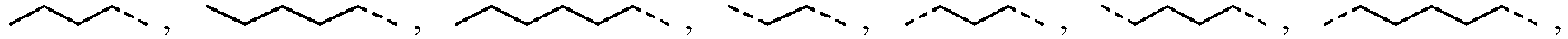 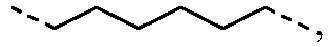 etc.

Unless otherwise specified, the term "C₁₋₃ alkyl" refers to a linear or branched saturated hydrocarbon group containing 1 to 3 carbon atoms. The C₁₋₃ alkyl includes C₁₋₂ and C₂₋₃ alkyl groups, etc.; it can be monovalent (such as CH₃), divalent (such as -CH₂-) or multivalent (such as 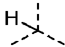 ). Examples of C₁₋₃ cycloalkyl include, but are not limited to, CH₃, 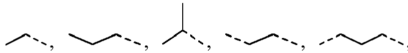 , etc.

Unless otherwise specified, the term "C₁₋₆ alkoxy" refers to those alkyl groups containing 1 to 6 carbon atoms connected to the rest of the molecule by one oxygen atom. The C₁₋₆ alkoxy includes C₁₋₄, C₁₋₃, C₁₋₂, C₂₋₆, C₂₋₄, C₆, C₅, C₄ and C₃ alkoxy groups, etc. Examples of C₁₋₆ alkoxy groups include, but are not limited to, methoxy, ethoxy, propoxy (including *n*-propoxy and isopropoxy), butoxy (including *n*-butoxy, isobutyoxy, s-butoxy and *t*-butoxy), pentoxy (including *n*-pentoxy, isopentoxy and neopentoxy), hexoxy, etc.

Unless otherwise specified, the term "C₁₋₃ alkoxy" refers to those alkyl groups containing 1 to 3 carbon atoms that are connected to the rest of the molecule through an oxygen atom. The "C₁₋₃ alkoxy" includes C₁₋₂, C₂₋₃, C₃ and C₂ alkoxy, etc. Examples of C₁₋₃ alkoxy include, but are not limited to, methoxy, ethoxy, propoxy (including *n*-propoxy and isopropoxy), etc.

Unless otherwise specified, the term "C₁₋₆ alkylamino" refers to those alkyl groups containing 1 to 6 carbon atoms that are connected to the rest of the molecule through an amino. The C₁₋₆ alkylamino includes C₁₋₄, C₁₃, C₁₋₂, C₂₋₆, C₂₋₄, C₆, C₅, C₄, C₃ and C₂ alkylamino, etc. Examples of C₁₋₆ alkylamino include, but are not limited to, -NHCH₃, -N(CH₃)₂, -NHCH₂CH₃, -N(CH₃)CH₂CH₃, -N(CH₂CH₃)(CH₂CH₃), -NHCH₂CH₂CH₃, -NHCH₂(CH₃)₂, -NHCH₂CH₂CH₂CH₃, etc.

Unless otherwise specified, the term "C₁₋₃ alkylamino" refers to those alkyl groups containing 1 to 3 carbon atoms that are connected to the rest of the molecule through an amino. The "C₁₋₃ alkylamino" includes C₁₋₂, C₃ and C₂ alkylamino, etc. Examples of C₁₋₃ alkylamino include, but are not limited to, - NHCH₃, -N(CH₃)₂, -NHCH₂CH₃, -N(CH₃)CH₂CH₃, -NHCH₂CH₂CH₃, -NHCH₂(CH₃)₂,, etc.

Unless otherwise specified, the term "C₁₋₆ alkylthio" refers to those alkyl groups containing 1 to 6 carbon atoms connected to the rest of the molecule by a sulfur atom. The C₁₋₆ alkylthio includes C₁₋₄, C₁₋₃, C₁₋₂, C₂₋₆, C₂₋₄, C₆, C₅, C₄, C₃ and C₂ alkylthio, etc. Examples of C₁₋₆ alkylthio include, but are not limited to, -SCH₃, -SCH₂CH₃, -SCH₂CH₂CH₃, -SCH₂(CH₃)₂, etc.

Unless otherwise specified, the term "C₁₋₃ alkylthio" refers to those alkyl groups containing 1 to 3 carbon atoms connected to the rest of the molecule by a sulfur atom. The "C₁₋₃ alkylthio" includes C₁₋₃, C₁₋₂ and C₃ alkylthio, etc. Examples of C₁₃ alkylthio groups include, but are not limited to, -SCH₃, -SCH₂CH₃, -SCH₂CH₂CH₃, -SCH₂(CH₃)₂, etc.

Unless otherwise specified, "C₃₋₆ cycloalkyl" refers to a saturated cyclic hydrocarbon group consisting of 3 to 6 carbon atoms, which is a monocyclic system and bicyclic system, the C₃₋₆ cycloalkyl includes C₃₋₅, C₄₋₅ and C₅₋₆ cycloalkyl, etc.; and it may be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "3-6 membered heterocycloalkyl" by itself or in combination with other terms refers to a saturated cyclic group consisting of 3 to 6 ring atoms, with 1, 2, 3 or 4 ring atoms being heteroatoms independently selected from O, S and N, and the rest are carbon atoms, wherein the N atom is optionally quaternized, and the N and S heteroatoms may be optionally oxidized (i.e., NO and S(O)ₚ, p is 1 or 2). It includes monocyclic and bicyclic systems, wherein the bicyclic system includes spiral rings, fused rings and bridged rings. In addition, for the "3- to 6- membered heterocycloalkyl", a heteroatom may occupy the connection position of the heterocycloalkyl with the rest of the molecule. The 3-6 membered heterocycloalkyl includes 4-6 membered, 5-6 membered, 4 membered, 5 membered and 6 membered heterocycloalkyl, etc. Examples of 3-6 membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl or homopiperidinyl, etc.

Unless otherwise specified, the term "3-6 membered ring" by itself or in combination with other terms refers to a saturated monocyclic group or an unsaturated monocyclic group consisting of 3 to 6 ring atoms, respectively, it may contain a pure carbon ring or a ring with heteroatoms. Wherein 3-6 refers to the number of atoms forming the ring. Examples of 3-6 membered ring include, but are not limited to, cyclopropyl, cyclobutyl, cyclopentyl, cyclohexyl, azetidinyl, oxetanyl, thietanyl, cyclopentanonyl, cyclohexanonyl, etc.

Unless otherwise specified, the term "5-6 membered heteroaromatic ring" and "5-6 membered heteroaryl" can be used interchangeably in the present disclosure, and the term "5-6 membered heteroaryl" refers to a monocyclic group with conjugated π electron system consisting of 5 to 6 ring atoms, of which 1, 2, 3 or 4 ring atoms are heteroatoms independently selected from O, S and N, and the rest are carbon atoms. Wherein the nitrogen atom is optionally quaternized, and the nitrogen and sulfur heteroatoms may be optionally oxidized (i.e., NO and S (O)ₚ, p is 1 or 2). 5-6 Membered heteroaryl may be connected to the rest of the molecule through a heteroatom or a carbon atom. The 5-6 membered heteroaryl includes 5 membered and 6 membered heteroaryl. Examples of the 5-6 membered heteroaryl include but are not limited to pyrrolyl (including *N*-pyrrolyl, 2-pyrrolyl, 3-pyrrolyl, etc.), pyrazolyl (including 2-pyrazolyl, 3-pyrazolyl, etc.), imidazol (including *N*-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1*H*-1,2,3-triazolyl, 2*H*-1,2,3-triazolyl, IH-1,2,4-triazolyl and 4*H*-1,2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furanyl (including 2-furanyl and 3-furanyl, etc.), thiophenyl (including 2-thiophenyl and 3-thiophenyl, etc.), pyridyl (including 2-pyridyl, 3-pyridyl and 4-pyridyl, etc.), pyrazinyl or pyrimidinyl (including 2-pyrimidinyl and 4-pyrimidinyl, etc.).

Accordingly, the term "heteroaromatic ring" as used herein refers to a ring of heteroaryl as defined above.

As used herein, "aryl" and "aromatic" follow the Hückel's rule, wherein the number of π electrons is equal to 4*n* + 2, and *n* is zero or any positive integer up to 6.

The term "carbonyl" as used herein refers to the group - C(O)-, and can also be expressed as -CO-.

The term "amino" as used herein refers to the group -NH₂.

The term "optionally" as used herein refers to that the event described later may or may not occur, and the description includes the circumstances in which the event occurs and the circumstances in which the event does not occur. For example, "optionally substituted alkyl" refers to unsubstituted alkyl and substituted alkyl, wherein the alkyl is as defined herein. Those skilled in the art should understand that for any group containing one or more substituents, the group does not include any spatially impractical, chemically incorrect, synthetically infeasible and/or inherently unstable substitution mode.

As used herein, the term "substituted" or "substituted by" means that one or more hydrogen atoms on a given atom or group are substituted, for example, by one or more substituents selected from a given substituent group, provided that the normal valence of the given atom is not exceeded. When the substituent is oxo (i.e., =O), then two hydrogen atoms on a single atom are substituted by oxygen. Such combinations are permitted only when combinations of substituents and/or variables result in chemically correct and stable compounds. A chemically correct and stable compound mean that the compound is stable enough to be separated from the reaction mixture, the chemical structure of the compound can be determined, and then can be prepared into preparation with at least practical utility. For example, in the absence of an explicit list of substituents, the term "be substituted" or "substituted" as used herein refers to that one or more hydrogen atoms on a given atom or group are independently substituted by one or more, for example 1, 2, 3 or 4 substituents, and the substituent is independently selected from: deuterium (D), halogen, - OH, sulfhydryl, cyano, -CD₃, alkyl (preferably C₁₋₆ alkyl), alkoxy (preferably C₁₋₆ alkoxy), haloalkyl (preferably halogenated C₁₋₆ alkyl), haloalkoxy (preferably halogenated C₁₋₆ alkoxy), -C(O)NRₐR_{b} and -N(Rₐ)C(O)R_{b} and -C(O)OC₁₋₄ alkyl (wherein Rₐ and R_{b} are each independently selected from hydrogen, C₁₋₄ alkyl, halogenated C₁₋₄ alkyl), carboxyl (- COOH), cycloalkyl (preferably 3-8 membered cycloalkyl), heterocyclyl (preferably 3-8 membered heterocyclyl), aryl, heteroaryl, aryl-C₁₋₆ alkyl -, heteroaryl C₁₋₆ alkyl-, -OC₁₋₆ alkylphenyl, -C₁₋₆ alkyl-OH (preferably -C₁₋₄ alkyl-OH), -C₁₋₆ alkyl-SH, -C₁₋₆ alkyl-O-C₁₋₂, -C₁₋₆ alkyl-NH₂ (preferably -C₁₋₃ alkyl-NH₂), -N(C₁₋₆ alkyl)₂ (preferably -N(C₁₋₃ alkyl)₂), -NH(C₁₋₆ alkyl) (preferably -NH(C₁₋₃ alkyl)), -N(C₁₋₆ alkyl)(C₁₋₆ alkylphenyl), -NH(C₁₋₆ alkylphenyl), nitro, -C(O)OC₁₋₆ alkyl (preferably -C(O)OC₁₋₃ alkyl), -NHC(O)(C₁₋₆ alkyl), -NHC(O)(phenyl), -N(C₁₋₆ alkyl)C(O)(C₁₋₆ alkyl), -N(C₁₋₆ alkyl)C(O)(phenyl), -C(O)C₁₋₆ alkyl, -C(O)heteroaryl (preferably -C(O)-5-7 membered heteroaryl), -C(O)C₁₋₆ alkylphenyl, -C(O)C₁₋₆ halogenated alkyl, -OC(O)C₁₋₆ alkyl(preferably -OC(O)C₁₋₃ alkyl), alkylsulfonyl (for example, -S(O)₂-C₁₋₆ alkyl), alkylsulfinyl (-S(O)-C₁₋₆ alkyl), -S(O)₂-phenyl, -S(O)₂-C₁₋₆ halogenated alkyl, -S(O)₂NH₂, -S(O)₂NH(C₁₋₆ alkyl), -S(O)₂NH(phenyl), -NHS(O)₂(C₁₋₆ alkyl), - NHS(O)₂(phenyl) and -NHS(O)₂(C₁₋₆ halogenated alkyl), wherein the alkyl, cycloalkyl, phenyl, aryl, heterocyclyl and heteroaryl are each optionally further substituted by one or more substituents selected from halogen, -OH, -NH₂, cycloalkyl, 3-8 membered heterocyclyl, C₁₋₄ alkyl, C₁₋₄ halogenated alkyl-, -OC₁₋₄ alkyl, -C₁₋₄ alkyl-OH, -C₁₋₄ alkyl-O-C₁₋₄ alkyl, -OC₁₋₄ halogenated alkyl, cyano, nitro, -C(O)-OH, -C(O)OC₁₋₆ alkyl, -CON(C₁₋₆ alkyl)₂, -CONH(C₁₋₆ alkyl), -CONH₂, -NHC(O)(C₁₋₆ alkyl), -NH(C₁₋₆ alkyl)C(O)(C₁₋₆ alkyl), -SO₂(C₁₋₆ alkyl), -SO₂(phenyl), -SO₂(C₁₋₆ halogenated alkyl), -SO₂NH₂, -SO₂NH(C₁₋₆ alkyl), -SO₂NH(phenyl), -NHSO₂(C₁₋₆ alkyl), -NHSO₂(phenyl) and -NHSO₂(C₁₋₆ halogenated alkyl). When an atom or group is substituted by a plurality of substituents, the substituents may be the same or different.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

The term "pharmaceutically acceptable" as used herein refers to non-toxic, biologically tolerable and suitable for individual application.

The term "pharmaceutically acceptable salt" used herein refers to the non-toxic, biologically tolerable acid addition salt or base addition salt of the compound as shown in formula (I) suitable for administration to an individual, including but not limited to: acid addition salt formed by the compound as shown in formula (I) and inorganic acid, such as hydrochloride, hydrobromate, carbonate, bicarbonate, phosphate, sulfate, sulfite, nitrate, etc.; and acid addition salt formed by the compound as shown in formula (I) and organic acid, such as formate, acetate, malate, maleate, fumarate, tartrate, succinate, citrate, lactate, methanesulfonate, *p*-toluenesulfonate, 2-hydroxyethylsulfonate, benzoate, salicylate, stearate, and other salts formed with alkane dicarboxylic acid HOOC-(CH₂)ₙ-COOH (wherein n is 0-4). The "pharmaceutically acceptable salt" also includes base addition salts formed by compound as shown in formula (I) with an acidic group and pharmaceutically acceptable cations such as sodium, potassium, calcium, aluminum, lithium and ammonium.

In addition, if the compound described herein is obtained in the form of acid addition salt, the free base form may be obtained by alkalizing the solution of the acid addition salt. Conversely, if the product is in the form of a free base, the acid addition salt, especially the pharmaceutically acceptable acid addition salt, may be prepared according to the conventional procedure for preparing acid addition salts from basic compounds by dissolving the free base in a suitable solvent and treating the solution with acid. Those skilled in the art can determine various synthetic methods that can be used to prepare non-toxic pharmaceutically acceptable acid addition salts without undue experimentation.

Those skilled in the art should understand that some compounds of the formula as shown in formula (I) may have one or more chiral centers, so there are two or more stereoisomers. Therefore, the compounds of the present disclosure may exist in the form of single stereoisomers (e.g., enantiomers, diastereomers) and mixtures of any ratios such as racemate, and in an appropriate situation, may exist in the form of tautomer and geometric isomer thereof.

The term "stereoisomer" used herein refers to the compound with the same chemical composition but different in the spatial arrangement of atoms or groups. Stereoisomers include enantiomers, diastereomers and conformational isomers.

The term "enantiomer" used herein refers to two stereoisomers of compound that are non-superimposable mirror images of each other.

The term "diastereomer" used herein refers to stereoisomers with two or more chiral centers and the molecules are not mirror images of each other. Diastereomers have different physical properties, such as melting point, boiling point, spectral properties or biological activity. Mixtures of diastereomers can be separated by high-resolution analytical methods such as electrophoresis and chromatography such as HPLC.

The definition and practice of stereochemistry can be followed in S. P. Parker, McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active form, that is, they have the ability to rotate the plane of plane-polarized light. When describing optically active compounds, the prefixes D and L or R and S are used to indicate the absolute configuration of the molecule with respect to its chiral center. The prefixes d and 1 or (+) and (-) are used to refer to the symbol of the compound rotating plane-polarized light, where (-) or 1 refers to that the compound is levorotatory. Compounds prefixed with (+) or D are dextral. For a given chemical structure, the stereoisomers are the same except that they mirror each other. Specific stereoisomers can also be called enantiomers, and mixtures of such isomers are usually called enantiomer mixtures. The 50:50 mixture of enantiomers is called racemic mixture or racemate, which can occur in the absence of stereoselectivity or stereospecificity in chemical reactions or methods. The terms "racemic mixture" and "racemate" refer to an equimolar mixture of two enantiomers that do not have optical activity.

Racemic mixtures can be used in their own form or split into individual isomers. Through resolution, stereochemically pure compounds or mixtures enriched in one or more isomers can be obtained. Methods for separating isomers are well known (see Allinger n. L. and Eliel E. L., "topics in stereochemistry", Vol. 6, Wiley Interscience, 1971), including physical methods, such as chromatography using chiral adsorbents. Individual isomers in chiral form may be prepared from chiral precursors. Alternatively, the individual isomer may be obtained by chemical separation from the mixture by forming a diastereoisomeric salt with a chiral acid (e.g., single enantiomer of 10-camphorsulfonic acid, camphoric acid, α-bromocamphoric acid, tartaric acid, diacetyltartaric acid, malic acid, pyrrolidone-5-carboxylic acid, etc.), crystallizing the salt in stages, and then freeing one or two of the separated bases, optionally repeating the process, thereby obtaining one or two isomers substantially free of the other isomer, i.e., the desired stereoisomer with an optical purity of, for example, at least 91%, 92%, 93%, 94%, 95%, 96%, 97%, 98%, 99% or 99.5% by weight. Alternatively, as is well known to those skilled in the art, the racemate can be covalently attached to a chiral compound (auxiliary) to obtain diastereomers.

The term "tautomer" or "tautomeric form" used herein refers to structural isomers of different energies that can be transformed into each other through low-energy barriers. For example, proton tautomers (also known as proton transfer tautomers) include interconversion by proton migration, such as keto-enol and imino-enamine isomerization. Valence tautomer includes interconversion through the recombination of some bonded electrons.

The term "treatment" as used herein refers to the administration of one or more pharmaceutical substances, especially the compounds as shown in formula (I) and/or a pharmaceutically acceptable salt thereof, to an individual suffering from or having symptoms of the disease to cure, relieve, alleviate, change, treat, improve, ameliorate or affect the disease or the symptoms of the disease. The term "prevention" as used herein refers to the administration of one or more pharmaceutical substances, especially the compound as shown in formula (I) and/or the pharmaceutically acceptable salt thereof, to an individual with a constitution prone to the disease to prevent the individual from suffering from the disease. When a chemical reaction is involved, the terms "treat", "contact" and "reaction" refer to the addition or mixing of two or more reagents under appropriate conditions to produce the shown and/or desired product. It should be understood that the reaction to produce the shown and/or desired product may not necessarily come directly from the combination of the two reagents initially added, i.e. there may be one or more intermediates generated in the mixture, which eventually lead to the formation of the shown and/or desired product.

The term "effective amount" as used herein refers to an amount that is usually sufficient to produce a beneficial effect on an individual. The effective amount of the compound of the present disclosure can be determined by conventional methods (such as modeling, dose escalation study or clinical trial) in combination with conventional influencing factors (such as mode of administration, the pharmacokinetics of the compound, severity and course of the disease, individual's medical history, individual's health status, individual's response to the drug, etc.).

The technical and scientific terms used herein that are not specifically defined have the meanings commonly understood by those skilled in the art to which the present disclosure belongs.

### Detailed description of the embodiments

### Embodiment

The present disclosure will be further explained below with specific embodiments. It should be understood that these embodiments are only used to illustrate the present disclosure and not to limit the scope of the present disclosure. In the following embodiment, if no specific conditions are specified, the experimental methods usually follow the conventional conditions of this type of reaction or the conditions recommended by the manufacturer. Unless otherwise specified, percentages and parts are weight percentages and parts by weight. Unless otherwise specified, the ratio of liquids is a volume ratio.

The experimental materials and reagents used in the following embodiment can be obtained from commercial available sources unless otherwise specified.

In the following embodiment, the ¹H-NMR spectrum was recorded with the Bluker AVANCE III HD 400MHz nuclear magnetic resonance instrument; the ¹³C-NMR spectrum was recorded with the Bluker AVANCE III HD 400MHz nuclear magnetic resonance instrument, and the chemical shift was expressed in δ (ppm); mass spectrum was recorded with Agilent 1260 (ESI) or Shimadzu LCMS-2020 (ESI) or Agilent 6215 (ESI) mass spectrometer; reversed-phase preparative HPLC separation was performed with Agilent 1290 UV-guided automatic purification system (Xtimate^{®} Prep C18 OBDTM 21.2^{∗}250mm 10µm column) or Gilson GX281 UV-guided automatic purification system (xBridge^{®} Prep C18 OBDTM 192^{∗}50mm 10µm column) or Waters QDa-guided automatic purification system (SunFire^{®} Prep C18 OBD 29^{∗}250mm 10µm Column).

Wherein, the names of reagents represented by chemical formulas or English abbreviations are as follows:

Aq refers to aqueous solution; Ar refers to argon; BH₃ refers to borane; br refers to wide peak; B₂Pin₂ refers to bis(pinacolato)diboron; °C refers to degrees celsius; CD₃OD refers to deuterated methanol; CDCl₃ refers to deuterated chloroform; conc. refers to concentrated; (COCl)₂ refers to oxalyl chloride; Cs₂CO₃ refers to cesium carbonate; CuI refers to cuprous iodide; d refers to double peak; DCM refers to dichloromethane; dioxane or 1,4-dioxane refers to dioxane; DIPEA or DIEA refers to *N, N-*diisopropylethylamine; DMF refers to dimethylformamide; DMSO refers to dimethyl sulfoxide; EA or EtOAc refers to ethyl acetate; ESI refers to electrospray ionization; g refers to gram; h refers to hour; H₂O refers to water; HATU refers to 1-[bis(dimethylamino)methylene]-1*H*-1,2,3-triazolo[4,5-*b*]pyridinium 3-oxide hexafluorophosphate; HOBt refers to 1-hydroxybenzotriazole; HPLC refers to high performance liquid chromatography; K₂CO₃ refers to potassium carbonate; KOAc refers to potassium acetate; LCMS refers to liquid chromatography-mass spectrometry; LiOH refers to lithium hydroxide; m refers to multiple peaks; m/z refers to mass charge ratio; MeCN, ACN or CH₃CN refers to acetonitrile; *m*-CPBA refers to *m-*chloroperoxybenzoic acid; MeOH refers to methanol; min refers to minute; mg refers to milligram; mL refers to millilitre; mmol refers to millimole; N₂ refers to nitrogen; Na₂CO₃ refers to sodium carbonate; NaCl refers to sodium chloride; NaHCO₃ refers to sodium bicarbonate; NaOH refers to sodium hydroxide; Na₂SO₄ refers to sodium sulfate; NMP refers to *N*-methyl-2-pyrrolidone; PBr₃ refers to phosphorus tribromide; Pd(dppf)Cl₂ or PdCl₂(dppf) refers to [1,1'-bis(diphenylphosphino)ferrocene]dichloropalladium; PE refers to petroleum ether; r. t. or RT refers to room temperature; s refers to single peak; SOCl₂ refers to dichlorosulfoxide; t refers to triple peak; TLC refers to thin layer chromatography; THF refers to tetrahydrofuran; Toluene or tol. refers to toluene.

### Synthesis of embodiment A1

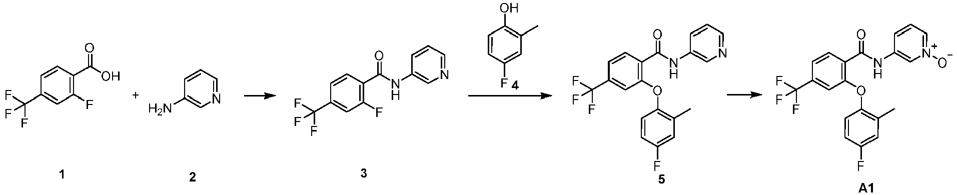

### Step 1, synthesis of intermediate 3

Compound **1** (2.2 g, 10.57 mmol) was added to DMF (10 mL), HATU (5.23 g, 13.75 mmol) was added thereto at 0°C, and the mixture was stirred for 10 min and compound **2** (1.09 g, 11.62 mmol) was added, then DIEA (1.78 g, 13.79 mmol) was slowly added dropwise, the reaction mixture was stirred at room temperature for 2h, LC-MS showed that the reaction was complete, H₂O (20 mL) was added to the reaction mixture, a solid precipitated out, then the mixture was filtered, the filter cake was the target compound, and was dried to obtain compound **3,** white solid, 2.2 g, yield: 73%. LCMS: m/z 285.0(M+H)⁺.

### Step 2, synthesis of intermediate 5

Compound **3** (500 mg, 1.76 mmol) was dissolved in NMP (5 mL), compound **4** (333 mg, 2.64 mmol) and K₂CO₃ (730 mg, 5.28 mmol) were added to the mixture, and the reaction mixture was stirred at 100°C overnight, LC-MS showed that the reaction was complete, H₂O (20 mL) was added to the reaction mixture, and the reaction mixture was extracted with EA (15 mL^{∗}3), the organic phase was combined, dried over anhydrous Na₂SO₄ and concentrated, and the crude product was purified by normal phase column (PE/EA =0-100%) to obtain compound **5,** yellow solid, 500 mg, yield: 72.8%. LCMS: m/z 391.1(M+H)⁺.

### Step 3, Synthesis of embodiment A1

Compound **5** (200 mg, 0.51 mmol) was dissolved in DCM (2 mL), *m*-CPBA (133 mg, 0.77 mmol) was added, and the reaction mixture was stirred at room temperature for 1 h, LC-MS showed that the reaction was complete. The pH value was adjusted by adding saturated NaHCO₃ to weakly basic, the mixture was extracted with EA (20 mL^{∗}2), the organic phase was combined, washed with saturated NaCl (20 mL), dried over anhydrous Na₂SO₄, and concentrated to obtain a crude product, the crude product was prepared (5-95% acetonitrile in water (containing 0.05% NH₄HCO₃)) to obtain compound **A1.** LCMS: m/z 407.2 (M+H) ⁺; ¹H NMR (400 MHz, DMSO-*d₆*) δ 10.93 (s, 1H), 8.68 (s, 1H), 8.01 (ddd, *J* = 6.4, 1.7, 0.9 Hz, 1H), 7.89 (d, *J* = 7.8 Hz, 1H), 7.62 (dd, *J* = 7.9, 0.8 Hz, 1H), 7.51 (d, *J* = 9.2 Hz, 1H), 7.39 (dd, *J* = 8.4, 6.4 Hz, 1H), 7.22 (dd, *J* = 9.2, 2.5 Hz, 1H), 7.13 - 7.05 (m, 2H), 7.01 (s, 1H), 2.17 (s, 3H).

Similar to the synthesis of embodiment **A1**, the following embodiments **A2-A40** were synthesized, as shown in Table 1 below:

**Table 1: Structural formula and analysis data of embodiments A2-A40**

| **Embodiment** | Structural formula | Analysis data |
|---|---|---|
| **A2** | 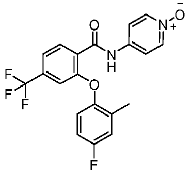 | LCMS: m/z 407.2 (M+H) ⁺; ¹H NMR (400 MHz, DMSO-*d₆*) δ 11.02 (s, 1H), 8.19 - 8.12 (m, 2H), 7.88 (d, *J* = 7.8 Hz, 1H), 7.67 (d, *J* = 7.5 Hz, 2H), 7.62 (dd, *J* = 7.9, 0.8 Hz, 1H), 7.21 (dd, *J* = 9.1, 2.3 Hz, 1H), 7.09 (dd, *J* = 6.8, 3.1 Hz, 2H), 7.00 (s, 1H), 2.16 (s, 3H). |
| **A3** | 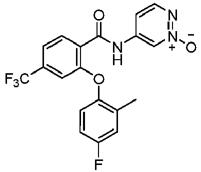 | LCMS: m/z 408.0 (M+H) ⁺; ¹H NMR (400 MHz, DMSO-*d₆*) δ 11.24 (s, 1H), 8.79 - 8.78 (d, *J* = 3.2 Hz, 1H), 8.28 - 8.26 (d, *J* = 6.8 Hz, 1H), 8.13 - 8.10 (m, 1H), 7.91 - 7.89 (d, *J* = 8.0 Hz, 1H), 7.64 - 7.62 (d, *J* = 8.0 Hz, 1H), 7.24 - 7.21 (m, 1H), 7.11 - 7.08 (m, 2H), 7.0 (s, 1H), 2.16 (s, 3H). |
| **A4** | 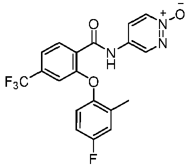 | LCMS: m/z 408.0 (M+H) ⁺; ¹H NMR (400 MHz, DMSO-*d₆*) δ 11.36 (s, 1H), 8.66 - 8.65 (d, *J* = 1.2 Hz, 1H), 8.47 - 8.46 (d, *J* = 6.4 Hz, 1H), 7.92 - 7.90 (d, *J* = 7.6 Hz, 1H), 7.65 - 7.63 (m, 1H), 7.43 - 7.41 (m, 1H), 7.24 - 7.21 (m, 1H), 7.14 - 7.07 (m, 2H), 7.01 (s, 1H), 2.16 (s, 3H). |
| **A5** | 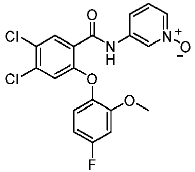 | LCMS: m/z 423.0 (M+H) ⁺; ¹H NMR (400 MHz, DMSO-*d₆*) δ 10.79 (s, 1H), 8.70 (s, 1H), 8.01 (dd, *J* = 6.3, 0.8 Hz, 1H), 7.94 (s, 1H), 7.53 (d, *J* = 8.7 Hz, 1H), 7.40 (dd, *J* = 8.4, 6.4 Hz, 1H), 7.26 (dd, *J* = 8.8, 5.8 Hz, 1H), 7.13 (dd, *J* = 10.7, 2.9 Hz, 1H), 6.89 (s, 1H), 6.86 - 6.77 (m, 1H), 3.75 (s, 3H). |
| **A6** | 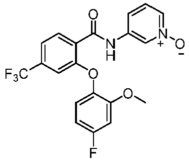 | LCMS: m/z 423.2 (M+H) ⁺; ¹H NMR (400 MHz, DMSO-*d₆*) δ 10.86 (s, 1H), 8.73 (s, 1H), 8.04 - 7.99 (m, 1H), 7.85 (d, *J* = 7.9 Hz, 1H), 7.60 - 7.52 (m, 2H), 7.40 (dd, *J* = 8.4, 6.4 Hz, 1H), 7.29 (dd, *J* = 8.8, 5.9 Hz, 1H), 7.15 (dd, *J* = 10.7, 2.9 Hz, 1H), 6.86 (dt, *J* = 8.6, 2.9 Hz, 2H), 3.75 (s, 3H). |
| **A7** | 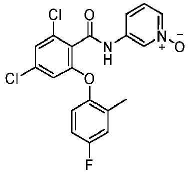 | LCMS: m/z 407.0 (M+H) ⁺; ¹H NMR (400 MHz, DMSO-*d₆*) δ 11.17 (s, 1H), 8.67 (t, *J* = 1.6 Hz, 1H), 8.07 - 7.97 (m, 1H), 7.54 (d, *J* = 1.8 Hz, 1H), 7.50 (dd, *J* = 8.5, 0.9 Hz, 1H), 7.43 - 7.35 (m, 1H), 7.25 - 7.19 (m, 1H), 7.12 (dd, *J* = 6.7, 3.3 Hz, 2H), 6.70 (d, *J* = 1.8 Hz, 1H), 2.14 (s, 3H). |
| **A8** | 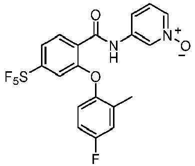 | LCMS: m/z 465.2 (M+H) ⁺; ¹H NMR (400 MHz, DMSO-*d₆*) δ 10.96 (s, 1H), 8.66 (s, 1H), 8.02 (ddd, *J* = 6.3, 1.6, 0.9 Hz, 1H), 7.91 (d, *J* = 8.4 Hz, 1H), 7.83 (dd, *J* = 8.5, 2.1 Hz, 1H), 7.49 (d, *J* = 9.2 Hz, 1H), 7.40 (dd, *J* = 8.4, 6.4 Hz, 1H), 7.23 (dd, *J* = 9.3, 2.8 Hz, 1H), 7.18 (d, *J* = 2.0 Hz, 1H), 7.14 - 7.03 (m, 2H), 2.17 (s, 3H). |
| **A9** | 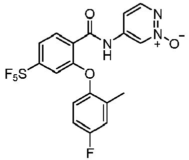 | LCMS: m/z 466.1 (M+H) ⁺; ¹H NMR (400 MHz, DMSO-*d₆*) δ 11.24 (s, 1H), 8.76 (d, *J* = 3.2 Hz, 1H), 8.28 (d, *J* = 7.1 Hz, 1H), 8.09 (dd, *J* = 7.1, 3.4 Hz, 1H), 7.99 - 7.76 (m, 2H), 7.24 (dd, *J* = 9.3, 2.8 Hz, 1H), 7.17 (d, *J* = 2.0 Hz, 1H), 7.15 - 7.05 (m, 2H), 2.16 (s, 3H). |
| **A10** | 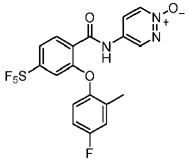 | LCMS: m/z 466.1 (M+H) ⁺; ¹H NMR (400 MHz, DMSO-*d₆*) δ 10.71 (s, 1H), 8.62 (d, *J* = 1.1 Hz, 1H), 8.45 (d, *J* = 6.2 Hz, 1H), 7.98 - 7.77 (m, 2H), 7.38 (dd, *J* = 6.3, 1.6 Hz, 1H), 7.24 (dd, *J* = 9.2, 2.8 Hz, 1H), 7.17 (d, *J* = 2.0 Hz, 1H), 7.14 - 7.03 (m, 2H), 2.17 (s, 3H). |
| **All** | 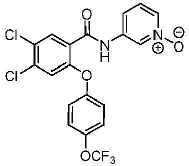 | LCMS: m/z 459.0 (M+H) ⁺; ¹H NMR (400 MHz, DMSO-*d₆*) *δ* 10.83 (s, 1H), 8.61 (s, 1H), 8.03 (s, 1H), 8.00 - 7.98 (m, 1H), 7.47 - 7.44 (m, 2H), 7.40 - 7.34 (m, 3H), 7.21- 7.16 (m, 2H). |
| **A12** | 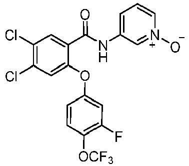 | LCMS: m/z 477.3 (M+H) ⁺; ¹H NMR (400 MHz, DMSO-*d₆*) *δ* 10.90 (s, 1H), 8.38 (s, 1H), 8.14 (s, 1H), 7.71 - 7.62 (m, 3H), 7.39 - 7.30 (m, 3H), 6.90 (s, 1H). |
| **A13** | 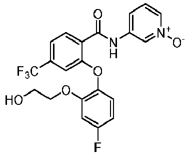 | LCMS: m/z 453.10 (M+H) ⁺; ¹H NMR (400 MHz, DMSO-*d₆*) δ 10.78 (s, 1H), 8.72 (d, *J* = 1.9 Hz, 1H), 8.02 (ddd, *J* = 6.4, 1.8, 0.9 Hz, 1H), 7.85 (d, *J* = 7.9 Hz, 1H), 7.63 - 7.48 (m, 2H), 7.40 (dd, *J* = 8.5, 6.3 Hz, 1H), 7.29 (dd, *J* = 8.9, 5.8 Hz, 1H), 7.17 (dd, *J* = 10.7, 2.9 Hz, 1H), 6.96 (d, *J* = 1.6 Hz, 1H), 6.89 - 6.77 (m, 1H), 4.83 (t, *J* = 5.4 Hz, 1H), 4.00 (t, *J* = 5.1 Hz, 2H), 3.53 - 3.48 (m, 2H). |
| **A14** | 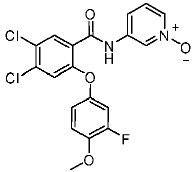 | LCMS: m/z 422.9 (M+H) ⁺; ¹H NMR (400 MHz, DMSO-*d₆*) δ 10.82 (s, 1H), 8.66 (s, 1H), 8.01-7.98 (m, 2H), 7.51-7.48 (d, *J* = 8.8 Hz, 1H), 7.40-7.37 (m, 1H), 7.22-7.13 (m, 3H), 6.96-6.93 (m, 1H), 3.83 (s, 3H). |
| **A15** | 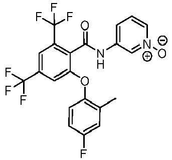 | LCMS: m/z 475.2 (M+H) ⁺; ¹H NMR (400 MHz, DMSO-*d₆*) δ 11.42 (s, 1H), 8.65 (s, 1H), 8.05-8.04 (t, *J* = 5.2 Hz, 1H), 7.96 (s, 1H), 7.49-7.39 (m, 2H), 7.28-7.12 (m, 4H), 2.15 (s, 3H). |
| **A16** | 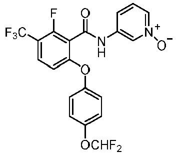 | LCMS: m/z 459.2 (M+H) ⁺; ¹H NMR (400 MHz, DMSO-*d₆*) δ 11.32 (s, 1H), 8.68(s, 1H), 8.05 (d, *J* = 7.6 Hz, 1H), 7.88(t, *J* = 8.4 Hz, 1H), 7.51 (d, *J* = 8.0 Hz, 1H), 7.44 - 7.04(m, 6H), 6.83 (d, *J* = 8.0 Hz, 1H). |
| **A17** | 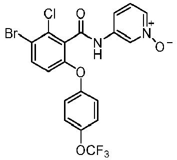 | LCMS: m/z :504.9 (M+H) ⁺; ¹H NMR (400 MHz, DMSO-*d₆*) δ 11.20 (s, 1H), 8.63 (s, 1H), 8.02 (d, *J* = 6.2 Hz, 1H), 7.88 (d, *J* = 8.9 Hz, 1H), 7.52 - 7.29 (m, 4H), 7.27 - 7.11 (m, 2H), 6.98 (d, *J* = 8.9 Hz, 1H). |
| **A18** | 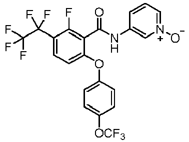 | LCMS: m/z 527.1 (M+H) ⁺; ¹H NMR (400 MHz, DMSO-*d₆*) δ 11.32 (s, 1H), 8.67 (s, 1H), 8.04 (d, *J* = 6.2 Hz, 1H), 7.84 (t, *J* = 8.7 Hz, 1H), 7.49 (t, *J* = 8.4 Hz, 3H), 7.43 - 7.36 (m, 3H), 6.94 (d, *J* = 8.9 Hz, 1H). |
| **A19** | 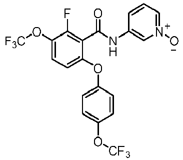 | LCMS: m/z 492.9 (M+H) ⁺; ¹H NMR (400 MHz, DMSO-*d₆*) δ11.30 (s, 1H), 8.64(s, 1H), 8.03 (d, *J* = 6.4 Hz, 1H), 7.73(t, *J* = 9.2 Hz, 1H), 7.51 - 7.35(m, 4H), 7.31 - 7.23(m, 2H), 6.94 (dd, *J* = 9.2, 1.2 Hz, 1H). |
| **A20** | 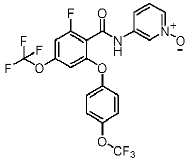 | LCMS: m/z 492.9 (M+H) ⁺; ¹H NMR (400 MHz, DMSO-*d₆*) δ11.22 (s, 1H), 8.63 (s, 1H), 8.02 (d, *J* = 7.2 Hz, 1H), 7.49 - 7.42(m, 4H), 7.41 - 7.35(m, 1H), 7.29 - 7.24(m, 2H), 6.92 (s, 1H). |
| **A21** | 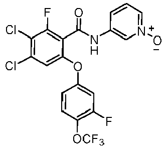 | LCMS: m/z 494.8 (M+H) ⁺; ¹H NMR (400 MHz, DMSO-*d₆*) δ 11.21 (s, 1H), 8.58 (d, *J* = 1.6 Hz, 1H), 8.03 (dt, *J* = 5.6, 1.7 Hz, 1H), 7.60 (t, *J* = 8.5 Hz, 1H), 7.52 (d, *J* = 1.9 Hz, 1H), 7.45 - 7.33 (m, 3H), 7.06 (ddd, *J* = 9.1, 2.9, 1.6 Hz, 1H). |
| **A22** | 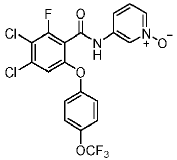 | LCMS: m/z 477.0 (M+H) ⁺; ¹H NMR (400 MHz, DMSO-*d₆*) δ 11.22 (s, 1H), 8.59 (s, 1H), 8.02 (dd, *J* = 6.0, 1.6 Hz, 1H), 7.40 (dt, *J* = 14.4, 7.7 Hz, 4H), 7.32 (d, *J* = 1.9 Hz, 1H), 7.29 - 7.20 (m, 2H). |
| **A23** | 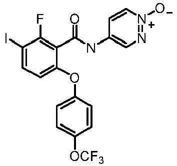 | LCMS: m/z 536.0 (M+H) ⁺; ¹H NMR (400 MHz, DMSO-*d₆*) δ 11.56 (s, 1H), 8.56 (s, 1H), 8.45 (d, *J* = 6.2 Hz, 1H), 8.02 - 7.92 (m, 1H), 7.42 (d, *J* = 8.8 Hz, 2H), 7.36 (d, *J* = 6.1 Hz, 1H), 7.26 - 7.17 (m, 2H), 6.74 (d, *J* = 8.8 Hz, 1H). |
| **A24** | 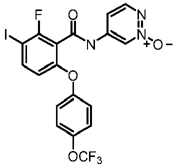 | LCMS: m/z 535.8 (M+H) ⁺; ¹H NMR (400 MHz, DMSO-*d₆*) δ 11.45 (s, 1H), 8.71 (d, *J* = 3.3 Hz, 1H), 8.25 (d, *J* = 7.1 Hz, 1H), 8.07 (dd, *J* = 7.1, 3.4 Hz, 1H), 8.01 - 7.91 (m, 1H), 7.41 (d, *J* = 8.8 Hz, 2H), 7.25 - 7.16 (m, 2H), 6.74 (d, *J* = 8.8 Hz, 1H). |
| **A25** | 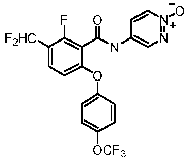 | LCMS: m/z 460.0 (M+H) ⁺; ¹H NMR (400 MHz, MeOD) *δ* 8.72 (d, *J* = 3.2 Hz, 1H), 8.27 - 8.25 (m, 1H), 8.23 - 8.20 (m, 1H), 7.62 (t, *J* = 8.0 Hz, 1H), 7.25 (d, *J* = 1.2 Hz, 2H), 7.23 (d, *J* = 1.2 Hz, 2H), 7.13 - 7.12 (m,0.3H), 7.11 - 7.10 (m, 0.5H), 6.92 - 6.82 (m, 1H), 6.80 - 6.78 (m,0.2H). |
| **A26** | 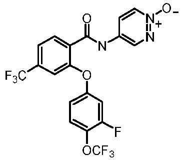 | LCMS: m/z 478.0 (M+H) ⁺; ¹H NMR (400 MHz, DMSO-*d₆*) *δ* 11.34 (s, 1H), 8.59 (d, *J* = 1.2 Hz, 1H), 8.45 (d, *J* = 6.4 Hz, 1H), 7.98 - 7.95 (m, 1H), 7.81 - 7.79 (m, 1H), 7.63 - 7.58 (m, 2H), 7.38 (dd, *J* = 6.4, 1.6 Hz, 1H), 7.31 (dd, *J* = 9.2, 2.8 Hz, 1H), 6.99 - 6.96 (m, 1H). |
| **A27** | 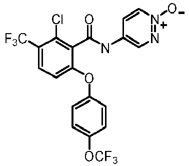 | LCMS: m/z 494.0 (M+H) ⁺; ¹H NMR (400 MHz, DMSO-*d₆*) *δ* 11.67 (s, 1H), 8.55 (d, *J* = 1.6 Hz, 1H), 8.41 (d, *J* = 6.4 Hz, 1H), 7.90 (d, *J* = 9.2 Hz, 1H), 7.45 - 7.41 (m, 2H), 7.34 (dd, *J* = 6.4, 1.6 Hz, 1H), 7.30 - 7.25 (m, 2H), 7.00 (d, 8.8 Hz, 1H). |
| **A28** | 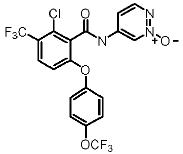 | LCMS: m/z 494.0 (M+H) ⁺; ¹H NMR (400 MHz, DMSO-*d₆*) *δ* 11.53 (s, 1H), 8.69 (dd, *J* = 3.6, 0.8 Hz, 1H), 8.20 (dd, *J* = 7.2, 0.8 Hz, 1H), 8.08 - 8.05 (m, 1H), 7.88 (d, *J* = 9.2 Hz, 1H), 7.44 - 7.40 (m, 2H), 7.29 - 7.25 (m, 2H), 7.99 (d, *J* = 9.2 Hz, 1H). |
| **A29** | 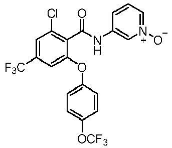 | LC-MS: MS 493.2 (M+H) ⁺; ¹H NMR (400 MHz, DMSO-*d₆*) *δ* 11.32 (s, 1H), 8.65 (s, 1H), 8.05 - 8.03 (m, 1H), 7.94 (s, 1H), 7.88 - 7.86 (m, 1H), 7.47 - 7.38 (m, 4H), 7.29 - 7.25 (m, 2H). |
| **A30** | 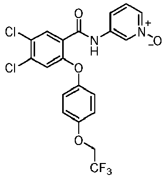 | LCMS: m/z 473.0 (M+H) ⁺; ¹H NMR (400 MHz, DMSO-*d₆*) δ 10.93 (s, 1H), 8.74 (s, 1H), 8.07 (d, *J* = 6.1 Hz, 1H), 7.98 (s, 1H), 7.58 (d, *J* = 8.4 Hz, 1H), 7.44 (dd, *J* = 8.5, 6.4 Hz, 1H), 7.20 - 7.04 (m, 5H), 4.75 (q, *J* = 8.9 Hz, 2H). |
| **A31** | 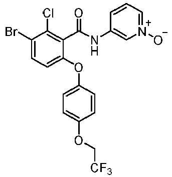 | LCMS: m/z :517.9 (M+H) ⁺; ¹H NMR (400 MHz, DMSO-*d₆*) δ 11.18 (s, 1H), 8.67 (t, J = 1.7 Hz, 1H), 8.08 - 7.98 (m, 1H), 7.82 (d, J = 9.0 Hz, 1H), 7.52 - 7.43 (m, 1H), 7.41 - 7.33 (m, 1H), 7.12 (s, 4H), 6.78 (d, J = 9.0 Hz, 1H), 4.75 (q, J = 8.9 Hz, 2H). |
| **A32** | 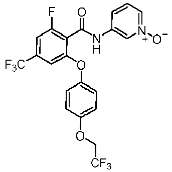 | LCMS: m/z 491.1 (M+H) ⁺; ¹H NMR (400 MHz, DMSO-*d₆*) δ11.29 (s, 1H), 8.69 (s, 1H), 8.04 (d, *J* = 6.0 Hz, 1H),7.85(t, *J* = 8.4Hz, 1H), 7.51 (d, *J* = 8.4Hz, 1H),7.46 - 7.36(m, 1H),7.25 - 7.14(m, 4H), 8.74 (d, *J* = 8.8 Hz, 1H), 4.78(q, *J* = 8.8Hz, 2H). |
| **A33** | 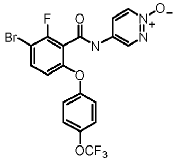 | LCMS: m/z: 488.1 (M+H) ⁺; ¹H NMR (400 MHz, DMSO-*d₆*) δ 11.71 (s, 1H), 8.56 (s, 1H), 8.46 - 8.38 (m, 1H), 7.90 - 7.77 (m, 1H), 7.42 (d, *J* = 8.4 Hz, 2H), 7.37 - 7.31 (m, 1H), 7.27 - 7.17 (m, 2H), 6.87 (dd, *J* = 9.1, 0.8 Hz, 1H). |
| **A34** | 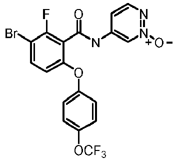 | LCMS: m/z: 488.1 (M+H) ⁺; ¹H NMR (400 MHz, DMSO-*d₆*) δ 11.51 (s, 1H), 8.71 (d, *J* = 3.1 Hz, 1H), 8.25 (d, *J* = 7.2 Hz, 1H), 8.07 (dd, *J* = 7.1, 3.4 Hz, 1H), 7.85 (t, *J* = 8.5 Hz, 1H), 7.42 (d, *J* = 8.6 Hz, 2H), 7.25 - 7.16 (m, 2H), 6.88 (dd, *J* = 9.0, 0.9 Hz, 1H). |
| **A35** | 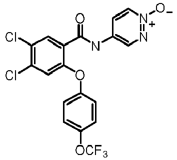 | LCMS: m/z 460.1 (M+H) ⁺; ¹H NMR (400 MHz, DMSO-*d₆*) δ 11.27 (s, 1H), 8.59 (d, *J* = 1.2 Hz, 1H), 8.44 (d, *J* = 6.3 Hz, 1H), 8.07 (d, *J* = 5.0 Hz, 1H), 7.47 (s, 1H), 7.44 - 7.34 (m, 3H), 7.27 - 7.06 (m, 2H). |
| **A36** | 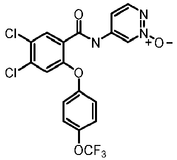 | LCMS: m/z 460.1 (M+H) ⁺; ¹H NMR (400 MHz, DMSO-*d₆*) δ 11.15 (s, 1H), 8.73 (d, *J* = 3.2 Hz, 1H), 8.25 (d, *J* = 7.1 Hz, 1H), 8.10 - 7.99 (m, 2H), 7.47 (s, 1H), 7.39 (d, *J* = 8.5 Hz, 2H), 7.25 - 7.07 (m, 2H). |
| **A37** | 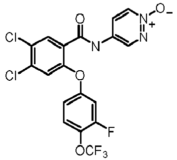 | LCMS: m/z 478.2 (M+H) ⁺; ¹H NMR (400 MHz, DMSO-*d₆*) δ 11.30 (s, 1H), 8.57 (d, *J* = 1.1 Hz, 1H), 8.43 (d, *J* = 6.3 Hz, 1H), 8.08 (s, 1H), 7.63 (s, 1H), 7.58 (t, *J* = 8.6 Hz, 1H), 7.37 (dd, *J* = 6.3, 1.6 Hz, 1H), 7.31 (dd, *J* = 11.4, 2.9 Hz, 1H), 6.98 (ddd, *J* = 9.1, 2.9, 1.5 Hz, 1H). |
| **A38** | 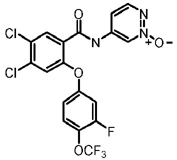 | LCMS: m/z 478.2 (M+H) ⁺; ¹H NMR (400 MHz, DMSO-*d₆*) δ 11.16 (s, 1H), 8.72 (d, *J* = 3.2 Hz, 1H), 8.25 (d, *J* = 7.1 Hz, 1H), 8.09 - 8.03 (m, 2H), 7.64 (s, 1H), 7.57 (t, *J* = 8.6 Hz, 1H), 7.30 (dd, *J* = 11.4, 2.9 Hz, 1H), 6.98 (ddd, *J* = 9.1, 2.8, 1.5 Hz, 1H). |
| **A39** | 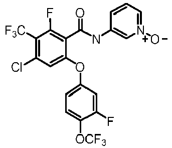 | LCMS: m/z 529.0 (M+H) ⁺; ¹H NMR (400 MHz, DMSO-*d₆*) δ 11.28 (s, 1H), 8.62 (s, 1H), 8.04 (dt, *J* = 5.9, 1.5 Hz, 1H), 7.66 (d, *J* = 8.8 Hz, 1H), 7.52 (dd, *J* = 11.1, 2.8 Hz, 1H), 7.47 - 7.36 (m, 3H), 7.22 - 7.10 (m, 1H). |
| **A40** | 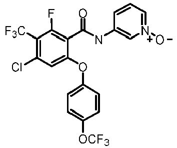 | LCMS: m/z 511.0 (M+H) ⁺; ¹H NMR (400 MHz, DMSO-*d₆*) δ 11.33 (s, 1H), 8.64 (s, 1H), 8.04 (d, *J* = 6.1 Hz, 1H), 7.55 - 7.31 (m, 6H), 7.16 (s, 1H). |

### Synthesis of embodiment A41

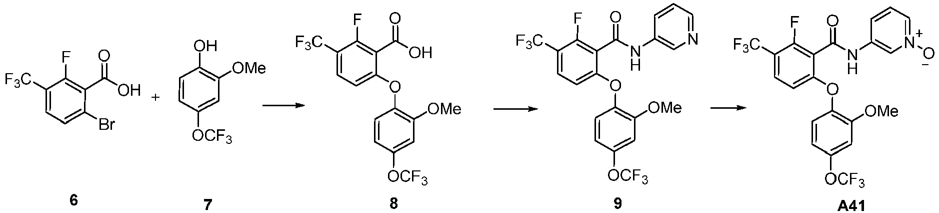

### Step 1, synthesis of intermediate 8

Compound **6** (450 mg, 1.6 mmol) was added to toluene (10 mL), Cs₂CO₃ (1.25 g, 3.84 mmol) and compound **7** (391 mg, 1.9 mmol) were added, the mixture was stirred at 100°C under nitrogen protection for 1.5 h, LC-MS showed that the reaction of the raw materials was complete, the mixture was cooled to room temperature, filtered, and the filter cake was washed with EA (30 mL^{∗}3 ), the combined organic phase was evaporated to dryness and purified by normal phase column (PE/EA=0-100%) to obtain compound **8,** white solid, 300 mg, yield 45%, LCMS: m/z 415.2(M+H)⁺.

### Step 2, synthesis of intermediate 9

Compound **8** (200 mg, 0.48 mmol) was dissolved into DCM (8 mL), cooled to 0°C in an ice bath, three drops of DMF were added, oxalyl chloride (245 mg, 1.93 mmol) was added dropwise, after the addition, the mixture was stirred at room temperature for 1 h, sampled, and the reaction was quenched by adding methanol, TLC showed that the reaction of the raw material was complete, and the reaction mixture was evaporated to dryness. DCM (10 mL) was added to the mixture, and the mixture was cooled to 0°C in an ice bath, DIPEA (250 mg, 1.93 mmol) and compound **2** (68 mg, 0.72 mmol) were added thereto, the mixture was stirred at room temperature for 2 h, the reaction was quenched by adding methanol (10 mL), the mixture was then evaporated to dryness and purified by normal phase column (PE/EA=0-100%) to obtain compound **9,** white solid, 70 mg, yield: 30%. LCMS: m/z 491.0(M+H)⁺.

### Step 3, Synthesis of embodiment A41

Compound **9** (70 mg, 0.14 mmol) was dissolved in DCM (10 mL), *m*-CPBA (50 mg, 0.29 mmol) was added, and the mixture was stirred for 4 h at room temperature, LCMS showed that the reaction of the raw materials was complete, saturated NaHCO₃ (20 mL) was added thereto, the mixture was then stirred for 30 min, the phases were separated, the aqueous phase was extracted with DCM (20 mL^{∗}2), the organic phase was washed with saturated NaCl (30 mL), dried over anhydrous Na₂SO₄, and evaporated to dryness (5-95% acetonitrile in water (containing 0.05% NH₄HCO₃)) to obtain compound **A41.** LCMS:m/z 507.2 (M+H) ⁺; ¹H NMR (400 MHz, DMSO-*d₆*) δ 11.28 (s, 1H), 8.69 - 8.68 (t, *J* = 1.2 Hz, 1H), 8.05 - 8.03 (d, *J* = 7.2 Hz, 1H), 7.83 - 7.79 (t, *J* = 8.4 Hz, 1H), 7.54 - 7.52 (d, *J* = 9.2 Hz, 1H), 7.43 - 7.36 (m, 2H), 7.27 - 7.26 (d, *J* = 2.4 Hz, 1H), 7.06 -7.04 (m, 1H), 6.69 - 6.67 (d, *J* = 8.8 Hz, 1H), 3.79 (s, 3H).

Similar to the synthesis of embodiments **A1** and **A41**, the following embodiments **A42-A114** were synthesized, as shown in Table 2 below:

**Table 2: Structural formula and analysis data of embodiments A42-A114**

| Embodiment | Structural formula | Analysis data |
|---|---|---|
| A42 | 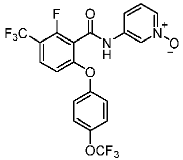 | LCMS: m/z 477.1 (M+H) ⁺; ¹H NMR (400 MHz, DMSO-*d₆*) δ 11.32 (s, 1H), 8.66 (t, *J* = 1.6 Hz, 1H), 8.08 - 8.00 (m, 1H), 7.89 (t, *J* = 8.7 Hz, 1H), 7.52 - 7.45 (m, 3H), 7.40 (dd, *J* = 8.4, 6.3 Hz, 1H), 7.37 - 7.31 (m, 2H), 6.91 (d, *J* = 8.9 Hz, 1H). |
| A43 | 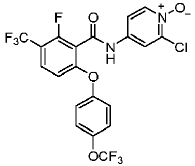 | LCMS: m/z 511.0 (M+H) ⁺; ¹H NMR (400 MHz, DMSO-*d₆*) δ 11.55 (s, 1H), 8.39 (d, *J* = 7.2 Hz, 1H), 8.04 (d, *J* = 2.9 Hz, 1H), 7.91 (t, *J* = 8.7 Hz, 1H), 7.63 - 7.42 (m, 3H), 7.35 (d, *J* = 9.1 Hz, 2H), 6.93 (d, *J* = 8.8 Hz, 1H). |
| A44 | 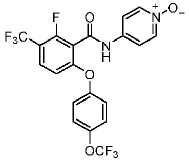 | LCMS: m/z 477.1 (M+H) ⁺; ¹H NMR (400 MHz, DMSO-*d₆*) δ 11.41 (s, 1H), 8.20 - 8.12 (m, 2H), 7.89 (t, *J* = 8.7 Hz, 1H), 7.68 - 7.60 (m, 2H), 7.49 (d, *J* = 8.5 Hz, 2H), 7.39 - 7.29 (m, 2H), 6.91 (d, *J* = 8.9 Hz, 1H). |
| A45 | 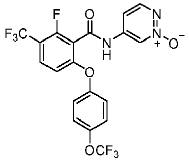 | LCMS: m/z 478.1 (M+H) ⁺; ¹H NMR (400 MHz, DMSO-*d₆*) δ 11.61 (s, 1H), 8.81 - 8.69 (m, 1H), 8.28 (d, *J* = 7.1 Hz, 1H), 8.12 (dd, *J* = 7.1, 3.4 Hz, 1H), 7.91 (t, *J* = 8.7 Hz, 1H), 7.54 - 7.45 (m, 2H), 7.39 - 7.29 (m, 2H), 6.93 (d, *J* = 8.9 Hz, 1H). |
| A46 | 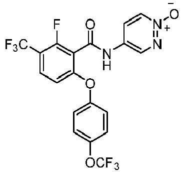 | LCMS: m/z 478.1 (M+H) ⁺; ¹H NMR (400 MHz, DMSO-*d₆*) δ 11.73 (s, 1H), 8.60 (d, *J* = 1.1 Hz, 1H), 8.49 (d, *J* = 6.3 Hz, 1H), 7.92 (t, *J* = 8.7 Hz, 1H), 7.50 (d, *J* = 8.4 Hz, 2H), 7.42 - 7.31 (m, 3H), 6.93 (d, *J* = 8.9 Hz, 1H). |
| A47 | 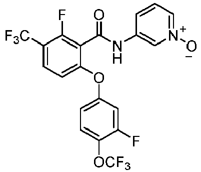 | LCMS: m/z 495.0 (M+H) ⁺; ¹H NMR (400 MHz, DMSO-*d₆*) δ 11.30 (s, 1H), 8.65 (t, *J* = 1.6 Hz, 1H), 8.05 (dd, *J* = 6.3, 1.6 Hz, 1H), 7.93 (t, *J* = 8.7 Hz, 1H), 7.69 (t, *J* = 8.9 Hz, 1H), 7.55 - 7.37 (m, 3H), 7.16 (ddd, *J* = 9.1, 2.8, 1.6 Hz, 1H), 7.09 (d, *J* = 8.9 Hz, 1H). |
| A48 | 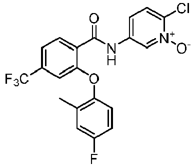 | LCMS: m/z 441.10 (M+H) ⁺; ¹H NMR (400 MHz, DMSO-*d₆*) δ 10.96 (s, 1H), 8.85 (d, *J* = 2.2 Hz, 1H), 7.83 (d, *J* = 7.9 Hz, 1H), 7.70 (d, *J* = 9.0 Hz, 1H), 7.56 (dd, *J* = 8.2, 1.6 Hz, 1H), 7.44 (dd, *J* = 9.0, 2.2 Hz, 1H), 7.15 (d, *J* = 9.2 Hz, 1H), 7.09 - 6.98 (m, 2H), 6.94 (d, *J* = 1.6 Hz, 1H), 2.09 (s, 3H). |
| A49 | 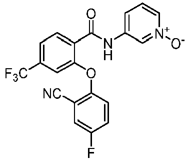 | LCMS: m/z 418.1 (M+H) ⁺; ¹H NMR (400 MHz, CH₃OH-*d₆*) δ 8.36 (s, 1H), 8.24 - 8.02 (m, 1H), 7.74 - 7.50 (m, 3H), 7.42 - 7.35 (m, 3H), 7.06 (d, *J* = 8.0 Hz, 1H), 6.82 (s, 1H). |
| A50 | 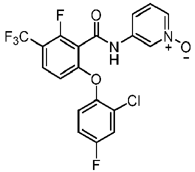 | LCMS: m/z 445.0 (M+H) ⁺; ¹H NMR (400 MHz, DMSO-*d₆*) *δ* 11.33 (s, 1H), 8.68 (s, 1H), 8.06 (d, *J* = 6.4 Hz, 1H), 7.87 (t, *J* = 8.4 Hz, 1H), 7.73 (dd, *J* = 8.0, 2.8 Hz, 1H), 7.54 - 7.47 (m, 2H), 7.44 - 7.36 (m, 2H), 6.74 (d, *J* = 8.8 Hz, 1H). |
| A51 | 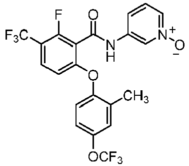 | LCMS: m/z 491.0 (M+H) ⁺;¹H NMR (400 MHz, DMSO-*d₆*) δ 11.34 (s, 1H), 8.68 (t, *J* = 1.6 Hz, 1H), 8.05 (d, *J* = 6.3 Hz, 1H), 7.86 (t, *J* = 8.7 Hz, 1H), 7.50 (d, *J* = 9.3 Hz, 1H), 7.44 - 7.40 (m, 2H), 7.36 - 7.24 (m, 2H), 6.74 (d, *J* = 8.9 Hz, 1H), 2.18 (s, 3H). |
| A52 | 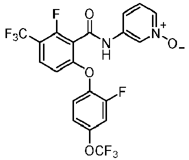 | LCMS: m/z 495.0 (M+H) ⁺; ¹H NMR (400 MHz, DMSO-*d₆*) δ 11.37 (s, 1H), 8.67 (t, *J* = 1.6 Hz, 1H), 8.05 (d, *J* = 6.3 Hz, 1H), 7.90 (t, *J* = 8.7 Hz, 1H), 7.73 (dd, *J* = 10.8, 2.5 Hz, 1H), 7.53 (dd, *J* = 18.5, 9.4 Hz, 2H), 7.46 - 7.33 (m, 2H), 6.97 (d, *J* = 8.9 Hz, 1H). |
| A53 | 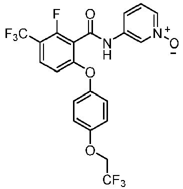 | LCMS: m/z 491.2 (M+H) ⁺;¹H NMR (400 MHz, DMSO-*d₆*) δ 11.29 (s, 1H), 8.69 (s, 1H), 8.04 (d, *J* = 6.3 Hz, 1H), 7.85 (t, *J* = 8.7 Hz, 1H), 7.51 (d, *J* = 9.3 Hz, 1H), 7.41 (dd, *J* = 8.5, 6.4 Hz, 1H), 7.28 - 7.12 (m, 4H), 6.74 (d, *J* = 8.9 Hz, 1H), 4.78 (q, *J* = 8.9 Hz, 2H). |
| A54 | 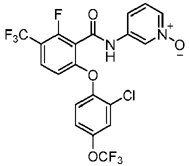 | LCMS: m/z 511.0 (M+H) ⁺;¹H NMR (400 MHz, DMSO-*d₆*) δ 11.34 (s, 1H), 8.67 (t, *J* = 1.6 Hz, 1H), 8.06 (d, *J* = 7.0 Hz, 1H), 7.90 (t, *J* = 8.7 Hz, 1H), 7.84 (s, 1H), 7.57 - 7.38 (m, 4H), 6.88 (d, *J* = 8.8 Hz, 1H). |
| A55 | 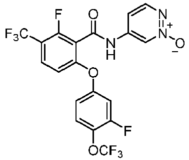 | LC-MS: m/z 496.0 (M+H) ⁺;¹H NMR (400 MHz, DMSO-*d₆*) δ 11.58 (s, 1H), 8.73 (d, *J* = 3.4 Hz, 1H), 8.28 (dd, *J* = 7.1, 0.6 Hz, 1H), 8.10 (dd, *J* = 7.1, 3.4 Hz, 1H), 7.94 (t, *J* = 8.7 Hz, 1H), 7.69 (t, *J* = 8.5 Hz, 1H), 7.51 (dd, *J* = 11.1, 2.9 Hz, 1H), 7.16 (ddd, *J* = 9.0, 2.8, 1.5 Hz, 1H), 7.10 (d, *J* = 8.9 Hz, 1H). |
| A56 | 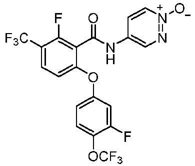 | LCMS: m/z 496.0 (M+H) ⁺;¹H NMR (400 MHz, DMSO-*d₆*) δ11.54 (s, 1H), 8.59 (d, *J* = 1.2 Hz, 1H), 8.48 (d, *J* = 6.2 Hz, 1H), 7.95 (t, *J* = 8.6 Hz, 1H), 7.69 (t, *J* = 9.0 Hz, 1H), 7.52 (dd, *J* = 11.1, 2.8 Hz, 1H), 7.38 (dd, *J* = 6.3, 1.6 Hz, 1H), 7.22 - 7.13 (m, 1H), 7.09 (d, *J* = 8.9 Hz, 1H). |
| A57 | 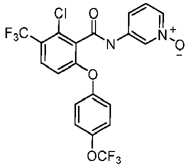 | LC-MS: m/z 493.0 (M+H) ⁺; ¹H NMR (400 MHz, DMSO-*d₆*) δ 11.34 (s, 1H), 8.69 (t, *J* = 1.5 Hz, 1H), 8.04 (d, *J* = 6.3 Hz, 1H), 7.94 (d, *J* = 9.0 Hz, 1H), 7.62 - 7.22 (m, 6H), 7.06 (d, *J* = 8.8 Hz, 1H). |
| A58 | 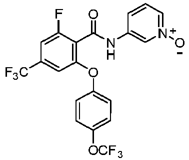 | LCMS: m/z 477.1 (M+H) ⁺; ¹H NMR (400 MHz, DMSO-*d₆*) δ 11.38 (s, 1H), 8.62 (d, *J* = 1.6 Hz, 1H), 8.03 (dd, *J* = 6.3, 1.6 Hz, 1H), 7.78 (d, *J* = 8.3 Hz, 1H), 7.50 - 7.34 (m, 4H), 7.31 - 7.10 (m, 3H). |
| A59 | 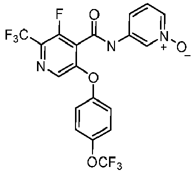 | LCMS: m/z 478.1 (M+H) ⁺; ¹H NMR (400 MHz, DMSO-*d₆*) δ 8.60 (s, 1H), 8.43 (s, 1H), 8.04 (d, *J* = 5.7 Hz, 1H), 7.41 (ddd, *J* = 13.6, 12.4, 5.7 Hz, 7H). |
| A60 | 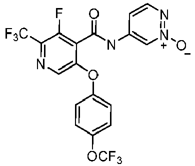 | LCMS: m/z 479.0 (M+H) ⁺; ¹H NMR (400 MHz, DMSO-*d₆*) δ 11.78 (s, 1H), 8.68 (d, *J* = 3.3 Hz, 1H), 8.46 (s, 1H), 8.26 (d, *J* = 7.1 Hz, 1H), 8.06 (dd, *J* = 7.1, 3.4 Hz, 1H), 7.56 - 7.16 (m, 4H). |
| A61 | 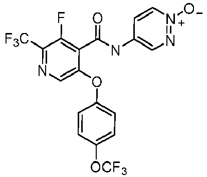 | LCMS: m/z 479.0 (M+H) ⁺; ¹H NMR (400 MHz, DMSO-*d₆*) δ 11.86 (s, 1H), 8.55 (s, 1H), 8.49 (d, *J* = 6.1 Hz, 2H), 7.48-7.34 (m, 5H). |
| A62 | 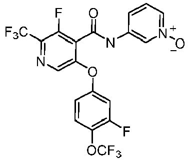 | LCMS: m/z 496.1 (M+H) ⁺; ¹H NMR (400 MHz, DMSO-*d₆*) δ 8.59 (d, *J* = 8.7 Hz, 2H), 8.06 (d, *J* = 5.8 Hz, 1H), 7.66 (s, 1H), 7.58 (dd, *J* = 11.2, 2.8 Hz, 1H), 7.49 - 7.32 (m, 2H), 7.23 (d, *J* = 9.0 Hz, 1H). |
| A63 | 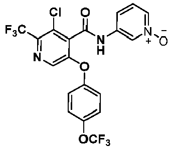 | LCMS: m/z 494.1 (M+H) ⁺; ¹H NMR (400 MHz, DMSO-*d₆*) δ11.43 (s, 1H), 8.62 (s, 1H), 8.52 (s, 1H), 8.06 (dt, *J* = 5.2, 1.6 Hz, 1H), 7.52 - 7.32 (m, 6H). |
| A64 | 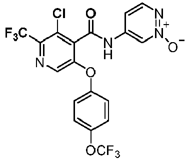 | LCMS: m/z 494.9 (M+H) ⁺; ¹H NMR (400 MHz, DMSO-*d₆*) δ11.89 (s, 1H), 8.69 (d, *J* = 2.8 Hz, 1H), 8.51 (s, 1H), 8.25 (d, *J* = 7.2 Hz, 1H), 8.08 (dd, *J* = 7.2, 3.6 Hz, 1H), 7.46 (d, *J* = 8.4 Hz, 2H), 7.40 - 7.33 (m, 2H). |
| A65 | 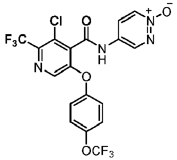 | LCMS: m/z 495.0 (M+H) ⁺; ¹H NMR (400 MHz, DMSO-*d₆*) δ12.03 (s, 1H), 8.52 (d, *J* = 12.8 Hz, 2H), 8.43 (d, *J* = 6.0 Hz, 1H), 7.46 (d, *J* = 8.8 Hz, 2H), 7.41 - 7.28 (m, 3H). |
| A66 | 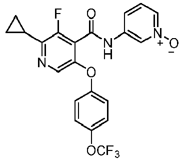 | LCMS: m/z 450.0 (M+H) ⁺ ; ¹H NMR (400 MHz, DMSO-*d₆*) δ 11.24 (s, 1H), 8.62 (s, 1H), 8.47 (s, 1H), 8.01 (d, *J* = 5.8 Hz, 1H), 7.47 - 7.18 (m, 4H), 7.07 - 6.92 (m, 2H), 2.15 - 2.05 (m, 1H), 2.03 - 1.90 (m, 1H), 0.99 - 0.70 (m, 3H). |
| A67 | 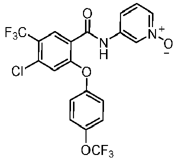 | LCMS: m/z 493.0 (M+H) ⁺; ¹H NMR (400 MHz, DMSO-*d₆*) δ 10.90 (s, 1H), 8.66 (s, 1H), 8.20 (s, 1H), 8.01 (d, *J* = 6.8 Hz, 1H), 7.49 - 7.42(m, 3H), 7.41 - 7.30 (m, 4H). |
| A68 | 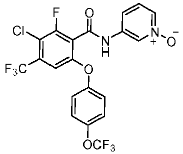 | LCMS: m/z 510.8 (M+H) ⁺; ¹H NMR (400 MHz, DMSO-*d₆*) δ 11.35 (s, 1H), 8.59 (s, 1H), 8.06 - 8.00 (m, 1H), 7.47 - 7.36 (m, 5H), 7.29 - 7.24(m, 2H). |
| A69 | 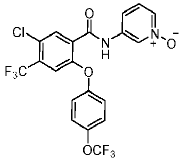 | LCMS: m/z 493.0 (M+H) ⁺; ¹H NMR (400 MHz, DMSO-*d₆*) δ 10.99 (s, 1H), 8.60 (d, *J* = 1.9 Hz, 1H), 8.13 (s, 1H), 8.01 (dt, *J* = 6.3, 1.3 Hz, 1H), 7.56 (s, 1H), 7.48 - 7.33 (m, 4H), 7.24 - 7.14 (m, 2H). |
| A70 | 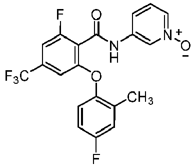 | LC-MS: m/z 425.1 (M+H) ⁺; ¹H NMR (400 MHz, DMSO-*d₆*) δ 11.30 (s, 1H), 8.66 (s, 1H), 8.04 (d, *J* = 6.4 Hz, 1H), 7.69 (d, *J* = 8.5 Hz, 1H), 7.49 (d, *J* = 8.4 Hz, 1H), 7.41 (dd, *J* = 8.4, 6.4 Hz, 1H), 7.27 - 7.21 (m, 1H), 7.20 - 7.07 (m, 2H), 6.82 (s, 1H), 2.15 (s, 3H). |
| A71 | 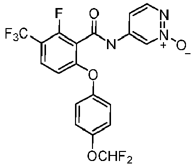 | LCMS: m/z 460.0 (M+H) ⁺; ¹H NMR (400 MHz, DMSO-*d₆*) δ 11.66 (s, 1H), 8.76 (d, *J* = 3.2 Hz, 1H), 8.27 (d, *J* = 7.1 Hz, 1H), 8.13 (dd, *J* = 7.1, 3.4 Hz, 1H), 7.88 (t, *J* = 8.7 Hz, 1H), 7.52 - 6.97 (m, 5H), 6.83 (d, *J* = 8.9 Hz, 1H). |
| A72 | 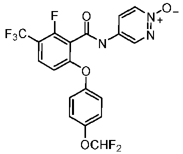 | LCMS: m/z 460.0 (M+H) ⁺; ¹H NMR (400 MHz, DMSO-*d₆*) δ 11.78 (s, 1H), 8.62 (s, 1H), 8.47 (d, *J* = 6.2 Hz, 1H), 7.89 (t, *J* = 8.7 Hz, 1H), 7.49 - 7.00 (m, 6H), 6.81 (t, *J* = 13.7 Hz, 1H). |
| A73 | 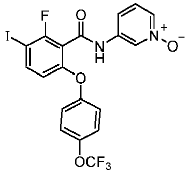 | LCMS: m/z: 535.1 (M+H) ⁺; ¹H NMR (400 MHz, DMSO-*d₆*) δ 11.16 (s, 1H), 8.62 (s, 1H), 8.01 (d, *J* = 6.2 Hz, 1H), 7.98 - 7.92 (m, 1H), 7.41 (td, *J* = 15.7, 8.9 Hz, 4H), 7.22 (d, *J* = 9.1 Hz, 2H), 6.73 (d, *J* = 8.8 Hz, 1H). |
| A74 | 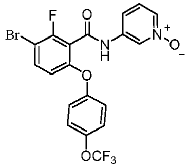 | LCMS: m/z: 488.9 (M+H) ⁺; ¹H NMR (400 MHz, DMSO-*d₆*) δ 11.24 (s, 1H), 8.62 (s, 1H), 8.02 (d, *J* = 6.5 Hz, 1H), 7.84 (t, *J* = 8.5 Hz, 1H), 7.52 - 7.33 (m, 4H), 7.29 - 7.17 (m, 2H), 6.87 (d, *J* = 9.0 Hz, 1H). |
| A75 | 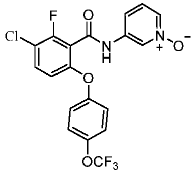 | LCMS: m/z 443.0 (M+H) ⁺; ¹H NMR (400 MHz, DMSO-*d₆*) *δ* 11.01 (s, 1H), 8.56 (s, 1H), 7.95 (d, *J* = 6.4 Hz, 1H), 7.67 (t, *J* = 8.8 Hz, 1H), 7.40 - 7.30 (m, 4H), 7.15 (d, *J* = 9.2 Hz, 2H), 6.86 (d, *J* = 9.2 Hz, 1H). |
| A76 | 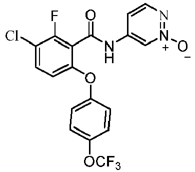 | LCMS: m/z 444.0 (M+H) ⁺; ¹H NMR (400 MHz, DMSO-*d₆*) *δ* 11.13 (s, 1H), 8.65 (s, 1H), 8.18 (d, *J* = 6.8 Hz, 1H), 8.02 - 7.99 (m, 1H), 7.69 (t, *J* = 8.8 Hz, 1H), 7.34 (d, *J* = 8.4 Hz, 2H), 7.15 (d, *J* = 8.8 Hz, 2H), 6.87 (dd, *J* = 9.2, 1.2 Hz, 1H). |
| A77 | 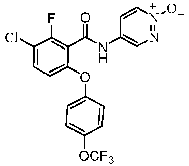 | LCMS: m/z 444.0 (M+H) ⁺; ¹H NMR (400 MHz, DMSO-*d₆*) *δ* 11.56 (s, 1H), 8.50 (s, 1H), 8.39 (d, *J* = 6.4 Hz, 1H), 7.70 (t, *J* = 8.8 Hz, 1H), 7.37 - 7.34 (m, 2H), 7.29 (dd, *J* = 6.4, 1.2 Hz, 1H), 7.19 - 7.15 (m, 2H), 6.87 (dd, *J* = 9.2, 1.2 Hz, 1H). |
| A78 | 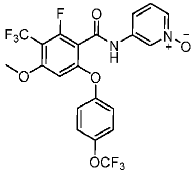 | LCMS: m/z: 507.2 (M+H) ⁺; ¹H NMR (400 MHz, DMSO-*d₆*) δ 11.12 (s, 1H), 8.60 (s, 1H), 8.05 - 7.97 (m, 1H), 7.47 - 7.33 (m, 4H), 7.33 - 7.23 (m, 2H), 6.73 (s, 1H), 3.32 (s, 3H). |
| A79 | 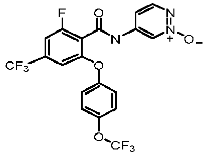 | LCMS: m/z 478.1 (M+H) ⁺; ¹H NMR (400 MHz, DMSO-*d₆*) δ 11.55 (s, 1H), 8.71 (dd, *J* = 3.4, 0.8 Hz, 1H), 8.27 (dd, *J* = 7.1, 0.7 Hz, 1H), 8.07 (dd, *J* = 7.1, 3.4 Hz, 1H), 7.81 (dd, *J* = 8.9, 1.4 Hz, 1H), 7.51 - 7.40 (m, 2H), 7.35 - 7.19 (m, 3H). |
| A80 | 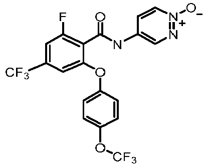 | LCMS: m/z 478.1 (M+H) ⁺; ¹H NMR (400 MHz, DMSO-*d₆*) δ 11.67 (s, 1H), 8.57 (d, *J* = 1.6 Hz, 1H), 8.48 (d, *J* = 6.2 Hz, 1H), 7.82 (dd, *J* = 9.0, 1.4 Hz, 1H), 7.48 - 7.43 (m, 2H), 7.36 (dd, *J* = 6.3, 1.7 Hz, 1H), 7.31 - 7.25 (m, 3H). |
| A81 | 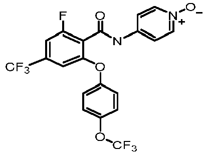 | LCMS: m/z 476.8 (M+H) ⁺; ¹H NMR (400 MHz, DMSO-*d₆*) δ 11.36 (s, 1H), 8.22 - 8.07 (m, 2H), 7.80 (d, *J* = 8.6 Hz, 1H), 7.67 - 7.55 (m, 2H), 7.45 (d, *J* = 8.7 Hz, 2H), 7.34 - 7.19 (m, 3H). |
| A82 | 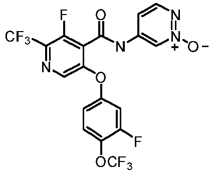 | LCMS: m/z 497.2 (M+H) ⁺; ¹H NMR (400 MHz, DMSO-*d₆*) δ 11.74 (s, 1H), 8.69 (dd, *J* = 3.5, 0.8 Hz, 1H), 8.61 (s, 1H), 8.28 (dd, *J* = 7.1, 0.8 Hz, 1H), 8.06 (dd, *J* = 7.1, 3.4 Hz, 1H), 7.67 (td, *J* = 9.0, 1.2 Hz, 1H), 7.57 (dd, *J* = 11.1, 2.9 Hz, 1H), 7.22 (ddd, *J* = 9.1, 2.9, 1.6 Hz, 1H). |
| A83 | 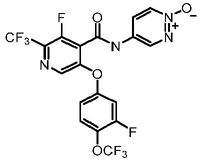 | LCMS: m/z 497.2 (M+H) ⁺; ¹H NMR (400 MHz, DMSO-*d₆*) δ 11.88 (s, 1H), 8.61 (s, 1H), 8.53 (d, *J* = 1.6 Hz, 1H), 8.49 (d, *J* = 6.3 Hz, 1H), 7.67 (td, *J* = 9.0, 1.3 Hz, 1H), 7.58 (dd, *J* = 11.1, 2.9 Hz, 1H), 7.34 (dd, *J* = 6.2, 1.7 Hz, 1H), 7.22 (ddd, *J* = 9.1, 3.0, 1.6 Hz, 1H). |
| A84 | 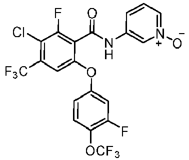 | LCMS: m/z 528.9 (M+H) ⁺; ¹H NMR (400 MHz, DMSO-*d₆*) δ 11.26 (s, 1H), 8.57 (s, 2H), 8.04 (s, 1H), 7.61 (m, 2H), 7.45 - 7.26 (m, 2H), 7.07 (m, 1H). |
| A85 | 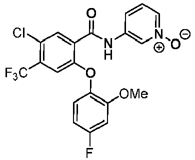 | LCMS: m/z 456.9 (M+H) ⁺; ¹H NMR (400 MHz, DMSO-d6) δ 10.96 (s, 1H), 8.70 (s, 1H), 8.04 (m, 2H), 7.53 (d, *J* = 8.3 Hz, 1H), 7.41 (dd, *J* = 8.4, 6.4 Hz, 1H), 7.28 (dd, *J* = 8.9, 5.8 Hz, 1H), 7.15 (dd, *J* = 10.6, 2.9 Hz, 1H), 7.01 (s, 1H), 6.85 (td, *J* = 8.5, 2.9 Hz, 1H), 3.74 (s, 3H). |
| A86 | 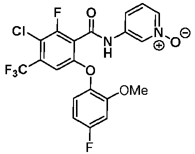 | LCMS: m/z 475.0 (M+H) ⁺; ¹H NMR (400 MHz, DMSO-d6) δ11.28 (s, 1H), 8.64-8.63 (m, 1H), 8.04 - 8.03 (m, 1H), 7.49 - 7.46 (m, 1H), 7.42 - 7.39(m, 1H), 7.28 - 7.24(m, 1H), 7.16 - 7.13(m, 1H), 6.86- 6.81(m, 2H), 3.74 (s, 3H). |
| A87 | 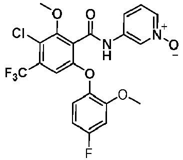 | LCMS: m/z 487.0 (M+H) ⁺; ¹H NMR (400 MHz, DMSO-d6) δ11.13(s, 1H), 8.70-8.69(m, 1H), 8.05 - 8.03 (m, 1H), 7.52 - 7.50 (m, 1H), 7.43 - 7.39(m, 1H), 7.26- 7.22(m, 1H), 7.17 - 7.14(m, 1H), 6.88- 6.83(m, 1H), 6.76(s, 1H), 3.92 (s, 3H), 3.77(s, 3H). |
| A88 | 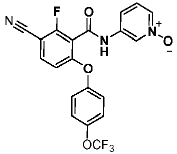 | LCMS: m/z 434.0 (M+H) ⁺; ¹H NMR (400 MHz, DMSO-d6) δ 11.31 (s, 1H), 8.66 (t, J = 1.8 Hz, 1H), 8.11 - 7.96 (m, 2H), 7.53 - 7.45 (m, 3H), 7.43 - 7.39 (m, 1H), 7.38 - 7.32 (m, 2H), 6.93 (dd, J = 8.9, 0.8 Hz, 1H). |
| A89 | 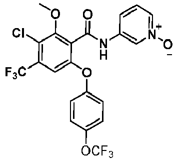 | LCMS: m/z 522.9 (M+H) ⁺; ¹H NMR (400 MHz, DMSO-d6) δ 8.63 (s, 1H), 7.99 (d, J = 6.2 Hz, 1H), 7.48 - 7.32 (m, 4H), 7.32 - 7.12 (m, 4H), 3.94 (s, 3H). |
| A90 | 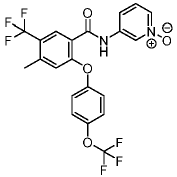 | LCMS: m/z 473.0 (M+H) ⁺; ¹H NMR (400 MHz, DMSO-d6) δ 10.79 (s, 1H), 8.66 (d, J = 1.9 Hz, 1H), 8.03 - 7.96 (m, 2H), 7.53 - 7.33 (m, 4H), 7.27 - 7.20 (m, 2H), 7.12 (s, 1H), 2.44 (s, 3H). |
| A91 | 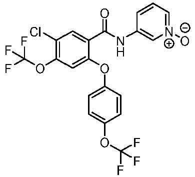 | LCMS: m/z 508.8 (M+H) ⁺; ¹H NMR (400 MHz, DMSO-d6) δ 10.90 (s, 1H), 8.60 (t, J = 1.8 Hz, 1H), 8.11 (s, 1H), 8.00 (ddd, J = 6.3, 1.8, 1.0 Hz, 1H), 7.48 - 7.34 (m, 5H), 7.22 - 7.16 (m, 2H). |
| A92 | 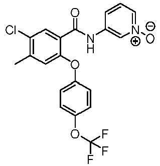 | LCMS: m/z 438.8 (M+H) ⁺; ¹H NMR (400 MHz, DMSO-d6) δ 10.72 (s, 1H), 8.62 (t, J = 1.8 Hz, 1H), 7.98 (ddd, J = 6.4, 1.8, 0.9 Hz, 1H), 7.78 (s, 1H), 7.48 (dt, J = 8.7, 1.1 Hz, 1H), 7.39 - 7.32 (m, 3H), 7.18 (d, J = 0.8 Hz, 1H), 7.13 - 7.08 (m, 2H), 2.35 (s, 3H). |
| A93 | 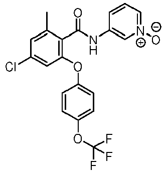 | LCMS: m/z 439.0 (M+H) ⁺; ¹H NMR (400 MHz, DMSO-d6) δ10.97 (s, 1H), 8.67 (s, 1H), 8.02-8.00 (d, 1H), 7.48-7.46 (m, 1H), 7.41-7.39 (m, 3H), 7.38 - 7.30 (m, 1H), 7.20-7.18 (m, 2H), 6.93-6.92 (s, 1H), 2.34 (s, 1H). |
| A94 | 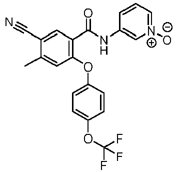 | LCMS: m/z 430.0 (M+H) ⁺; ¹H NMR (400 MHz, DMSO-d6) δ 10.82 (s, 1H), 8.66 (d, J = 1.9 Hz, 1H), 8.16 (s, 1H), 8.04 - 7.96 (m, 1H), 7.52 - 7.41 (m, 3H), 7.38 (dd, J = 8.5, 6.4 Hz, 1H), 7.30 - 7.23 (m, 2H), 7.09 (s, 1H), 2.48 (s, 3H). |
| A95 | 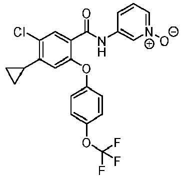 | LCMS: m/z 465.0 (M+H) ⁺; ¹H NMR (400 MHz, DMSO-d6) δ 10.69 (s, 1H), 8.57 (s, 1H), 7.96 (d, 1H), 7.78 (s, 1H), 7.44 (d, 1H), 7.35-7.31(m, 3H), 7.02 (d, 2H), 6.81 (s, 1H), 2.21-2.18 (m,1H), 1.08 - 1.03 (m, 2H), 0.72-0.71(m, 2H). |
| A96 | 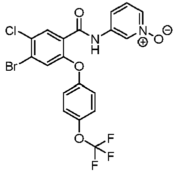 | LCMS: m/z 504.9 (M+H) ⁺; ¹H NMR (400 MHz, DMSO-d6) δ 10.84 (s, 1H), 8.60 (s, 1H), 8.05 - 7.89 (m, 2H), 7.54 (s, 1H), 7.47-7.44(m, 1H), 7.40 - 7.34 (m, 3H), 7.17 (m, 2H). |
| A97 | 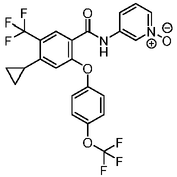 | LCMS: m/z 499.1 (M+H) ⁺; ¹H NMR (400 MHz, DMSO-d6) δ 10.74 (s, 1H), 8.61 (s, 1H), 7.98 (d, J = 5.6 Hz, 2H), 7.45 (d, J = 8.6 Hz, 1H), 7.41 - 7.28 (m, 3H), 7.20 - 7.05 (m, 2H), 6.77 (s, 1H), 2.21 - 2.08 (m, 1H), 1.08 (m, 2H), 0.74 (m, 2H). |
| A98 | 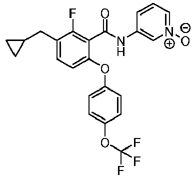 | LCMS: m/z 463.0 (M+H) ⁺; ¹H NMR (400 MHz, MeOD) δ 8.95 (t, 1H), 8.11 (d, 1H), 7.69 (dd, 1H), 7.53 - 7.42 (m, 2H), 7.26 (d, 2H), 7.15 - 7.05 (m, 2H), 6.81 (d, 1H), 2.60 (d, 2H), 1.10 - 0.94 (m, 1H), 0.59 - 0.47 (m, 2H), 0.26-0.22 (m, 2H). |
| A99 | 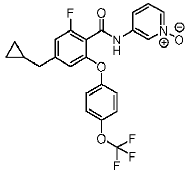 | LCMS: m/z 463.0 (M+H) ⁺; ¹H NMR (400 MHz, DMSO-d6) δ 11.09 (s, 1H), 8.64 (s, 1H), 8.02 (d, J = 7.1 Hz, 1H), 7.48 (d, J = 9.3 Hz, 1H), 7.44 - 7.33 (m, 3H), 7.24 - 7.11 (m, 3H), 6.82 (s, 1H), 2.54-2.50(m, 2H),1.02 - 0.89 (m, 1H), 0.51 - 0.41 (m, 2H), 0.26 - 0.14 (m, 2H). |
| A100 | 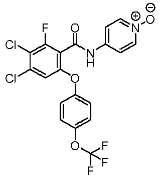 | LCMS: m/z 477.0 (M+H) ⁺; ¹H NMR (400 MHz, DMSO-d6) δ 11.36 (s, 1H), 8.21 - 8.05 (m, 2H), 7.64 - 7.54 (m, 2H), 7.41 (d, J = 8.4 Hz, 2H), 7.31 (d, J = 1.9 Hz, 2H), 7.28 - 7.20 (m, 1H). |
| A101 | 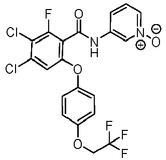 | LCMS: m/z 491.0 (M+H) ⁺; ¹H NMR (400 MHz, DMSO-d6) δ 11.24 (s, 1H), 8.64 (t, J = 1.6 Hz, 1H), 8.08 - 7.96 (m, 1H), 7.51 - 7.34 (m, 2H), 7.21 - 7.08 (m, 3H), 6.96 (d, J = 1.8 Hz, 1H), 4.76 (dt, J = 8.9, 5.9 Hz, 2H). |
| A102 | 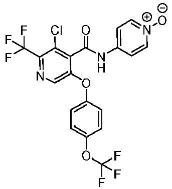 | LCMS: m/z 494.0 (M+H) ⁺; 1H NMR (400 MHz, DMSO-d6) δ 11.63 (s, 1H), 8.50 (s, 1H), 8.16 (d, J = 7.6 Hz,,2H), 7.61 (d, J = 7.2 Hz,,2H), 7.46 (d, J = 8.8 Hz,,2H),7.40 - 7.32(m, 2H). |
| A103 | 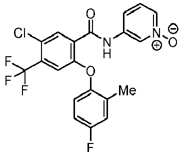 | LCMS: m/z 440.8 (M+H) ⁺; 1H NMR (400 MHz, DMSO-d6) δ 11.00 (s, 1H), 8.65 (t, J = 1.9 Hz, 1H), 8.10 (s, 1H), 8.02 (dt, J = 6.5, 1.3 Hz, 1H), 7.48 (dt, J = 8.6, 1.1 Hz, 1H), 7.40 (dd, J = 8.5, 6.3 Hz, 1H), 7.21 (dd, J = 8.9, 1.9 Hz, 1H), 7.14 (s, 1H), 7.09 (dd, J = 6.6, 1.7 Hz, 2H), 2.17 (s, 3H). |
| A104 | 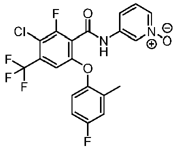 | LCMS: m/z 459.0 (M+H) ⁺; ¹H NMR (400 MHz, DMSO-d6) δ 11.33 (s, 1H), 8.64 (s, 1H), 8.11 - 8.03 (m, 1H), 7.48 - 7.40 (m, 2H), 7.26-7.23 (m, 1H), 7.21 - 7.09 (m, 2H), 6.99 (s, 1H), 2.16 (s, 3H). |
| A105 | 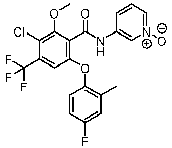 | LCMS: m/z 471.0 (M+H) ⁺; ¹H NMR (400 MHz, DMSO-d6) δ 11.17 (s, 1H), 8.67 (s, 1H), 8.04 (d, J = 6.2 Hz, 1H), 7.51 - 7.36 (m, 2H), 7.24-7.21 (m, 1H), 7.17 - 7.08 (m, 2H), 6.85 (s, 1H), 3.94 (s, 3H), 2.15 (s, 3H). |
| A106 | 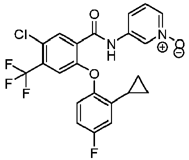 | LCMS: m/z 466.9 (M+H) ⁺; ¹H NMR (400 MHz, DMSO-d6) δ 11.00 (s, 1H), 8.67 (s, 1H), 8.09 (s, 1H), 8.03 (d, J = 7.0 Hz, 1H), 7.50 (d, J = 9.0 Hz, 1H), 7.41 (dd, J = 8.4, 6.4 Hz, 1H), 7.15 (dd, J = 8.9, 5.1 Hz, 1H), 7.08 (s, 1H), 7.07 - 7.01 (m, 1H), 6.86 (dd, J = 10.0, 3.0 Hz, 1H), 2.04 - 1.94 (m, 1H), 0.89 - 0.79 (m, 2H), 0.73 - 0.63 (m, 2H). |
| A107 | 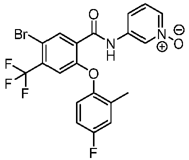 | LCMS: m/z 487.0 (M+H) ⁺; ¹H NMR (400 MHz, DMSO-d6) δ 10.99 (s, 1H), 8.65 (t, J = 1.8 Hz, 1H), 8.22 (s, 1H), 8.02 (ddd, J = 6.3, 1.8, 1.0 Hz, 1H), 7.51 - 7.45 (m, 1H), 7.40 (dd, J = 8.5, 6.3 Hz, 1H), 7.25 - 7.19 (m, 1H), 7.13 - 7.07 (m, 3H), 2.17 (s, 3H). |
| A108 | 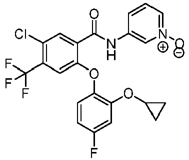 | LCMS: m/z 483.0 (M+H) ⁺; ¹H NMR (400 MHz, DMSO-d6) δ 10.92 (s, 1H), 8.68 (s, 1H), 8.03 (d, J = 6.3 Hz, 2H), 7.52 (d, J = 8.4 Hz, 1H), 7.41 (dd, J = 8.4, 6.3 Hz, 1H), 7.29 (ddd, J = 14.6, 9.6, 4.4 Hz, 2H), 7.00 (s, 1H), 6.88 (td, J = 8.6, 2.9 Hz, 1H), 4.00 - 3.87 (m, 1H), 0.75 (q, J = 5.8 Hz, 2H), 0.50 - 0.29 (m, 2H). |
| A109 | 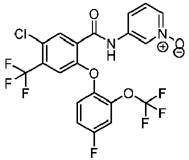 | LCMS: m/z 510.8 (M+H) ⁺; ¹H NMR (400 MHz, DMSO-d6) δ 11.01 (s, 1H), 8.62 (s, 1H), 8.15 (s, 1H), 8.02 (d, J = 6.1 Hz, 1H), 7.59 (d, J = 8.5 Hz, 1H), 7.52 - 7.38 (m, 3H), 7.35 (d, J = 5.8 Hz, 2H). |
| A110 | 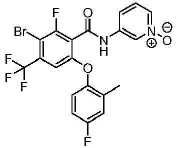 | LCMS: m/z 502.8 (M+H) ⁺; ¹H NMR (400 MHz, Chloroformd) δ 11.33 (s, 1H), 8.60 (s, 1H), 8.25 (d, J = 8.6 Hz, 1H), 7.39 (d, J = 6.3 Hz, 1H), 7.19 (s, 1H), 7.11 - 7.00 (m, 1H), 7.00 - 6.85 (m, 2H), 6.70 (s, 1H), 2.17 (d, J = 2.4 Hz, 3H). |
| A111 | 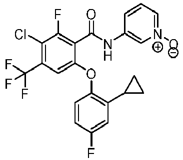 | LCMS: m/z 485.0 (M+H) ⁺; ¹H NMR (400 MHz, DMSO-d6) δ 11.37 (s, 1H), 8.65 (s, 1H), 8.06 (d, J = 6.1 Hz, 1H), 7.52 - 7.38 (m, 2H), 7.20 (m, 1H), 7.08 (td, J = 8.4, 3.0 Hz, 1H), 6.94 (s, 1H), 6.89 (dd, J = 10.0, 3.0 Hz, 1H), 1.97- 1.90 (m, 1H), 0.89 - 0.82 (m, 2H), 0.72 - 0.66 (m, 2H). |
| A112 | 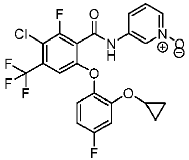 | LCMS: m/z 500.9 (M+H) ⁺; ¹H NMR (400 MHz, DMSO) δ 11.36 (s, 1H), 8.60 (s, 1H), 8.00 (d, J = 6.3 Hz, 1H), 7.45 (d, J = 8.7 Hz, 1H), 7.39-7.35 (m, 1H), 7.29-7.21 (m, 2H), 6.86 - 6.79 (m, 2H), 3.92-3.87 (m, 1H), 0.77 - 0.65 (m, 2H), 0.47 - 0.37 (m, 2H). |
| A113 | 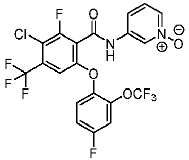 | LCMS: m/z 528.9 (M+H) ⁺; ¹H NMR (400 MHz, DMSO) δ 11.35 (s, 1H), 8.55 (s, 1H), 7.99 (dt, J = 5.4, 1.7 Hz, 1H), 7.56 (dd, J = 8.6, 1.5 Hz, 1H), 7.42 - 7.29 (m, 4H), 7.25 (s, 1H). |

### Synthesis of embodiment A114

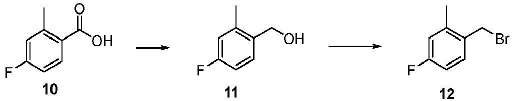

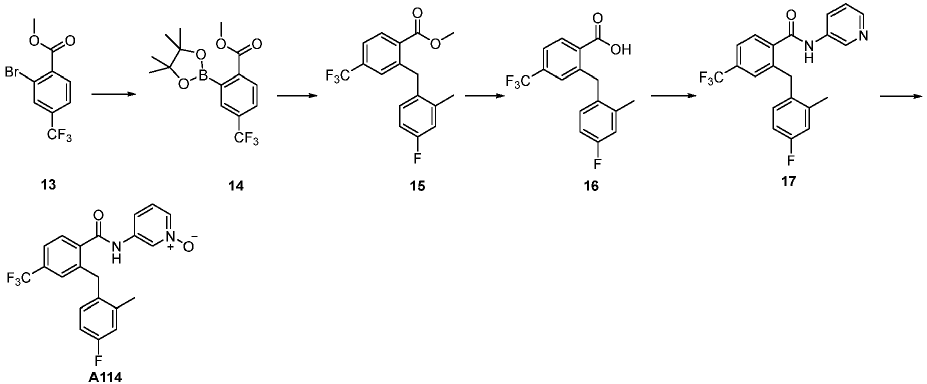

### Step 1, synthesis of intermediate 11

Compound **10** (5 g, 32.4 mmol) was added to anhydrous THF (20 mL), under nitrogen protection, 1M tetrahydrofuran borane complex (65 mL) was added dropwise in an ice-water bath, and the reaction was carried out for 3 h, TLC showed that the reaction was complete, the reaction was quenched by adding methanol (5 mL), the reaction mixture was concentrated, EA (100 mL) was added, and the mixture was washed with water (50 mL), and then washed with saturated NaCl (50 mL), then dried over anhydrous Na₂SO₄, filtered and concentrated to obtain compound **11,** 4.46 g, white solid, yield 98% . ¹H NMR (400 MHz, DMSO-*d₆*) δ 7.40 - 7.29 (m, 1H), 7.03 - 6.90 (m, 2H), 5.10 (t, *J* = 5.4 Hz, 1H), 4.45 (d, *J* = 5.4 Hz, 2H), 2.25 (s, 3H).

### Step 2, synthesis of intermediate 12

Compound **11** (4.4g, 31.4 mmol) was added to dichloromethane (40 mL), under nitrogen protection, PBr₃ (3.6 mL) was added dropwise in an ice water bath, and the reaction was carried out at room temperature for 2 h, TLC showed that the reaction was complete, the reaction mixture was poured into ice water and extracted by DCM (50 mL × 2), the organic phase was combined, dried over anhydrous Na₂SO₄, then purified by normal phase column (PE / EA = 0-100%) to obtain compound **12,** colorless liquid, 5.6g, yield: 88%. ¹H NMR (400 MHz, DMSO-*d₆*) δ 7.51 - 7.40 (m, 1H), 7.15 - 6.95 (m, 2H), 4.72 (s, 2H), 2.36 (s, 3H).

### Step 3, synthesis of intermediate 14

Compound **13** (2g, 7.09 mmol), bis(pinacolato)diboron (1.98 g, 7.80 mmol), KOAc (2.09 g, 21.3 mmol) and Pd(dppf)(Cl)₂ (0.26 g, 0.35 mmol) were added to a 50 mL three-necked flask, after ventilated with nitrogen for 3 times, 1,4-dioxane (20 mL) was added as solvent, the reaction was carried out in an oil bath at 85°C for 4 h, LC-MS showed that the reaction was complete, the reaction mixture was poured into ice water and extracted with EA (50 mL × 2), the organic phase was combined, dried over anhydrous Na₂SO₄ and purified by normal phase column (PE / EA = 0-100%) to obtain compound **14,** 1.8 g, yield: 77%. ¹H NMR (400 MHz, CDCl₃) δ 8.03 (d, *J* = 8.2 Hz, 1H), 7.74 (s, 1H), 7.68 (d, *J* = 8.2 Hz, 1H), 3.95 (s, 3H), 1.43 (s, 12H).

### Step 4, synthesis of intermediate 15

Compound **14** (359 mg, 1.1 mmol), compound **12** (200 mg, 1 mmol), K₂CO₃ (273 mg, 1.98 mmol) and Pd(dppf)(Cl)₂ (36 mg, 0.05 mmol) were added to a 25 mL three-necked flask, after ventilated with nitrogen for 3 times, a mixed solvent of 1,4-dioxane (10 mL) and water (3 mL) was added, the reaction was carried out in an oil bath at 100°C for 2 h, LC-MS showed that the reaction was complete, the reaction mixture was poured into ice water and extracted with EA (20 mL × 2), the organic phase was combined, dried over anhydrous Na₂SO₄, and purified by normal phase column (PE / EA = 0-100%) to obtain compound **15,** colorless oil, 200 mg, yield: 62%. LC-MS: m/z 327 (M+H)⁺.

### Step 5, synthesis of intermediate 16

Compound **15** (160 mg, 0.49 mmol) was added to a mixed solvent of tetrahydrofuran (6 mL) and methanol (3 mL), and lithium hydroxide monohydrate (82 mg, 1.96 mmol) was added in an ice-water bath, then water (2 mL) was added thereto. The reaction was carried out at room temperature for 2 h, TLC showed that the reaction mixture was complete, then the reaction mixture was poured into ice water, the PH value was adjusted to 3-4 with dilute hydrochloric acid, and then the mixture was extracted with EA (10 mL×2), the organic phase was combined, dried over anhydrous Na₂SO₄, filtered and concentrated to obtain compound **16,** white solid, yield 98%. LCMS: m/z 311 (M-H) ⁻.

### Step 6, synthesis of intermediate 17

Compound **16** (150 mg, 0.48 mmol) was added to DCM (5 mL), 0.2 mL of oxalyl chloride was added dropwise in an ice-water bath, and 2 drops of DMF was used to catalyze, the reaction was carried out at room temperature for 2 h, TLC showed that the reaction was complete, and the reaction mixture was directly concentrated to dryness to obtain 160 mg of product. The product was added to dichloromethane (20 mL), DIPEA (0.24 mL, 1.45 mmol) was added dropwise in an ice-water bath, then compound **2** (160 mg, 0.48 mmol) was slowly added dropwise, and the reaction was carried out at room temperature for 2 h, LC-MS showed that the reaction was complete, the reaction mixture was poured into ice water, extracted with DCM (20 mL×2), the organic phase was combined, dried over anhydrous Na₂SO₄, concentrated and purified by normal phase column (PE/EA=0-100%) to obtain compound **17,** white solid, 63 mg, yield: 33%. LCMS: m/z 389(M+H)⁺.

### Step 7, Synthesis of embodiment A114

Compound **17** (63 mg, 0.16 mmol) was added to DCM (5 mL), *m*-CPBA (56 mg, 0.32 mmol) was added at 0°C, and the reaction was carried out at room temperature for 2 h, LC-MS showed that the reaction was complete, the reaction mixture was poured into ice water, the pH value was adjusted with saturated NaHCO₃ to weakly basic, then the mixture was extracted with DCM (20 mL×2), the organic phase was combined, dried over anhydrous Na₂SO₄, filtered and concentrated to obtain a crude product, and the crude product was prepared to obtain (5-95% acetonitrile in water (containing 0.05% NH₄HCO₃)) the product **A114**. LCMS: m/z 405.2 (M+H) ⁺; ¹H NMR (400 MHz, DMSO-*d₆*) δ 10.83 (s, 1H), 8.65 (s, 1H), 8.06 - 7.96 (m, 1H), 7.78 (s, 2H), 7.47 (d, *J* = 10.7 Hz, 2H), 7.38 (dd, *J* = 8.4, 6.3 Hz, 1H), 7.03 - 6.94 (m, 2H), 6.88 (td, *J* = 8.6, 2.7 Hz, 1H), 4.18 (s, 2H), 2.16 (s, 3H).

### Effect embodiment:

### I. The blocking activity of the compound of the present disclosure on sodium ion channel 1.8 (NaV1.8)

1. Test method: patch clamp technique was used to detect the influence of compounds on voltage-gated sodium channel (NaV) 1.1-1.8 subtype current
2. Preparation and analysis of dosing formulations
2.1 Preparation method of dosing formulation storage solution
   **Control:** an appropriate volume of DMSO was weighted as a storage solution.
   **Test compound:** an appropriate mass of the compound (actual amount = theoretical concentration ^{∗} volume x molecular weight/purity) was weighed, the required DMSO volume according to the formula was calculated, and then the final required DMSO mass was obtained. Then the powder was dissolved with weighed DMSO. The actual storage solution concentration was calculated according to the final DMSO usage, generally, the actual storage solution concentration was slightly different from the theoretical concentration.
2.2 Preparation method and concentration of the working solution for dosing formulations
   Before the NaV channel current test, the control and test compound storage solutions were diluted into 10 mL of extracellular fluid as a working solution and were sonicated for 20 min.

3. Experimental system
3.1. Cell culture
   1) The specific information of the CHO cell line stably expressing the Nav1.8 channel was as follows: SCN10A: NM_006514
   2) Cells were cultured in HAM'S/F-12 medium containing 10% fetal bovine serum and 10 µg/mL Blasticidin, 200 µg/mLHygromycin B and 100 µg/mLZeocin, culture temperature was 37 °C and the concentration of CO₂ was 5%.
   3) Cell passage: the old medium was removed and the cells were washed with PBS once, then 1 mL of 0.25%-Trypsin-EDTA solution was added thereto, then incubated at 37 °C for 1.5 min. The cells were detached from the bottom of the dish, 5 mL of complete medium pre-warmed at 37°C was added. The cell suspension was gently blown with a pipette to separate the aggregated cells. The cell suspension was transferred to a sterile centrifuge tube and centrifuged at 1000 rpm for 5 min to collect cells. The culture was amplified or maintained, the cells were inoculated in a 6 cm cell culture dish, and the amount of cells inoculated in each cell culture dish was 2.5 ^{∗} 10⁵ cells (final volume: 5 mL).
   4) In order to maintain the electrophysiological activity of cells, the cell density must not exceed 80%.
   5) Patch clamp detection, before the experiment, the cells were separated with 0.25% - trypsin EDTA, inoculated into a 24 well plate with a density of 8 ^{∗} 10³ cells per well (final volume: 500 µL), tetracycline was added thereto, and experimental test was performed on the next day.
3.2. Electrophysiological solution
   1) Extracellular fluid: 140 mM NaCl, 3.5mM KCl, 2mM CaCl₂, lOmM HEPES, 1.25mM NaH₂PO₄, 1mM MgCl₂, 10mM Glucose, pH=7.4 (NaOH).
   2) Intracellular fluid: 50mM CsCl, lOmM NaCl, lOmM HEPES , 20mM EGTA, 60mM CsF, pH=7.2 (CsOH).

4. Test method
4.1. The instrument was shown in Table 3 below

**Table 3: Instrument supplier and model**

| **Name** | **Supplier** | **Model** |
|---|---|---|
| Amplifier | HEKA (Germany) | EPC10 |
| Micromanipulator | Sutter Instruments (USA) | MP285 |
| Electrode drawing instrument | Sutter Instruments (USA) | P97 |
| Microscope | Olympus (Japan) | IX71 |
| Fur glass tube | Sutter Instruments (USA) | BF150-86-10 |
| Data acquisition and analysis software | HEKA (Germany) | Patchmaster & IGOR |

4.2 Patch clamp detection

The voltage stimulation scheme of whole-cell patch clamp recording of Nav channel current was as follows: first the membrane potential of the cell was clamped at at -130mV, and then the voltage was stepped to -40mV or -20mV at 10mv step intervals for 8 s. The clamping voltage was maintained at - 120mV, and data was collected repeatedly every 20 seconds. The peak amplitude of its inward current was measured to determine its half-inactivationvoltage.

The cell clamping potential was set at -120 mV. The resting and half-inactivation inhibition of sodium current was measured using double pulse mode. The double pulse mode was completed by two 0 mV depolarization test pulses (TP1 and TP2) lasting 50 ms. The conditional voltage between the two depolarization pulses was set near the half-inactivation voltage (lasting 8 s). Before giving the second depolarization pulse, the cell membrane potential was clamped to -120 mv for 20 ms to restore the channel that was not bounded to the compound and in an inactive state. The data acquisition was repeated at an interval of 20 s and the current peak was measured at the two test pulses.

The experimental data was collected by EPC-10 amplifier (HEKA) and stored in PatchMaster (HEKA) software (software version: v2x73.2).

The capillary glass tube (BF150-86-10, Sutter Instruments) was drawn into a recording electrode with a microelectrode drawing instrument (P97, Sutter Instruments). The microelectrode manipulator (MP285) was manipulated under an inverted microscope (IX71) to bring the recording electrode into contact with the cells, giving negative pressure suction to form a GΩ seal. After forming the GΩ seal, fast capacitance compensation was performed, and then continued to give negative pressure to suck and break the cell membrane to form a whole-cell recording mode. Then slow capacitance compensation was performed, and the film capacitance and series resistance were recorded, no leakage compensation was given.

When the current of the Nav channel recorded by the whole cell was stable, the drug was administered, after each drug concentration was applied for 5 minutes (or the current was stable), the next concentration would be tested, and multiple concentrations would be tested for each test compound. The cover glass covered with cells was placed in the recording bath of an inverted microscope, the test compound and the external liquid without compound flowed through the recording chambers in turns from low concentration to high concentration by gravity perfusion, so as to act on the cells, in the recording, the liquid exchange was carried out by vacuum pump. The current detected in each cell in the compound-free external fluid served as its own control group. Multiple cells were detected independently and repeatedly. All electrophysiological experiments were performed at room temperature.

### 4.3 Data analysis

First, the current after the action of each drug concentration and the blank control current were normalized, and then the blocking rate corresponding to each drug concentration was calculated. The mean and standard error were calculated for each concentration, and all the above values were calculated by Microsoft Excel 2013. In addition, the half-inhibitory concentration of each compound was calculated by IGOR software with the following equation: retardation rate = 1/[1+(IC₅₀/c)^{h}].

The dose-dependent effect was nonlinearly fitted with the above equation, wherein, c refers to the drug concentration, IC₅₀ was the half inhibitory concentration, and h refers to the Hill coefficient. The curve fitting and the calculation of IC₅₀ were completed by IGOR software (software version: 6.0.1.0).

In this embodiment, the half blocking activity (IC₅₀) of some compounds of the present disclosure against Nav 1.8 was measured, as shown in Table 4, and the blocking rate of some compounds to Nav1.8 at 100 nM was shown in Table 5. Wherein:

**Table 4: The IC₅₀ value (nM) of the blocking activity of the compounds of the present disclosure against NaV1.8**

| **Number** | **NaV1.8 IC₅₀(nM)** | **Number** | **NaV1.8 IC₅₀(nM)** | **Number** | **NaV1.8 IC₅₀(nM)** |
|---|---|---|---|---|---|
| A5 | 7.4 | A11 | 6.2 | A19 | 10.0 |
| A22 | 0.32 | A27 | 0.64 | A28 | 2.9 |
| A33 | 4.6 | A35 | 3.0 | A37 | 3.2 |
| A38 | 7.3 | A41 | 1.3 | A42 | 3.6 |
| A46 | 3.1 | A47 | 3.0 | A51 | 2.7 |
| A56 | 3.7 | A57 | 1.8 | A61 | 10.2 |
| A67 | 6.7 | A68 | 0.27 | A69 | 2.0 |
| A73 | 3.2 | A74 | 3.5 | A80 | 8.4 |
| A85 | 1.7 | A86 | 0.08 | A106 | 3.0 |

**Table 5: The blocking rate of the compounds of the present disclosure against NaV1.8 at a certain concentration**

| Number | Blocking rate (%) @ 100nM | Number | Blocking rate (%) @ 100nM | Number | Blocking rate (%) @ 100nM | Number | Blocking rate (%) @ 100nM |
|---|---|---|---|---|---|---|---|
| A8 | 81.85 | A10 | 78.17 | A16 | 85.08 | A18 | 96.02 |
| A20 | 87.14 | A21 | 98.02 | A23 | 97.04 | A24 | 91.28 |
| A25 | 87.92 | A26 | 79.32 | A29 | 87.63 | A31 | 88.88 |
| A32 | 82.91 | A34 | 90.16 | A36 | 87.40 | A39 | 97.82 |
| A40 | 99.91 | A43 | 76.10 | A44 | 77.61 | A45 | 78.46 |
| A50 | 79.66 | A52 | 80.75 | A53 | 85.77 | A54 | 94.99 |
| A55 | 83.29 | A58 | 82.55 | A59 | 87.57 | A62 | 89.79 |
| A63 | 93.13 | A65 | 93.18 | A68 | 99.15 | A72 | 85.63 |
| A75 | 88.87 | A78 | 96.55 | A83 | 84.98 | A84 | 99.14 |
| A90 | 89.50 | A91 | 92.81 | A92 | 85.44 | A95 | 93.84 |
| A96 | 88.78 | A97 | 86.65 | A98 | 95.70 | A100 | 97.60 |
| A101 | 98.47 | A102 | 82.71 | A103 | 92.47 | A104 | 97.93 |
| A105 | 98.42 | A107 | 95.60 | A108 | 96.92 | A109 | 88.79 |
| A110 | 98.75 | A111 | 99.42 | | | | |

It can be seen that the compound of the present disclosure has a significant blocking effect against NaV1.8 channel activity.

### II. Pharmacokinetic test results of the compound of the present disclosure

In this experimental embodiment, the *in vivo* pharmacokinetics of rat was evaluated by a single intravenous injection or oral administration by gavage.

Experimental methods and conditions: Male Sprague Dawley rats, all animals were fasted overnight, and the test compound was given 1 mg/Kg (intravenous injection, solvent 5% DMSO/10% Solutol/85% Saline) and 10 mg/Kg (administrated by gavage) respectively, 5, 15, 30 min, 1, 2, 4, 6, 8 and 24 hr after administration, blood was collected through the submandibular vein, about 0.20 mL of each sample was collected, heparin sodium was used for anticoagulation, the samples were placed on ice after the collection, then centrifuged within 1 hour to separate the plasma for testing. The blood drug concentration in plasma was detected by liquid-phase tandem mass spectrometry (LC/MS/MS), and the pharmacokinetic parameters were calculated by the measured concentration. The results were shown in Table 6 and Table 7 below.

**Table 6: Pharmacokinetics of intravenous administration (1 mg/kg)**

| Number | T_{1/2} (hr) | AUC_{inf} (ng^{∗}hr/mL) | V_{z} (mL/kg) | CL (mL/min/kg) |
|---|---|---|---|---|
| A11 | 0.70 | 1462.27 | 699.19 | 11.44 |
| A46 | 20.08 | 15625.27 | 1847.50 | 1.08 |
| A56 | 13.09 | 9602.04 | 1969.82 | 1.74 |
| A67 | 0.67 | 2400.69 | 441.48 | 7.61 |
| A68 | 5.24 | 33349.32 | 228.44 | 0.50 |
| A69 | 12.27 | 56697.73 | 311.95 | 0.30 |
| A84 | 4.53 | 18823.37 | 354.87 | 0.91 |
| A103 | 2.28 | 2758.59 | 1190.18 | 6.15 |

**Table 7: Pharmacokinetics of intragastric injection (10 mg/kg)**

| Number | T_{1/2} (hr) | Cmax (ng/mL) | AUC_{inf} (ng^{∗}hr/mL) | F (%) |
|---|---|---|---|---|
| A11 | 2.51 | 1326.67 | 10614.74 | 72.59 |
| A46 | 914.98 | 2056.67 | 2934150.31 | 46.75 |
| A56 | 27.88 | 3000.00 | 117018.94 | 68.16 |
| A67 | 1.61 | 3543.33 | 12373.07 | 51.54 |
| A68 | 5.72 | 9703.33 | 139330.03 | 41.78 |
| A69 | 12.15 | 17100.00 | 398902.00 | 70.36 |
| A84 | 5.75 | 4916.67 | 67173.65 | 35.69 |
| A103 | 2.47 | 1713.33 | 13988.83 | 50.71 |

It can be seen that the compound of the present disclosure has good pharmacokinetic absorption in rats and has pharmacokinetic advantages.

All documents mentioned in the present disclosure are incorporated by reference in this application as if each document are individually incorporated by reference. It should also be understood that after reading the above teaching content of the present disclosure, a person skilled in the art may make various changes or modifications to the present disclosure, which in equivalent form likewise fall within the scope of the claims appended to this disclosure.

## Claims

1. A compound as shown in formula (I), an optical isomer thereof and a pharmaceutically acceptable salt thereof, 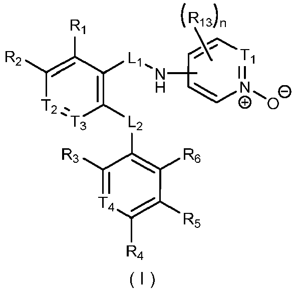 wherein,
T₁ is selected from N or C(R₇);
T₂ is selected from N or C(R₈);
T₃ is selected from N or C(R₉);
T₄ is selected from N or C(R₁₀);
R₁, R₂, R₈, R₉ are each independently selected from H, halogen, OH, NH₂, CN, SF₅, C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-O-, phenyl-C₁₋₃ alkyl-, C₃₋₆ cycloalkyl-C₁₋₃ alkyl-O-, 3-6 membered heterocycloalkyl-C₁₋₃ alkyl-O-, phenyl-C₁₋₃ alkyl-O-, phenyl-C₁₋₃ alkyl-NH-, 5-6 membered heteroaryl-C₁₋₃ alkyl-, 5-6 membered heteroaryl-C₁₋₃ alkyl-O- and 5-6 membered heteroaryl-C₁₋₃ alkyl-NH-, the C₁₋₆ alkyl, C₁₋₆ alkoxy, C₁₋₆ alkylamino, vinyl-C₁₋₆ alkyl-, C₃₋₆ cycloalkyl, 3-6 membered heterocycloalkyl, C₃₋₆ cycloalkyl-C₁₋₆ alkyl-, 3-6 membered heterocycloalkyl-C₁₋₆ alkyl-, 3-6 membered heterocycloalkyl-C₁₋₆ alkyl-O-, phenyl-C₁₋₃ alkyl-, C₃₋₆ cycloalkyl-C₁₋₃ alkyl-O-, 3-6 membered heterocycloalkyl-C₁₋₃ alkyl-O-, phenyl-C₁₋₃ alkyl-O-, phenyl-C₁₋₃ alkyl-NH-, 5-6 membered heteroaryl-C₁₋₃ alkyl-, 5-6 membered heteroaryl-C₁₋₃ alkyl-O- or 5-6 membered heteroaryl-C₁₋₃ alkyl-NH- is optionally substituted by 1, 2 or 3 R;
and, when T₃ is selected from N, R₁ is not H;
R₃, R₄, R₅, R₆, R₁₀ are each independently selected from H, halogen, OH, NH₂, SF₅, CN, C₁₋₆ alkyl, C₁₋₆ alkylamino, C₁₋₆ alkoxy, C₃₋₆ cycloalkayl, -O-C₃₋₆ cycloalkyl, 3-6 membered heterocycloalkyl, C₃₋₆ cycloalkyl-C₁₋₆ alkyl- and 3-6 membered heterocycloalkyl-C₁₋₆ alkyl-, the C₁₋₆ alkyl, C₁₋₆ alkylamino, C₁₋₆ alkoxy, C₃₋₆ cycloalkayl, -O-C₃₋₆ cycloalkyl, 3-6 membered heterocycloalkyl, C₃₋₆ cycloalkyl-C₁₋₆ alkyl- or 3-6 membered heterocycloalkyl-C₁₋₆ alkyl- is optionally substituted by 1, 2 or 3 R;
R₇ is selected from H, F, Cl, Br, I, C₁₋₆ alkyl, C₁₋₆ alkoxy and C₁₋₆ alkylamino, the C₁₋₆ alkyl, C₁₋₆ alkoxy or C₁₋₆ alkylamino is optionally substituted by 1, 2 or 3 R;
L₁ is selected from C(=O), NH and 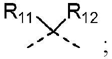
L₂ is selected from O, S, NH and CH₂, the CH₂ is optionally substituted by 1 or 2 R, and NH is optionally substituted by R;
R₁₁ and R₁₂ are each independently selected from H, halogen, OH, NH₂ and C₁₋₆ alkyl, the C₁₋₆ alkyl is optionally substituted by 1, 2 or 3 R; or, R₁₁ and R₁₂ are connected together to form a 3-6 membered ring;
each of R₁₃ is independently selected from H, halogen and C₁₋₆ alkyl, the C₁₋₆ alkyl is optionally substituted by 1, 2 or 3 R;
n is selected from 1, 2 or 3;
each of R is independently selected from H, D, halogen, OH, NH₂, CN, 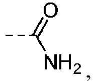 C₁₋₆ alkyl, C₁₋₆ alkoxy, C₁₋₆ alkylthio and C₁₋₆ alkylamino, the C₁₋₆ alkyl, C₁₋₆ alkoxy, C₁₋₆ alkylthio or C₁₋₆ alkylamino is optionally substituted by 1, 2 or 3 R';
R' is selected from F, Cl, Br, I, OH, NH₂ and CH₃;
the 3-6 membered heterocycloalkyl or 5-6 membered heteroaryl contains 1, 2 or 3 heteroatoms or heteroatom groups independently selected from -O-, -NH-, -S-, -C(=O)-, -C(=O)O-, -S(=O)-, -S(=O)₂- and N.

2. The compound as defined in claim 1, the optical isomer thereof and the pharmaceutically acceptable salt thereof, wherein, R is selected from H, D, F, Cl, Br, I, OH, NH₂, 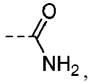 Me, CF₃, CHF₂, CH₂F,

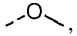

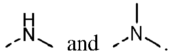

3. The compound as defined in claim 1 or 2, the optical isomer thereof and the pharmaceutically acceptable salt thereof, wherein, R₁, R₂, R₈, R₉ are each independently selected from H, halogen, OH, NH₂, CN, SF₅, C₁₋₃ alkyl, C₁₋₃ alkoxy, C₁₋₃ alkylamino, vinyl-C₁₋₃ alkyl-, C₃₋₆ cycloalkyl, 3-6 membered heterocycloalkyl, C₃₋₆ cycloalkyl-C₁₋₃ alkyl-, 3-6 membered heterocycloalkyl-C₁₋₃ alkyl-, 3-6 membered heterocycloalkyl-C₁₋₃ alkyl-O-, phenyl-C₁₋₃ alkyl-, phenyl-C₁₋₃ alkyl-O-, phenyl-C₁₋₃ alkyl-NH-, pyridyl-C₁₋₃ alkyl-, pyrimidinyl-C₁₋₃ alkyl-, thiophenyl-C₁₋₃ alkyl-, thiazolyl-C₁₋₃ alkyl-, pyrazolyl-C₁₋₃ alkyl-, imidazolyl-C₁₋₃ alkyl-, pyridyl-C₁₋₃ alkyl-O-, pyrimidinyl-C₁₋₃ alkyl-O-, thiophenyl-C₁₋₃ alkyl-O-, thiazolyl-C₁₋₃ alkyl-O-, pyrazolyl-C₁₋₃ alkyl-O-, imidazolyl-C₁₋₃ alkyl-O-, pyridyl-C₁₋₃ alkyl -NH-, pyrimidinyl-C₁₋₃ alkyl-NH-, thiophenyl-C₁₋₃ alkyl-NH-, thiazolyl-C₁₋₃ alkyl-NH-, pyrazolyl-C₁₋₃ alkyl-NH- and imidazolyl-C₁₋₃ alkyl-NH-, the C₁₋₃ alkyl, C₁₋₃ alkoxy, C₁₋₃ alkylamino, vinyl-C₁₋₃ alkyl-, C₃₋₆ cycloalkyl, 3-6 membered heterocycloalkyl, C₃₋₆ cycloalkyl-C₁₋₃ alkyl-, 3-6 membered heterocycloalkyl- C₁₋₃ alkyl-, 3-6 membered heterocycloalkyl-C₁₋₃ alkyl-O-, phenyl-C₁₋₃ alkyl-, phenyl-C₁₋₃ alkyl-O-, phenyl- C₁₋₃ alkyl-NH-, pyridyl-C₁₋₃ alkyl-, pyrimidinyl-C₁₋₃ alkyl-, thiophenyl-C₁₋₃ alkyl-, thiazolyl-C₁₋₃ alkyl-, pyrazolyl-C₁₋₃ alkyl-, imidazolyl-C₁₋₃ alkyl-, pyridyl-C₁₋₃ alkyl-O-, pyrimidinyl-C₁₋₃ alkyl-O-, thiophenyl-C₁₋₃ alkyl-O-, thiazolyl-C₁₋₃ alkyl-O-, pyrazolyl-C₁₋₃ alkyl-O-, imidazolyl-C₁₋₃ alkyl-O-, pyridyl-C₁₋₃ alkyl -NH-, pyrimidinyl-C₁₋₃ alkyl-NH-, thiophenyl-C₁₋₃ alkyl-NH-, thiazolyl-C₁₋₃ alkyl-NH-, pyrazolyl-C₁₋₃ alkyl-NH- or imidazolyl-C₁₋₃ alkyl-NH- is optionally substituted by 1, 2 or 3 R.

4. The compound as defined in claim 3, the optical isomer thereof and the pharmaceutically acceptable salt thereof, wherein, R₁, R₂, R₈, R₉ are each independently selected from H, F, Cl, Br, I, OH, NH₂, CN, SF₅, Me, CF₃, CHF₂, CF₃CF₂, CH₂F, OCF₃, HOCH₂CH₂O, CH₃NHCH₂CH₂O, (CH₃)₂NCH₂CH₂O,

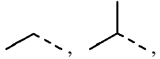

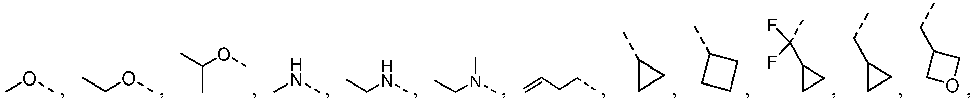

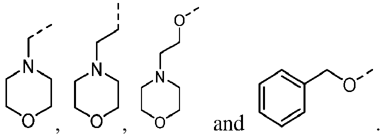

5. The compound as defined in claim 1 or 4, the optical isomer thereof and the pharmaceutically acceptable salt thereof, wherein, the structural moiety 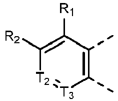 is selected from

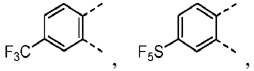

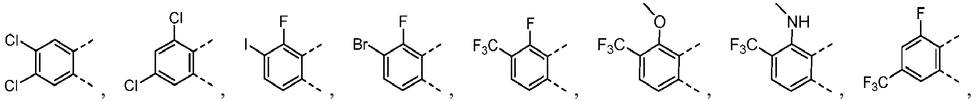

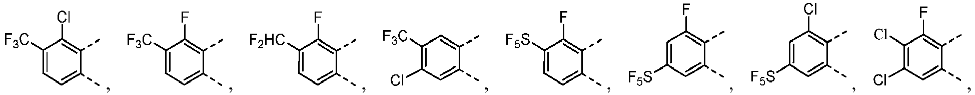

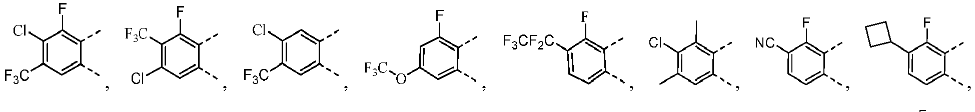

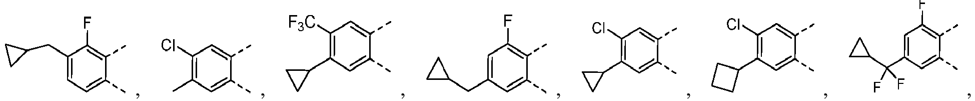

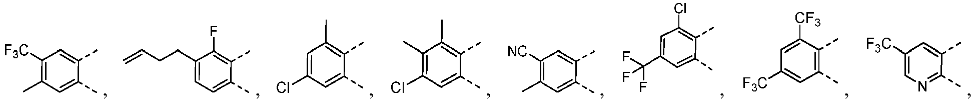

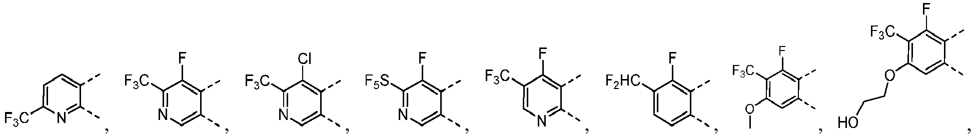

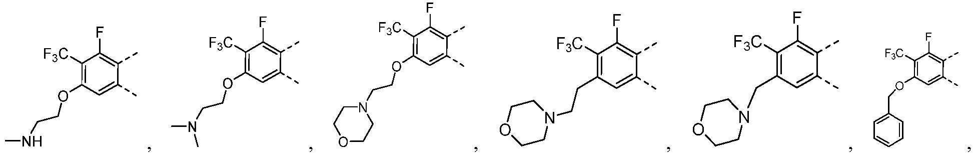

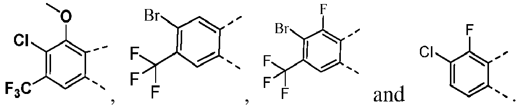

6. The compound as defined in claim 1 or 2, the optical isomer thereof and the pharmaceutically acceptable salt thereof, wherein, R₃, R₄, R₅, R₆, R₁₀ are each independently selected from H, halogen, OH, NH₂, SF₅, CN, C₁₋₃ alkyl, C₁₋₃ alkylamino, C₁₋₃ alkoxy, C₃₋₆ cycloalkayl, -O-C₃₋₆ cycloalkyl, 3-6 membered heterocycloalkyl, C₃₋₆ cycloalkyl-C₁₋₃ alkyl- and 3-6 membered heterocycloalkyl-C₁₋₃ alkyl-, the C₁₋₃ alkyl, C₁₋₃ alkylamino, C₁₋₃ alkoxy, C₃₋₆ cycloalkayl, -O-C₃₋₆ cycloalkyl, 3-6 membered heterocycloalkyl, C₃₋₆ cycloalkyl-C₁₋₃ alkyl- or 3-6 membered heterocycloalkyl-C₁₋₃ alkyl- is optionally substituted by 1, 2 or 3 R.

7. The compound as defined in claim 6, the optical isomer thereof and the pharmaceutically acceptable salt thereof, wherein, R₃, R₄, R₅, R₆, R₁₀ are each independently selected from H, F, Cl, Br, I, OH, NH₂, SF₅, Me, CF₃, CHF₂, CH₂F, CN, CH(F₂)CH₃, CD₃, OCD₃,

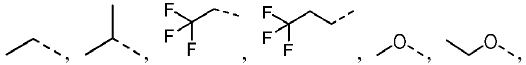

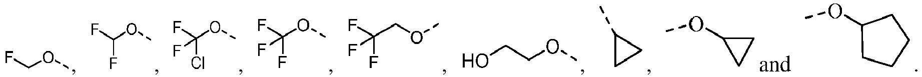

8. The compound as defined in claim 1 or 7, the optical isomer thereof and the pharmaceutically acceptable salt thereof, wherein, the structural moiety 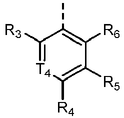 is selected from

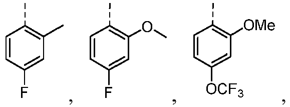

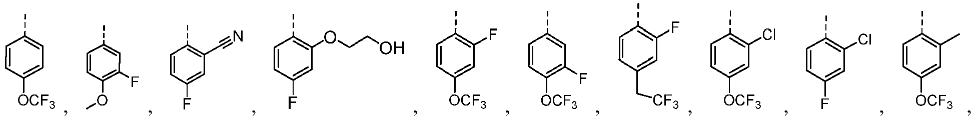

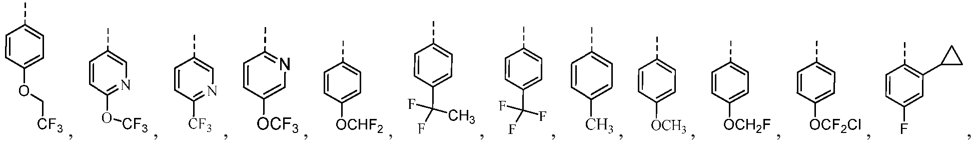

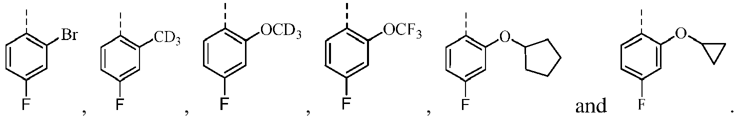

9. The compound as defined in claim 1, the optical isomer thereof and the pharmaceutically acceptable salt thereof, wherein, the structural moiety 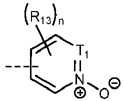 is selected from

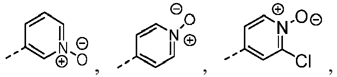

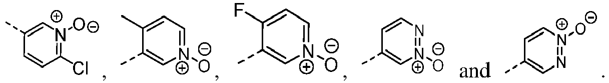

10. The compound as defined in claim 1, the optical isomer thereof and the pharmaceutically acceptable salt thereof, wherein, R₁₁, R₁₂ are each independently selected from H, F, Cl, Br, I, OH, NH₂, Me, CHF₂, CF₃,

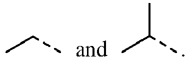

11. The compound as defined in claim 1, the optical isomer thereof and the pharmaceutically acceptable salt thereof, wherein, R₁₁ and R₁₂ are connected together to form a cyclopropyl, oxetanyl, azetidinyl and cyclopentanonyl.

12. The compound as defined in claim 10 or 11, the optical isomer thereof and the pharmaceutically acceptable salt thereof, wherein, L₁ is selected from C(=O), CH₂, NH, CH(CH₃), CHF, CF₂, CHCHF₂, CHCF₃,

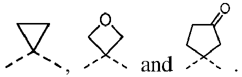

13. The compound as defined in any one of claims 1-12, the optical isomer thereof and the pharmaceutically acceptable salt thereof, wherein, the compound is selected from 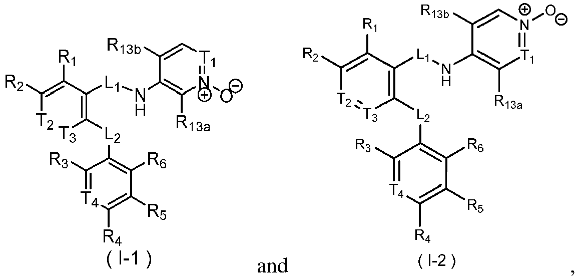 wherein,
T₁ is as defined in claim 1 or 9;
R₁, R₂, T₂, T₃ are as defined in claim 1, 3, 4 or 5;
R₃, R₄, R₅, R₆, T₄ are as defined in claim 1, 6, 7 or 8;
L₁ is as defined in claim 1, 10, 11 or 12;
L₂ is as defined in claim 1;
R₁₃ₐ, R_{13b} are each independently selected from H, halogen and C₁₋₆ alkyl, the C₁₋₆ alkyl is optionally substituted by 1, 2 or 3 R;
R is as defined in claim 1 or 2.

14. A compound as shown in the formula below, an optical isomer thereof and a pharmaceutically acceptable salt thereof selected from

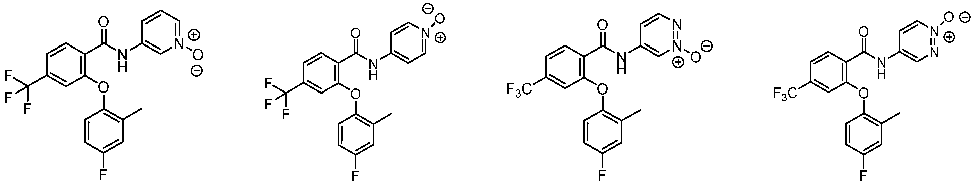

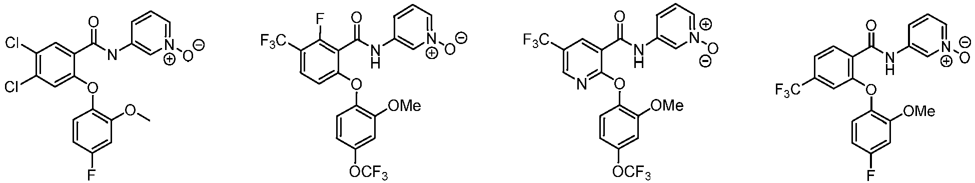

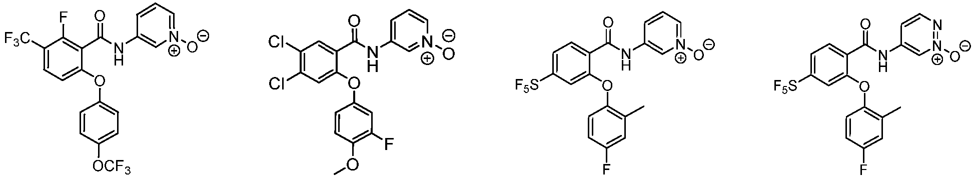

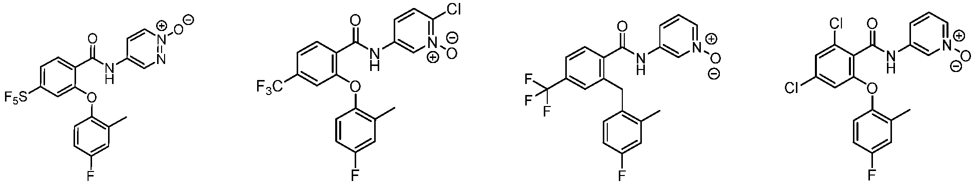

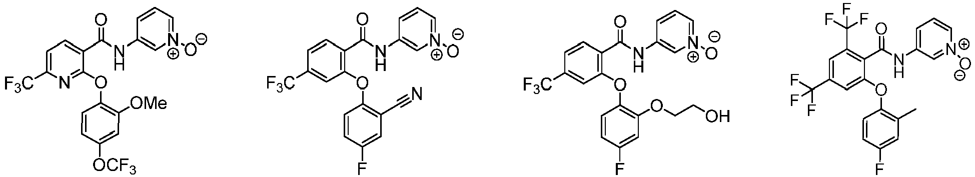

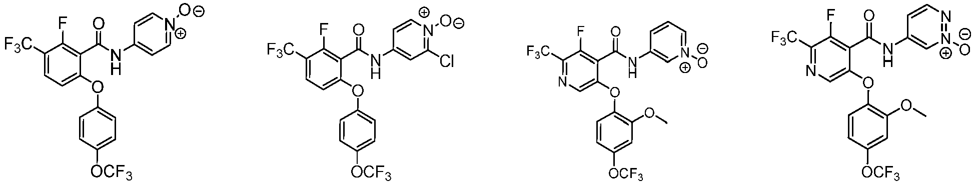

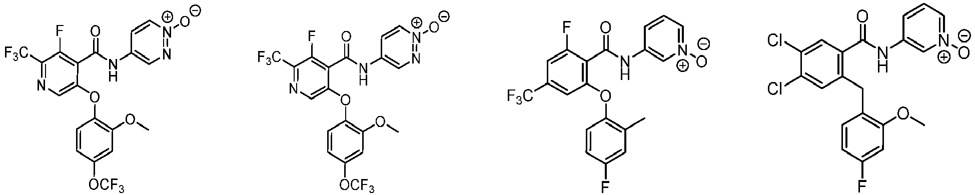

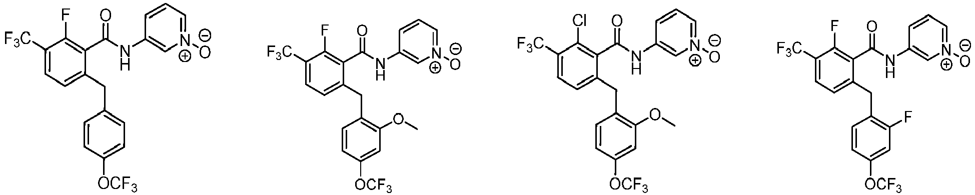

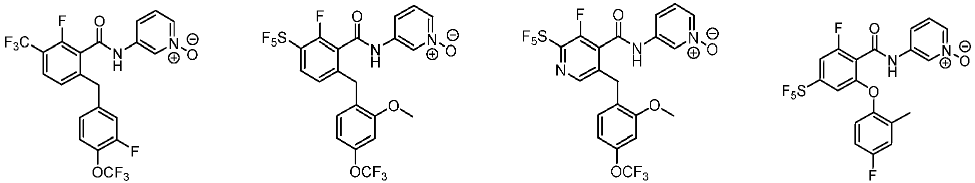

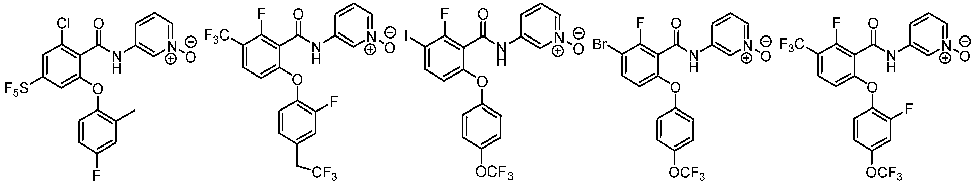

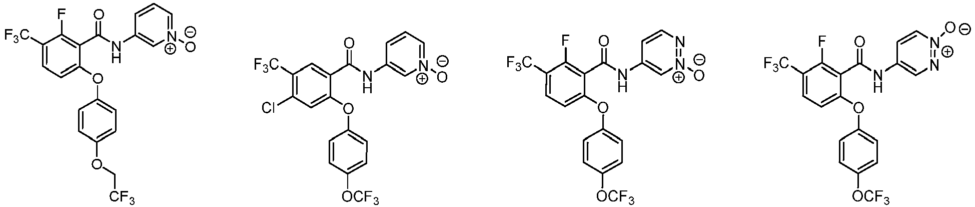

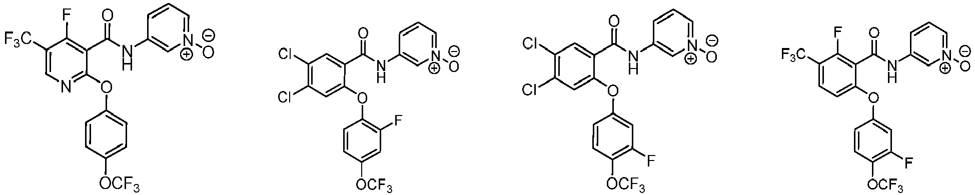

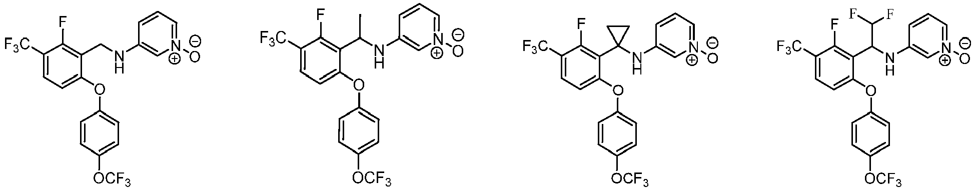

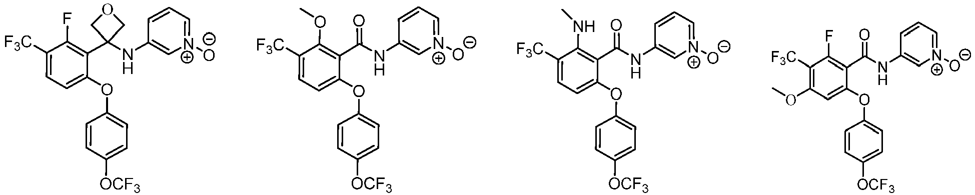

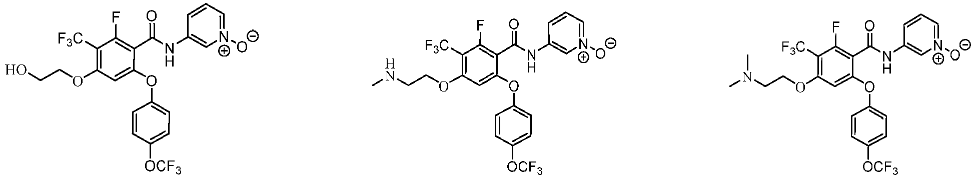

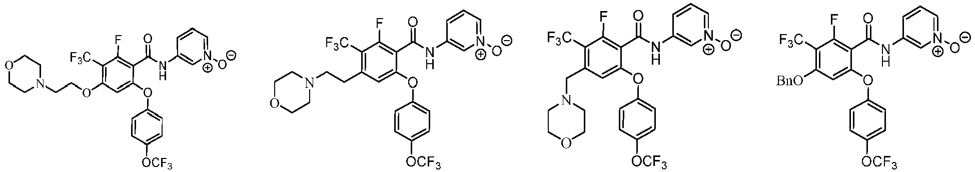

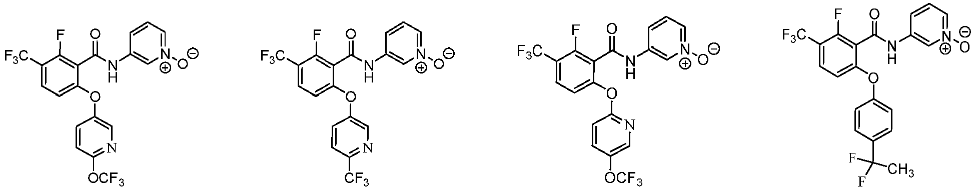

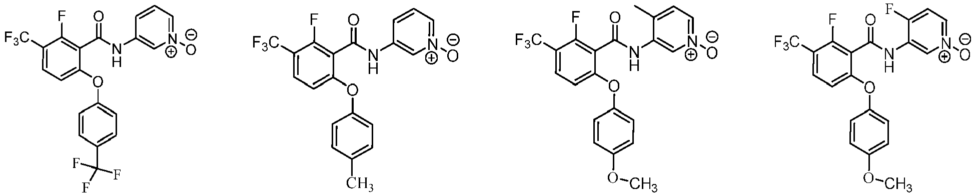

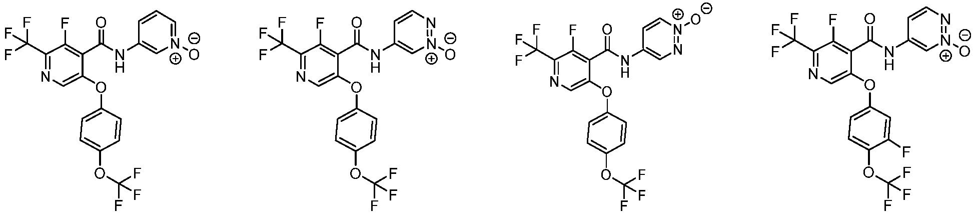

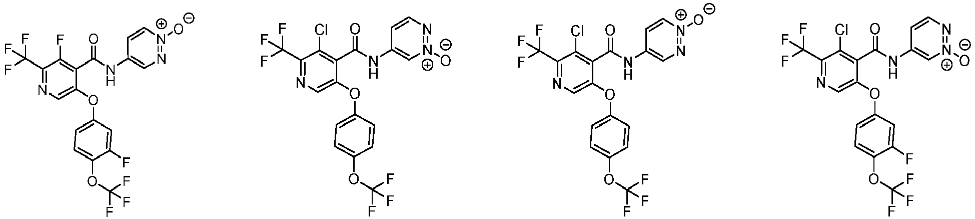

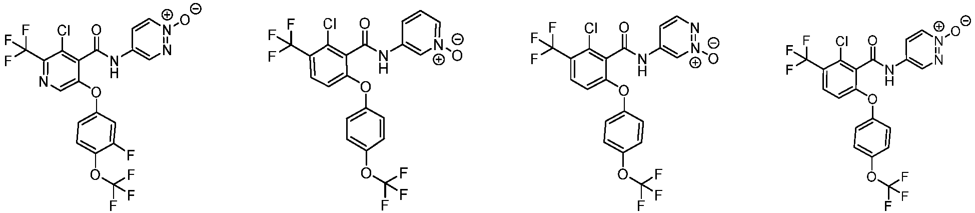

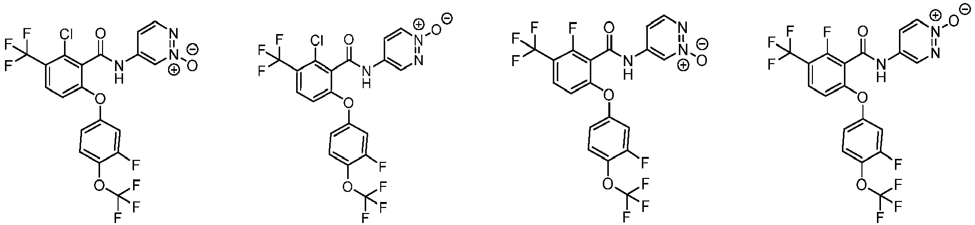

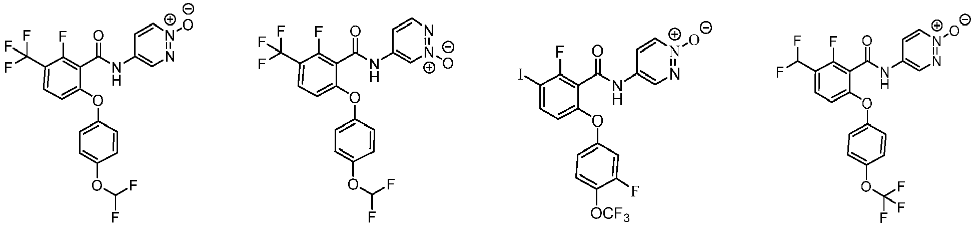

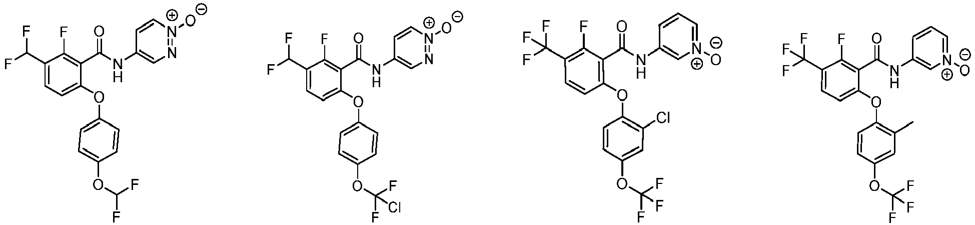

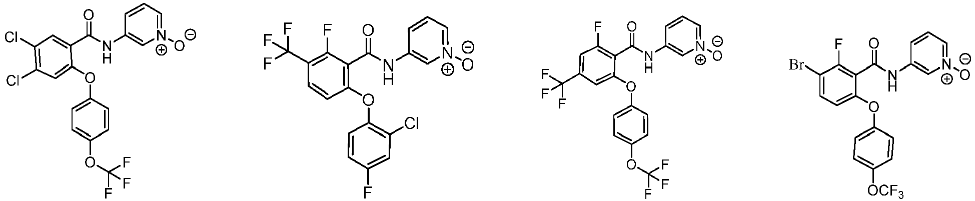

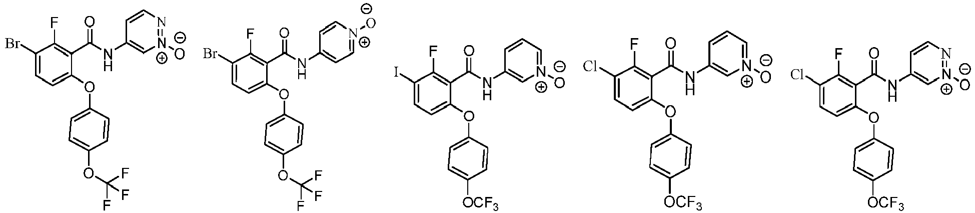

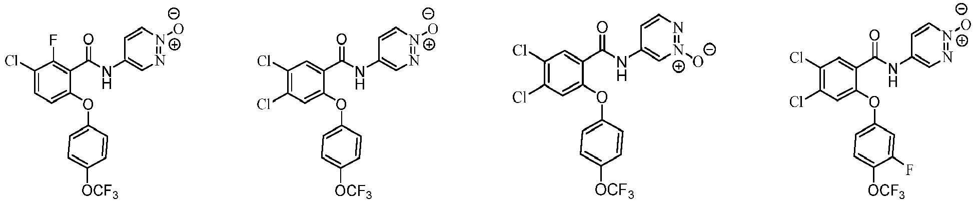

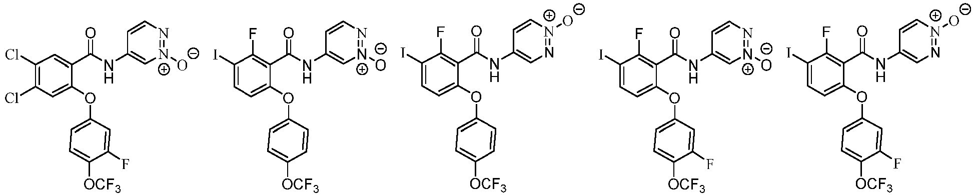

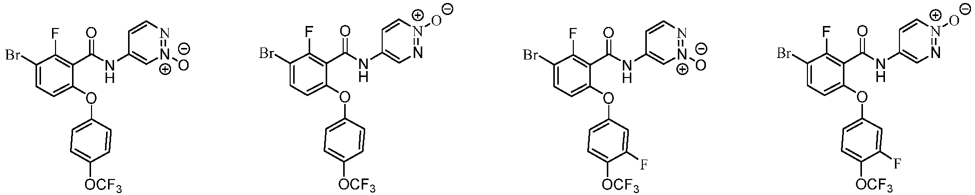

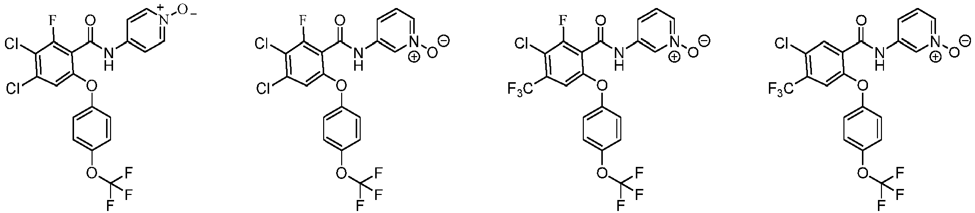

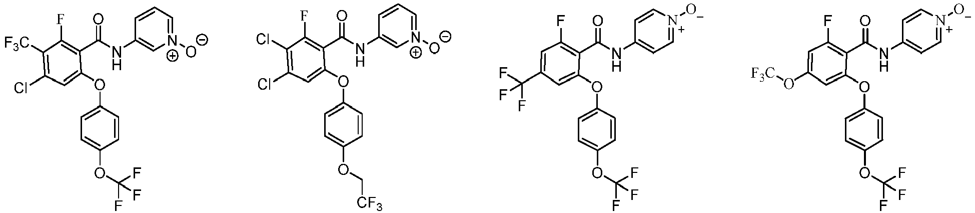

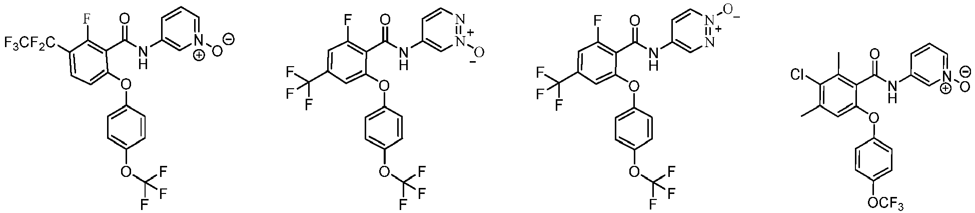

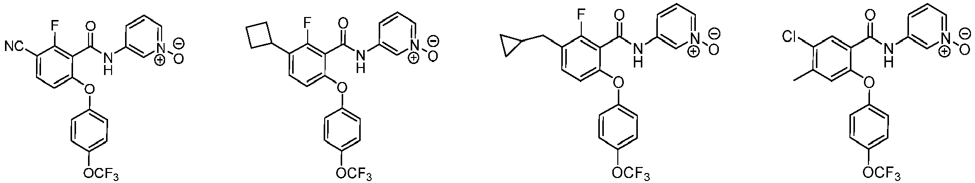

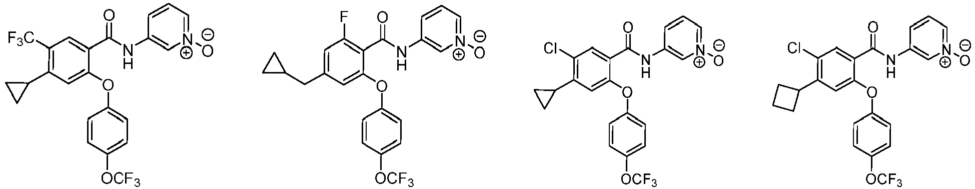

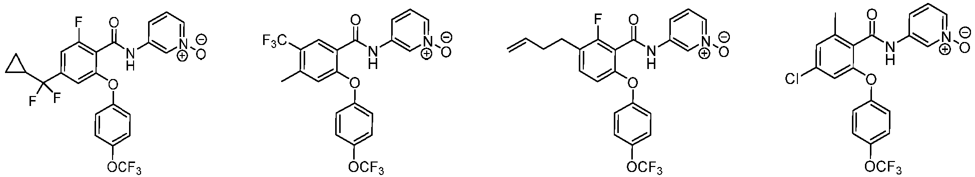

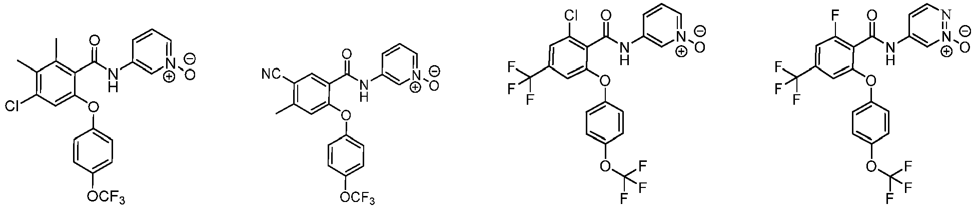

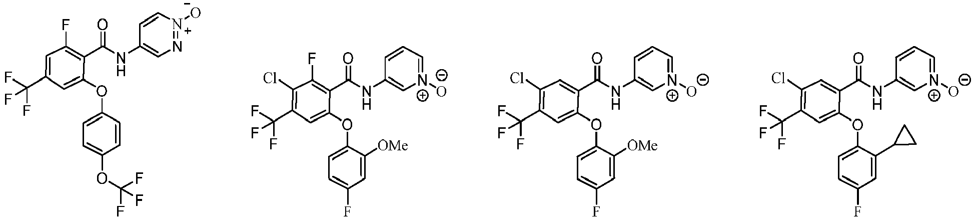

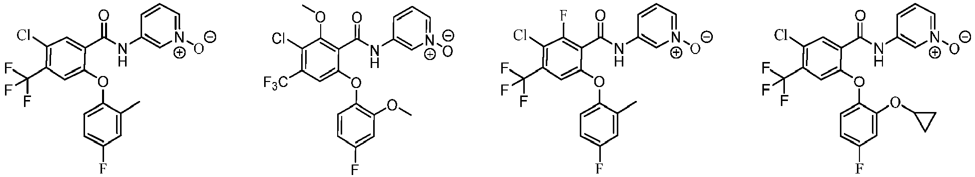

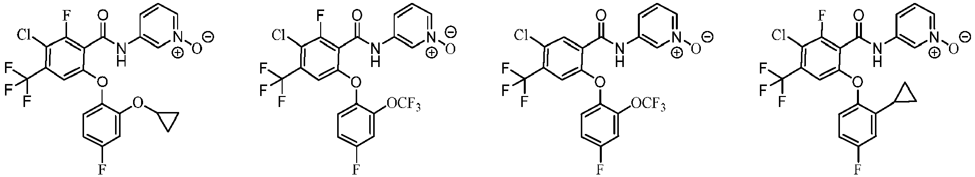

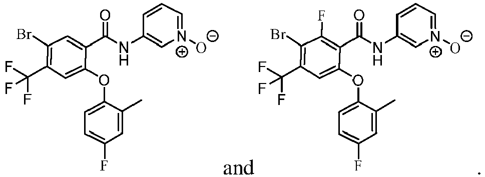

15. A pharmaceutical composition, wherein, the pharmaceutical composition comprises the compound as defined in any one of claims 1-14, the optical isomer thereof and the pharmaceutically acceptable salt thereof.

16. The pharmaceutical composition as defined in claim 15, wherein, the pharmaceutical composition further comprises a pharmaceutically acceptable carrier or excipient.

17. A use of the compound as defined in any one of claims 1-14, the optical isomer thereof and the pharmaceutically acceptable salt thereof, or the pharmaceutical composition as defined in claim 15 or 16 in the manufacture of a medicament for inhibiting the voltage-gated sodium ion channel in an individual.

18. The use as defined in claim 17, wherein, the voltage-gated sodium ion channel is Nav1.8.

19. A use of the compound as defined in any one of claims 1-14, the optical isomer thereof and the pharmaceutically acceptable salt thereof, or the pharmaceutical composition as defined in claim 15 or 16 in the manufacture of a medicament for treating and/or preventing pain or cough, or relieving the severity of pain or cough in an individual.

20. The use as defined in claim 19, wherein, the pain is selected from chronic pain, intestinal pain, neuropathic pain, musculoskeletal pain, acute pain, inflammatory pain, cancer pain, primary pain, postoperative pain, visceral pain, multiple sclerosis, Charcot, Marfan and Down syndrome, incontinence and arrhythmia.
